(19) Europäisches Patentamt
European Patent Office
Office européen des brevets

(11) Veröffentlichungsnummer: **0 402 316 B1**

(12) **EUROPÄISCHE PATENTSCHRIFT**

(45) Veröffentlichungstag der Patentschrift: **14.09.94**

(21) Anmeldenummer: **90810392.2**

(22) Anmeldetag: **29.05.90**

(51) Int. Cl.5: **C07D 401/12**, C07D 405/14, C07D 409/14, C07D 213/70, C07D 405/12, C07D 409/12, A01N 47/36

Die Akte enthält technische Angaben, die nach dem Eingang der Anmeldung eingereicht wurden und die nicht in dieser Patentschrift enthalten sind.

(54) **Sulfonylharnstoffe.**

(30) Priorität: **06.06.89 CH 2135/89**
**18.07.89 CH 2679/89**

(43) Veröffentlichungstag der Anmeldung:
**12.12.90 Patentblatt 90/50**

(45) Bekanntmachung des Hinweises auf die Patenterteilung:
**14.09.94 Patentblatt 94/37**

(84) Benannte Vertragsstaaten:
**AT BE CH DE DK ES FR GB GR IT LI LU NL SE**

(56) Entgegenhaltungen:
**EP-A- 0 035 893**
**EP-A- 0 044 807**
**EP-A- 0 072 347**
**EP-A- 0 074 282**
**EP-A- 0 084 224**
**EP-A- 0 103 543**
**WO-A-88/04297**

**CHEMICAL ABSTRACTS, Band 102, Nr. 7, 18 FEbruar 1985, Columbus, Ohio, US, Zusammenfassung Nr. 62050z**

(73) Patentinhaber: **CIBA-GEIGY AG**
**Klybeckstrasse 141**
**CH-4002 Basel (CH)**

(72) Erfinder: **Föry, Werner, Dr.**
**Inzlingerstrasse 11**
**CH-4125 Riehen (CH)**
Erfinder: **Schurter, Rolf, Dr.**
**Holzmattstrasse 45**
**CH-4102 Binningen (CH)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue, herbizid wirksame und pflanzenwuchsregulierende N-Pyridinsulfonyl-N'-pyrimidinyl- und -triazinylharnstoffe, Verfahren zu ihrer Herstellung, sie als Wirkstoffe enthaltende Mittel, sowie deren Verwendung zum Bekämpfen von Unkräutern, vor allem selektiv in Nutzpflanzenkulturen oder zum Regulieren und Hemmen des Pflanzenwachstums.

Harnstoffverbindungen, Triazinverbindungen und Pyrimidinverbindungen mit herbizider Wirkung sind allgemein bekannt. Beispielsweise beschreibt das Europäische Patent Nr. 103 543 herbizid wirksame und pflanzenwuchsregulierende N-Pyridinsulfonyl-N'-pyrimidinyl- und -triazinylharnstoffe. Die dort offenbarten Wirkstoffe können jedoch die Anforderungen bezüglich Wirkungsstärke und Selektivität nicht immer erfüllen. Es besteht somit ein Bedarf nach besser wirksamen und selektiveren Wirkstoffen.

Es wurden nun neue Sulfonylharnstoffe mit verbesserten herbiziden und pflanzenwachstumsregulierenden Eigenschaften gefunden.

Die erfindungsgemässen N-Pyridinsulfonyl-N'-pyrimidinyl- und -triazinylharnstoffe entsprechen der Formel I

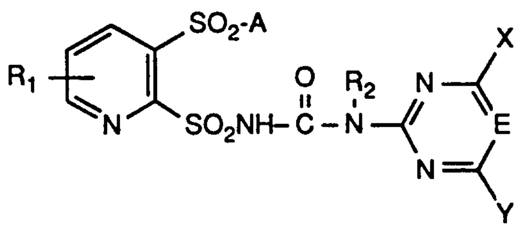 (I)

worin

$R_1$ Wasserstoff, Halogen, $C_1$-$C_4$-Alkyl, $C_1$-$C_4$-Alkoxy, $C_1$-$C_4$-Alkylthio, $C_1$-$C_4$-Halogenalkoxy oder $CF_3$;

$R_2$ Wasserstoff oder $C_1$-$C_4$-Alkyl;

A durch Halogen ein- bis dreifach substituiertes $C_1$-$C_6$-Alkyl oder $C_3$-$C_6$-Cycloalkyl oder durch $C_1$-$C_4$-Alkoxy, $C_1$-$C_4$-Alkylthio, $C_1$-$C_4$-Alkylsulfonyl, $C_1$-$C_4$-Alkylsulfinyl, $C_2$-$C_4$-Alkenyl, $C_5$-$C_6$-Cycloalkenyl, Amino, $C_1$-$C_4$-Alkylamino, $C_1$-$C_4$-Dialkylamino oder $C_2$-$C_4$-Alkinyl substituiertes $C_1$-$C_4$-Alkyl oder $C_3$-$C_6$-Cycloalkyl, wobei der $C_2$-$C_4$-Alkenylrest sowie der $C_5$-$C_6$-Cycloalkenylrest noch ein- bis dreifach durch Halogen substituiert sein kann; oder $C_1$-$C_4$-Alkyl welches durch einen 4- bis 6-gliedrigen gesättigten Heterocyclus, der ein Heteroatom ausgewählt aus der Gruppe O, N und $SO_2$ enthält, substituiert ist;

X $C_1$-$C_3$-Alkyl, durch Halogen ein- bis dreifach substituiertes $C_1$-$C_3$-Alkyl, $C_1$-$C_3$-Alkoxy oder durch Halogen ein- bis dreifach substituiertes $C_1$-$C_3$-Alkoxy;

Y Halogen, $C_1$-$C_3$-Alkyl, durch Halogen ein- bis dreifach substituiertes $C_1$-$C_3$-Alkyl, $C_1$-$C_3$-Alkoxy, durch Halogen ein- bis dreifach substituiertes $C_1$-$C_3$-Alkoxy, Cyclopropyl, Methylamino oder Dlinethylamino; und

E Stickstoff oder die Methingruppe bedeuten,

sowie die Salze dieser Verbindungen.

Die als oder in den Substituenten $R_1$, $R_2$ und A vorkommenden $C_1$-$C_4$-Alkylgruppen können geradkettig oder verzweigt sein und umfassen im einzelnen Methyl, Ethyl, n-Propyl, iso-Propyl, n-Butyl, sek.-Butyl, iso-Butyl und tert.-Butyl. Vorzugsweise besitzen die als oder in den Substituenten $R_1$, $R_2$ und A vorhandenen Alkylgruppen ein bis drei Kohlenstoffatome. Für die Substituenten $R_1$ und $R_2$ ist Methyl besonders bevorzugt.

Halogenalkoxy ist z.B. Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy; vorzugsweise Difluormethoxy, 2-Chlorethoxy und Trifluormethoxy.

Der Substituent A umfasst als ein- bis dreifach durch Halogen substituierte $C_1$-$C_6$-Alkylgruppe geradkettige oder verzweigte Alkylgruppen wie Methyl, Ethyl, n-Propyl, iso-Propyl, n-Butyl, sek.-Butyl, iso-Butyl, tert.-Butyl, n-Pentyl, die isomeren Pentyl, n-Hexyl sowie die isomeren Hexyl, die ein- bis dreifach durch Halogen substituiert sind, wobei Halogen im einzelnen Fluor, Chlor, Brom oder Jod bedeutet.

Unter diesen ein- bis dreifach durch Halogen substituierten $C_1$-$C_6$-Alkylgruppen sind einbis dreifach durch Halogen substituierte $C_1$-$C_4$-Alkylgruppen bevorzugt. Bevorzugte Halogenatome, die als Substituenten der $C_2$-$C_6$-Alkylgruppen auftreten können, sind Fluor und Chlor. Speziell bevorzugte ein- bis dreifach durch Halogen substituierte Alkylgruppen sind Trifluormethyl, 1-Fluorethyl, 1,1-Dichlorethyl, 3,3,3-Trifluorpropyl, 2-Fluorisopropyl, 3-Fluorpropyl, 1,1,1-Trichlorpenryl, 1-Fluor-3-methylpentyl oder 1-Bromhexyl. Ganz

besonders bevorzugt sind 3-Fluorpropyl und 2-Fluorisopropyl.

Die im Substituenten A vorkommenden $C_2$-$C_4$-Alkenylreste können in der Z-Form (cis) oder in der E-Form (trans) vorliegen und können geradkettig oder verzweigt sein. Bevorzugt sind Alkenylreste mit einer Kettenlänge von zwei bis drei Kohlenstoffatomen. Beispiele für $C_2$-$C_4$-Alkenylreste sind: Vinyl, Allyl, Methallyl, 1-Methylvinyl, But-2-en-1-yl. Bevorzugt ist Vinyl und Allyl. Bei den $C_2$-$C_4$-Alkenylresten, welche einbis dreifach durch Halogen substituiert sind, steht Halogen im einzelnen für Fluor, Chlor, Brom und Jod. Bevorzugte Halogenatome, die als Substituenten der $C_2$-$C_4$-Alkenylreste auftreten können, sind Fluor und Chlor. Unter den durch Halogen ein- bis dreifach substituierten $C_2$-$C_4$-Alkenylresten sind diejenigen bevorzugt, die eine Kettenlänge von zwei bis drei Kohlenstoffatomen besitzen. Speziell bevorzugte, durch Halogen ein- bis dreifach substituierte $C_2$-$C_4$-Alkenylreste sind 1-Chlorvinyl, 2-Chlorvinyl, 3-Fluorallyl und 4,4,4-Trifluor-but-2-en-1-yl. Ganz besonders bevorzugt sind 1-Chlorvinyl und 2-Chlorvinyl.

Die in den Definitionen des Substituenten A vorkommenden $C_2$-$C_4$-Alkinylreste können geradkettig oder verzweigt sein. Bevorzugt sind Alkinylreste mit einer Kettenlänge von 2 bis 3 Kohlenstoffatomen. $C_2$-$C_4$-Alkinylreste stehen beispielsweise für Ethinyl, Propargyl, 1-Propinyl, 3-Butinyl oder 1-Methylpropargyl, wobei Ethinyl und Propargyl besonders bevorzugt sind.

Die im Substituenten A vorkommenden durch Halogen ein- bis dreifach substituierte $C_3$-$C_6$-Cycloalkylgruppen umfassen beispielsweise 2-Fluorcyclopropyl, 2,3-Difluorcyclopropyl, 2,2-Dichlorcyclopropyl, 3-Bromcyclopropyl, 2,3,4-Trifluorcyclopenryl oder 2,3-Dichlorcyclohexyl. Von diesen $C_3$-$C_6$-Cycloalkylgruppen sind die ein- bis zweifach durch Halogen substituierte Cyclopropyl-, Cyclopentyl- und Cyclohexylgruppen bevorzugt wobei Halogen für Fluor, Chlor, Brom und Jod, insbesondere aber für Fluor und Chlor steht.

Weitere substituierte $C_3$-$C_6$-Cycloalkylgruppen sind beispielsweise 2-Methoxycyclopropyl, 3-Ethylthiocyclobutyl, 2-Methylsulfonylcyclopentyl, 3-Ethylsulfinylcyclohexyl, 4-Allyl-cyclohexyl oder 3-Propargylcyclohexyl.

Die $C_5$-$C_6$-Cycloalkenylreste können ein- bis dreifach durch Halogen substituiert sein. Bevorzugte Halogenatome sind dabei Fluor und Chlor. Beispiele für $C_5$-$C_6$-Cycloalkenylreste sind 3-Cyclopenten, 2-Cyclopenten, 4-Chlor-cyclopent-3-en, 3,4-Difluorcyclopent-3-en, 2-Cyclohexen, 3-Cyclohexen, 4,5-Dibromcyclohex-2-en oder 4,4,5-Trifluorcyclohex-2-en.

Beispiele für gesättigte 4- bis 6-gliedrige Heterocyclen sind Oxetan-3-yl, Oxolan-2-yl, Oxan-2-yl, Azetidin-3-yl, Azolidin-3-yl, Perhydroazin-4-yl (Piperidin-4-yl), Dioxothiolan-2-yl, Dioxothiethan-3-yl oder Dioxothian-4-yl. Das Stickstoffatom in Heterocyclus kann auch durch $C_1$-$C_4$-Alkyl substituiert sein wie beispielsweise in N-Methylperhydroazin-4-yl oder N-Isopropylazolidin-2-yl.

Die als oder in den Definitionen der Substituenten X und Y vorkommenden $C_1$-$C_3$-Alkylreste umfassen im einzelnen Methyl, Ethyl, n-Propyl und iso-Propyl sowie die von diesen Resten abgeleiteten ein- bis dreifach durch Halogen substituierten Halogenalkyle. Vorzugsweise besitzen die als oder in den Substituentendefinitionen X und Y vorkommenden Alkylreste ein bis zwei Kohlenstoffatome.

Bei den als oder in den Definitionen der Substituenten X und Y vorkommenden ein- bis dreifach durch Halogen substituierten $C_1$-$C_3$-Alkylgruppen sind ein- bis dreifach durch Fluor oder Chlor substituierte $C_1$-$C_2$-Alkylgruppen bevorzugt. Speziell bevorzugte ein- bis dreifach durch Halogen substituierte $C_1$-$C_3$-Alkylreste sind Trifluormethyl, Difluormethyl, 2-Chlorethyl, Chlordifluormethyl, Dichlormethyl, Chlorfluormethyl, 1,1-Dichlorethyl, Trifluorethyl, 3,3,3-Trifluorpropyl oder 2,3-Dichlorpropyl, besonders bevorzugt sind Fluormethyl, Chlormethyl, Difluormethyl und Trifluormethyl.

Die Erfindung umfasst ebenfalls die Salze, die die Verbindungen der Formel I mit Aminen, Alkali- und Erdalkalimetallbasen oder quaternären Ammoniumbasen bilden können.

Unter Alkali- und Erdalkalimetallhydroxiden als Salzbildner sind die Hydroxide von Lithium, Natrium, Kalium, Magnesium oder Calcium hervorzuheben, insbesondere aber die von Natrium oder Kalium.

Beispiele für zur Salzbildung geeignete Amine sind primäre, sekundäre und tertiäre aliphatische und aromatische Amine wie Methylamin, Ethylamin, Propylamin, iso-Propylamin, die vier isomeren Butylamine, Dimethylamin, Diethylamin, Diethanolamin, Dipropylamin, Diisopropylamin, Di-n-butylamin, Pyrrolidin, Piperidin, Morpholin, Trimethylamin, Triethylamin, Tripropylamin, Chinuclidin, Pyridin, Chinolin und iso-Chinolin, insbesondere aber Ethyl-, Propyl-, Diethyl- oder Triethylamin, vor allem aber iso-Propylamin und Diethanolamin.

Beispiele für quaternäre Ammoniumbasen sind im allgemeinen die Kationen von Halogenammoniumsalzen, z.B. das Tetramethylammoniumkation, das Trimethylbenzylammoniumkation, das Triethylbenzylammoniumkation, das Tetraethylammoniumkation, das Trimethylethylammoniumkation, aber auch das Ammoniumkation.

Unter den Verbindungen der Formel I sind diejenigen bevorzugt, in denen

$R_1$ Wasserstoff, Halogen, $C_1$-$C_4$-Alkyl, $C_1$-$C_4$-Alkoxy, $C_1$-$C_4$-Alkylthio oder $CF_3$;

$R_2$ Wasserstoff oder $C_1$-$C_4$-Alkyl;
A durch Halogen ein- bis dreifach substituiertes $C_2$-$C_6$-Alkyl; oder durch $C_1$-$C_4$-Alkoxy, $C_1$-$C_4$-Alkylthio, $C_1$-$C_4$-Alkylsulfonyl, $C_1$-$C_4$-Alkylsulfinyl, $C_2$-$C_4$-Alkenyl oder $C_2$-$C_4$-Alkinyl substituiertes $C_1$-$C_4$-Alkyl, wobei der $C_2$-$C_4$-Alkenylrest noch ein-bis dreifach durch Halogen substituiert sein kann;
X $C_1$-$C_3$-Alkyl, durch Halogen ein- bis dreifach substituiertes $C_1$-$C_3$-Alkyl, $C_1$-$C_3$-Alkoxy oder durch Halogen ein- bis dreifach substituiertes $C_1$-$C_3$-Alkoxy;
Y Halogen, $C_1$-$C_3$-Alkyl, durch Halogen ein- bis dreifach substituiertes $C_1$-$C_3$-Alkyl, $C_1$-$C_3$-Alkoxy, durch Halogen ein- bis dreifach substituiertes $C_1$-$C_3$-Alkoxy, Cyclopropyl, Methylamino oder Dimethylamino; und
E Stickstoff oder die Methingruppe bedeuten.

Ferner sind unter den Verbindungen der Formel I diejenigen bevorzugt, in denen
a) $R_2$ Wasserstoff bedeutet;
b) $R_1$ und $R_2$ Wasserstoff bedeuten;
c) $R_1$ für Wasserstoff, Methyl, Methoxy, Fluor oder Chlor steht;
d) X für $C_1$-$C_3$ Alkyl, $C_1$-$C_3$-Alkoxy oder durch Halogen ein- bis dreifach substituiertes $C_1$-$C_3$-Alkoxy; und Y für $C_1$-$C_3$-Alkyl, $C_1$-$C_3$-Alkoxy, durch Halogen ein- bis dreifach substituiertes $C_1$-$C_3$-Alkoxy oder für Chlor steht;
e) A $C_1$-$C_6$-Alkyl oder $C_3$-$C_6$-Cycloalkyl, welches ein- bis dreifach durch Halogen substituiert ist; oder $C_1$-$C_4$-Alkyl oder $C_3$-$C_6$-Cycloalkyl, welches durch $C_1$-$C_4$-Alkoxy, $C_5$-$C_6$-Cycloalkenyl oder $C_2$-$C_4$-Alkenyl substituiert ist, bedeutet; wobei der $C_2$-$C_4$-Alkenylrest sowie der $C_5$-$C_6$-Cycloalkenylrest noch ein- bis dreifach durch Halogen substituiert sein kann.

Von diesen Verbindungen sind diejenigen besonders hervorzuheben, in denen
a) A für Allyl, Methallyl, 3-Chlorallyl, Methoxyethyl, 2-Fluor-isopropyl oder 3-Fluorpropyl steht;
b) $R_1$ und $R_2$ Wasserstoff bedeuten;
c) X für Methyl, Methoxy, Ethoxy oder Difluormethoxy und Y für Methyl, Methoxy, Ethoxy, Difluormethoxy oder Chlor steht.

Als bevorzugte Untergruppe von Verbindungen der Formel I ist die Gruppe hervorzuheben, worin
$R_1$ Wasserstoff, Halogen, $C_1$-$C_2$-Alkyl, $C_1$-$C_2$-Alkoxy, $C_1$-$C_2$-Alkylthio, $C_1$-$C_2$-Halogenalkoxy oder $CF_3$;
$R_2$ Wasserstoff oder $C_1$-$C_2$-Alkyl;
A ein- bis dreifach durch Halogen substituiertes $C_1$-$C_4$-Alkyl oder $C_3$-$C_6$-Cycloalkyl, oder durch $C_1$-$C_2$-Alkoxy, $C_1$-$C_2$-Alkylthio, $C_1$-$C_2$-Alkylsulfonyl, $C_1$-$C_2$-Alkylsulfinyl, $C_2$-$C_3$-Alkenyl, $C_5$-$C_6$-Cycloalkenyl, Amino, $C_1$-$C_2$-Alkylamino, $C_1$-$C_4$-Dialkylamino oder $C_2$-$C_3$-Alkinyl substituiertes $C_1$-$C_2$-Alkyl oder $C_3$-$C_6$-Cycloalkyl, wobei der $C_2$-$C_3$-Alkenylrest sowie der $C_5$-$C_6$-Cycloalkenylrest noch ein- bis dreifach durch Halogen substituiert sein kann; oder $C_1$-$C_2$-Alkyl welches durch einen 4- bis 6-gliedrigen gesättigten Heterocyclus, der ein Heteroatom ausgewählt aus der Gruppe O, N und $SO_2$ enthält, substituiert ist,
X $C_1$-$C_2$-Alkyl, durch Halogen ein- bis dreifach substituiertes $C_1$-$C_2$-Alkyl, $C_1$-$C_2$-Alkoxy oder durch Halogen ein- bis dreifach substituiertes $C_1$-$C_2$-Alkoxy;
Y Halogen, $C_1$-$C_2$-Alkyl, durch Halogen ein- bis dreifach substituiertes $C_1$-$C_2$-Alkyl, $C_1$-$C_2$-Alkoxy, durch Halogen ein- bis dreifach substituiertes $C_1$-$C_2$-Alkoxy, Cyclopropyl, Methylamino oder Diinethylamino bedeuten.

Eine weitere Untergruppe von Verbindungen der Formel I ist dadurch gekennzeichnet, dass
$R_1$ Wasserstoff, Halogen, $C_1$-$C_2$-Alkyl, $C_1$-$C_2$-Alkylthio oder $CF_3$;
$R_2$ Wasserstoff oder $C_1$-$C_2$-Alkyl; A ein- bis dreifach durch Halogen substituiertes $C_2$-$C_4$-Alkyl oder durch $C_1$-$C_2$-Alkoxy, $C_1$-$C_2$-Alkylthio, $C_1$-$C_2$-Alkylsulfonyl, $C_1$-$C_2$-Alkylsulfinyl, $C_2$-$C_3$-Alkenyl oder $C_2$-$C_3$-Alkinyl substituiertes $C_1$-$C_2$-Alkyl, wobei der $C_2$-$C_3$-Alkenylrest noch ein- bis dreifach durch Halogen substituiert sein kann;
X $C_1$-$C_2$-Alkyl, durch Halogen ein- bis dreifach substituiertes $C_1$-$C_2$-Alkyl, $C_1$-$C_2$-Alkoxy, oder durch Halogen ein- bis dreifach substituiertes $C_1$-$C_2$-Alkoxy;
Y Halogen, $C_1$-$C_2$-Alkyl, durch Halogen ein- bis dreifach substituiertes $C_1$-$C_2$-Alkyl, $C_1$-$C_2$-Alkoxy, durch Halogen ein- bis dreifach substituiertes $C_1$-$C_2$-Alkoxy, Cyclopropyl, Methylamino oder Dimethylamino bedeuten.

Als weitere bevorzugte Untergruppe von Verbindungen der Formel I ist die Gruppe hervorzuheben, worin
$R_1$ und $R_2$ Wasserstoff;
A durch Halogen ein- bis dreifach substituiertes $C_1$-$C_4$-Alkyl oder durch $C_1$-$C_4$-Alkoxy, $C_5$-$C_6$-Cycloalkenyl oder $C_1$-$C_2$-Alkenyl substituiertes $C_1$-$C_2$-Alkyl oder $C_3$-$C_6$-Cycloalkyl, wobei der $C_2$-$C_3$-Alkenylrest sowie der $C_5$-$C_6$-Cycloalkenylrest noch ein- bis dreifach durch Fluor oder Chlor substituiert sein kann; bedeutet.

Von dieser Gruppe fallen durch ihre gute biologische Wirkung besonders diejenigen Verbindungen ins Auge, in denen

A für Allyl, Methallyl, 3-Chlorallyl, Methoxyethyl, 2-Fluor-isopropyl oder 3-Fluorpropyl;
X für Methyl, Methoxy, Ethoxy oder Difluormethoxy, Y für Methyl, Methoxy, Ethoxy, Difluormethoxy oder Chlor und
E für die Methingruppe steht.

Als bevorzugte Einzelverbindungen aus dem Umfang der Formel I sind zu nennen:
N-(3-Allylsulfonyl-pyrdin-2-yl-sulfonyl)-N'-(4,6-dimethoxypyrimidin-2-yl)-harnstoff, N-(3-Methoxyethylsulfonyl-pyridin-2-yl-sulfonyl)-N'-(4,6-dimethoxypyrimidin-2-yl)-harn stoff, N-(3-Fluorpropylsulfonyl-pyridin-2-yl-sulfonyl)-N'-(4,6-dimethoxypyrimidin-2-yl)-harnstoff und N-(3-Methallylsulfonylpyridin-2-yl-sulfonyl)-N'-(4,6-dimethoxypyrimidin-2-yl)-harnstoff.

Nach einem ersten Verfahren können die Verbindungen der Formel I hergestellt werden, indem man ein Pyridylsulfonamid der Formel II

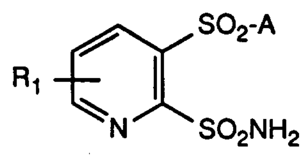

(II)

worin $R_1$ und A die unter Formel I angegebene Bedeutung haben mit der Massgabe, dass A nicht $\alpha$-Halogenalkyl oder $\alpha$-Halogencycloalkyl sein kann, in Gegenwart einer Base mit einem N-Pyrimidinyl- oder N-Triazinylcarbamat der Formel IV

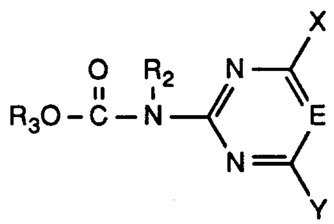

(IV)

worin X, Y, $R_2$ und E die unter Formel I angegebene Bedeutung haben und $R_3$ $C_1$-$C_4$-Alkyl oder Phenyl, welches durch $C_1$-$C_4$-Alkyl oder Halogen substituiert sein kann, bedeutet; umsetzt.

Nach einem zweiten Verfahren können die Verbindungen der Formel I hergestellt werden, indem man ein Pyridylsulfonamid der Formel II

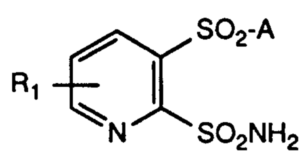

(II)

worin $R_1$ und A die unter Formel I angegebene Bedeutung haben, in Gegenwart einer Base mit einem Pyrimidinyl- oder Triazinylisocyanat der Formel VIII

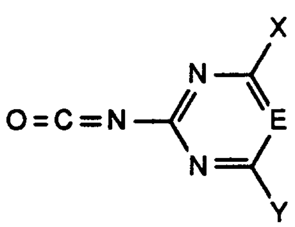

(VIII)

worin E, X und Y die unter Formel I angegebene Bedeutung haben, umsetzt.

Nach einem dritten Verfahren können die Verbindungen der Formel I hergestellt werden, indem man aus dem Europäischen Patent No. 103 543 bekannte Verbindungen der Formel XVI

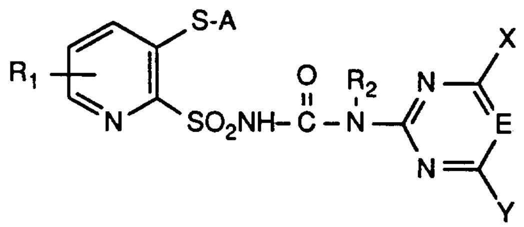

(XVI)

worin $R_1$, $R_2$, A, E, X und Y die unter Formel I angegebene Bedeutung haben, nach an sich bekannten Verfahren oxidiert.

Oxidationen von Thioverbindungen zu den entsprechenden Sulfonylverbindungen sind im U.S. Patent Nr. 4,774,337 beschrieben. Für diese Reaktion geeignete Oxidationsmittel sind beispielsweise Persäuren oder Wasserstoffperoxid. Die erhaltenen Harnstoffe der Formel I können gewünschtenfalls mittels Aminen, Alkalimetall- oder Erdalkalimetallhydroxiden oder quaternären Ammoniumbasen in Salze überführt werden. Dieses geschieht beispielsweise durch Umsetzen mit der äquimolaren Menge Base und Verdampfen des Lösungsmittels.

Die Umsetzungen zu Verbindungen der Formel I werden vorteilhalterweise in aprotischen, inerten organischen Lösungsmitteln vorgenommen. Solche Losungsmittel sind Kohlenwasserstoffe wie Benzol, Toluol, Xylol oder Cyclohexan, chlorierte Kohlenwasserstoffe wie Dichlormethan, Trichlormethan, Tetrachlormethan oder Chlorbenzol, Ether wie Diethylether, Ethylenglykoldimethylether, Diethylenglykoldimethylether, Tetrahydiofuran oder Dioxan, Nitrile wie Acetonitril oder Propionitril, Amide wie Dimethylformamid, Diäthylformamid oder N-Methylpyrrolidinon. Die Reaktionstemperaturen liegen vorzugsweise zwischen -20° und +120°C. Die
Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base als Reaktionskatalysator verkürzt werden. Als Basen sind insbesondere tertiäre Amine wie Trimethylamin, Triethylamin, Chinuclidin, 1,4-Diazabicyclo-(2.2.2)-octan, 1,5-Diazabicyclo(4.3.0)non-5-en oder 1,5-Diazabicyclo(5.4.0)undec-7-en geeignet. Als Basen können aber auch anorganische Basen wie Hydride wie Natrium- oder Calciumhydrid, Hydroxide wie Natrium- und Kaliumhydroxid, Carbonate wie Natrium- und Kaliumcarbonat oder Hydrogencarbonate wie Kalium- und Natriumhydrogencarbonat verwendet werden.

Die Endprodukte der Formel I können durch Einengen und/oder Verdampfen des Lösungsmittels isoliert und durch Umkristallisieren oder Zerreiben des festen Rückstandes in Lösungsmitteln, in denen sie sich nicht gut lösen, wie Ether, aromatischen Kohlenwasserstoffen oder chlorierten Kohlenwasserstoffen, gereinigt werden.

Die Zwischenprodukte der Formel II sind neu und wurden speziell für die Synthese der Verbindungen der Formel I entwickelt. Sie bilden daher einen Bestandteil der vorliegenden Erfindung.

Die neuen Zwischenprodukte der Formel II können nach verschiedenen Verfahren hergestellt werden. So erhält man die Verbindungen der Formel IIc

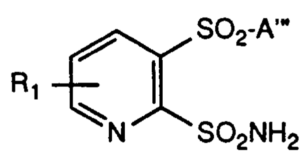

(IIc)

worin $R_1$ die unter Formel I angegebene Bedeutung hat und A''' durch $C_2$-$C_4$-Alkenyl, $C_5$-$C_6$-Cycloalkenyl, $C_2$-$C_4$-Alkinyl, $C_1$-$C_4$-Alkoxy, $C_1$-$C_4$-Alkylthio, $C_1$-$C_4$-Alkylsulfonyl oder $C_1$-$C_4$-Alkylsulfinyl substituiertes $C_1$-$C_4$-Alkyl oder $C_3$-$C_6$-Cycloalkyl oder durch Fluor substituiertes $C_1$-$C_6$-Alkyl oder $C_3$-$C_6$-Cycloalkyl bedeutet, indem man ein 3-Fluorpyridin-2-ylsulfonamid der Formel IIIa

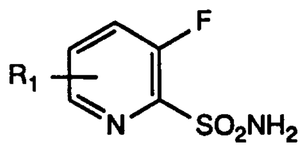

(IIIa)

worin $R_1$ die unter Formel I angegebene Bedeutung hat, in Gegenwart einer Base mit einem Mercaptan der Formel V

A'''-SH (V)

worin A''' die unter Formel IIc angegebene Bedeutung hat, umsetzt, und anschliessend die so erhaltene Verbindung der Formel VI

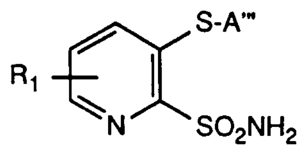

(VI)

worin $R_1$ die unter Formel I angegebene Bedeutung hat und A''' die unter Formel IIc angegebene Bedeutung hat, nach an sich bekannten Verfahren zur Verbindung der Formel IIc oxidiert.

Zwischenprodukte der Formel II

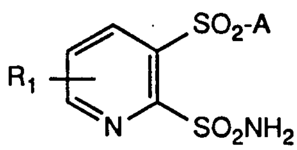

(II)

worin $R_1$ und A die unter Formel I angegebene Bedeutung haben, werden auch erhalten, indem man ein 3-Mercapto-pyridin-2-ylsulfonamid der Formel IIIb

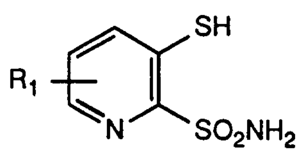

(IIIb)

worin $R_1$ die unter Formel I angegebene Bedeutung hat, in Gegenwart einer Base mit einer Verbindung der Formel XVIII

Z-A (XVIII)

worin A die unter Formel I angegebene Bedeutung hat und Z für Chlor, Brom, Jod, $CH_3SO_2O$- oder

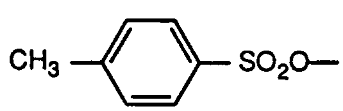

steht, zur Verbindung der Formel XX

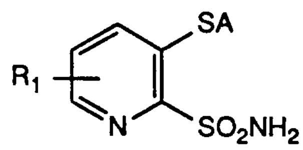

(XX)

worin A die unter Formel I angegebene Bedeutung hat, umsetzt und diese anschliessend zur Verbindung der Formel II oxidiert.

Die Zwischenprodukte der Formel III

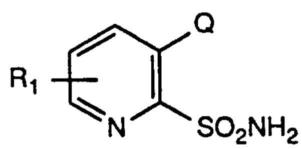

(III)

worin $R_1$ die unter Formel I angegebene Bedeutung hat und Q für Fluor oder -SH steht sind, mit Ausnahme der Verbindung in der $R_1$ Wasserstoff und Q -SH bedeutet, neu und bilden ebenfalls einen Gegenstand der vorliegenden Erfindung. Unter den Umfang der Formel III fallen die Verbindungen der Unterformeln IIIa und IIIb.

Die Verbindungen der Formeln IIIa und IIIb können nach an sich bekannten Methoden hergestellt werden, indem man im Falle der Verbindung der Formel IIIa ein 2,3-Difluorpyridin der Formel IX

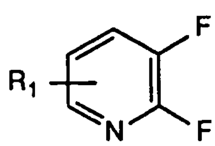

(IX)

worin $R_1$ die unter Formel I angegebene Bedeutung hat, mit einem Mercaptan der Formel X

$R_4SH$ (X)

worin $R_4$ für Benzyl oder iso-Propyl steht, zu einer Verbindung der Formel XI

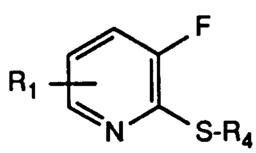

(XI)

worin $R_1$ die unter Formel I angegebene Bedeutung hat und $R_4$ für Benzyl oder iso-Propyl steht, umsetzt, um anschliessend durch Chlorierung mit Chlorgas und Umsetzen mit Ammoniak die Verbindung der Formel IIIa zu erhalten.

Verbindungen der Formel IIIa können auch hergestellt werden, indem man ein 3-Aminopyridin-2-yl-sulfonamid der Formel XXI

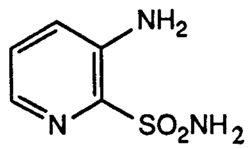

(XXI)

in Gegenwart von Fluorwasserstoff und gegebenenfalls in Gegenwart eines inerten Lösungsmittels mit einem Nitrit diazotiert.

Geeignete Nitrite sind beispielsweise Natriumnitrit oder Kaliumnitrit, als Lösungsmittel sind Dimethylsulfoxid, Sulfolan, Dimethylformamid oder Dimethylacetamid besonders gut geeignet.

Zwischenprodukte der Formel IIIb werden hergestellt, indem man ein 3-Fluorpyridin-2-ylsulfonamid der Formel IIIa mit einem Mercaptid der Formel XIX

$(Me)_nH_2\text{-}nS^n$ (XIX)

worin Me für Natrium, Kalium oder Lithium steht und n 1 oder 2 bedeutet, umsetzt.

Verfahren zur Herstellung der Verbindungen der Formel IX finden sich beispielsweise in J. Fluorine Chem 21, 171 (1982) ($R_1$ = H); USP 4,713,109 ($R_1$ = Cl) oder in Advanc. Heterocycl. Chem. 6, 229 (1966).

Verbindungen der Unterformel IIIa

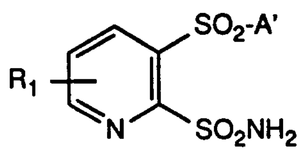

(IIa)

worin $R_1$ die unter Formel I angegebene Bedeutung hat und A' durch Fluor substituiertes $C_1$-$C_6$-Alkyl oder $C_3$-$C_6$-Cycloalkyl oder durch $C_1$-$C_4$-Alkoxy oder $C_1$-$C_4$-Alkylthio substituiertes $C_1$-$C_4$-Alkyl oder $C_3$-$C_6$-Cycloalkyl bedeutet, können auch analog eines in USP 4,774,337 beschriebenen Verfahrens hergestellt werden, indem man eine Verbindung der Formel XII

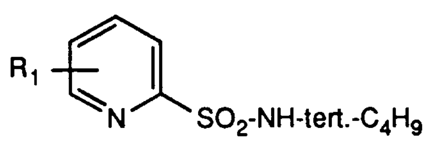

(XII)

worin $R_1$ die unter Formel I angegebene Bedeutung hat, in Gegenwart einer starken Base wie beispielsweise n-Butyllithium oder Lithiumdiisopropylamid, mit einem Disulfid der Formel XIII

'A-S-S-A' (XIII)

worin A' die unter Formel IIIa angegebene Bedeutung hat, umsetzt, die so erhaltene Verbindung der Formel XIV

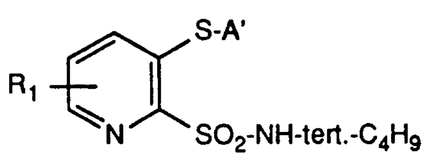

(XIV)

worin $R_1$ die unter Formel I angegebene Bedeutung hat und A' die unter Formel IIa angegebene Bedeutung hat, mit einer geeigneten Säure, wie beispielsweise Trifluoressigsäure, behandelt und das so erhaltene Sulfonamid der Formel XV

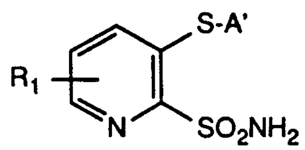

(XV)

worin $R_1$ die unter Formel I angegebene Bedeutung hat und A' die unter Formel IIa angegebene Bedeutung hat, anschliessend zur Verbindung der Formel II oxidiert.

Die Zwischenprodukte der Unterformel IIb

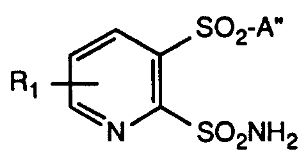

(IIb)

worin $R_1$ die unter Formel I angegebene Bedeutung hat und A" für durch Fluor, Chlor oder Brom substituiertes $C_2$-$C_4$-Alkyl steht, werden ferner erhalten, indem man ein Hydroxysulfonamid der Formel XVII

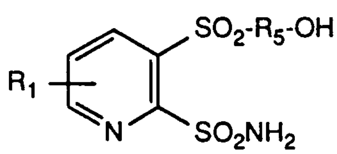

(XVII)

worin $R_1$ die unter Formel I angegebene Bedeutung hat und $R_5$ für $C_2$-$C_4$-Alkylen steht, in die entsprechenden Halogenide umwandelt Solche Umsetzungen sind beispielsweise in Can. J. Chem. 57, 3193 (1979) oder in der französischen Patentschrift Nr. 2 509 725 beschrieben.

Die Herstellung der Mercaptane der Formeln V und X sowie des Disulfids der Formel XIII erfolgt nach literaturbekannten Methoden. Aus der grossen Zahl der publizierten Artikel seien stellvertretend genannt:
DE 2 832 977;
J. Am. Chem. Soc. 77, 285 (1955);
J. Am. Chem. Soc. 72, 1687 (1950) und
Org. Synth. 35, 66 (1955).

Die Wirkstoffe der Formel I werden in der Regel mit Aufwandmengen von 0,001 bis 5 kg/ha insbesondere 0,005 bis 3 kg/ha erfolgreich eingesetzt. Die für die erwünschte Wirkung erforderliche Dosierung kann durch Versuche ermittelt werden. Sie ist abhängig von der Art der Wirkung, dem Entwicklungsstadium der Kulturpflanze und des Unkrauts sowie von der Applikation (Ort, Zeit, Verfahren) und kann, bedingt durch diese Parameter, innerhalb weiter Bereiche variieren.

Bei geringeren Aufwandmengen zeichnen sich die Verbindungen der Formel I durch wuchshemmende und herbizide Eigenschaften aus, die sie ausgezeichnet zum Einsatz in Kulturen von Nutzpflanzen, insbesondere in Getreide, Baumwolle, Soja, Raps, Mais und Reis befähigen, wobei der Einsatz in Maiskultur ganz besonders bevorzugt ist.

Die Erfindung betrifft auch herbizide und pflanzenwachstumsregulierende Mittel, welche einen neuen Wirkstoff der Formel I enthalten, sowie Verfahren zur Hemmung des Pflanzenwuchses.

Pflanzenwachstumsregulatoren sind Substanzen, die in/an der Pflanze agronomisch erwünschte biochemische und/oder physiologische und/oder morphologische Veränderungen bewirken.

Die in den erfindungsgemässen Mitteln enthaltenen Wirkstoffe beeinflussen das Pflanzenwachstum je nach Applikationszeitpunkt, Dosierung, Applikationsart und Umweltbedingungen in verschiedener Weise. Pflanzenwuchsregulatoren der Formel I können zum Beispiel das vegetative Wachstum von Pflanzen hemmen. Diese Art der Wirkung ist von Interesse auf Rasenflächen, im Zierpflanzenbau, in Obstplantagen, an Strassenböschungen, auf Sport- und Industrieanlagen, aber auch bei der gezielten Hemmung von Nebentrieben wie bei Tabak. Im Ackerbau führt die Hemmung des vegetativen Wachstums bei Getreide über eine Halmverstärkung zu reduziertem Lager, ähnliche agronomische Wirkungen erreicht man in Raps, Sonnenblumen, Mais und anderen Kulturpflanzen. Des weiteren kann durch Hemmung des vegetativen

Wachstums die Anzahl Pflanzen pro Fläche erhöht werden. Ein weiteres Einsatzgebiet von Wuchshemmern ist die selektive Kontrolle von bodendeckenden Pflanzen in Plantagen oder weitreihigen Kulturen durch starke Wuchshemmung, ohne diese Bodendecker abzutöten, sodass die Konkurrenz gegenüber der Hauptkultur ausgeschaltet ist, die agronomisch positiven Effekte wie Erosionsverhinderung, Stickstoffbindung und Bodenlockerung aber erhalten bleiben.

Unter einem Verfahren zur Hemmung des Pflanzenwachstums ist eine Steuerung der natürlichen Pflanzenentwicklung zu verstehen, ohne den von genetischen Eigenschaften determinierten Lebenszyklus der Pflanze im Sinne einer Mutation zu verändern. Das Verfahren der Wuchsregulierung wird zu einem im Einzelfall zu bestimmenden Entwicklungszeitpunkt der Pflanze angewendet. Die Applikation der Wirkstoffe der Formel I kann vor oder nach dem Auflaufen der Pflanzen erfolgen, beispielsweise bereits auf die Samen oder die Sämlinge, auf Wurzeln, Knollen, Stengel, Blätter, Blüten oder andere Pflanzenteile. Dies kann z.B. durch Aufbringen des Wirkstoffes selbst oder in Form eines Mittels auf die Pflanzen und/oder durch Behandlung des Nährmediums der Pflanze (Erdboden) geschehen.

Für die Verwendung der Verbindung der Formel I oder sie enthaltender Mittel zur Regulierung des Pflanzenwachstums kommen verschiedene Methoden und Techniken in Betracht, wie beispielsweise die folgenden:

i) <u>Samenbeizung</u>

a) Beizung der Samen mit einem als Spritzpulver formulierten Wirkstoff durch Schütteln in einem Gefäss bis zur gleichmässigen Verteilung auf der Samenoberfläche (Trockenbeizung). Man verwendet dabei bis zu 4 g Wirkstoff der Formel I (bei einer 50 %igen Formulierung: bis zu 8,0 g Spritzpulver) pro 1 kg Saatgut.

b) Beizung der Samen mit einem Emulsionskonzentrat des Wirkstoffs oder mit einer wässrigen Lösung des als Spritzpulver formulierten Wirkstoffs der Formel I nach Methode a) (Nassbeizung).

c) Beizung durch Tauchen des Saatguts in einer Brühe mit bis zu 1000 ppm Wirkstoff der Formel I während 1 bis 72 Stunden und gegebenenfalls nachfolgendes Trocknen der Samen (Tauchbeizung, Seed soaking).

Die Beizung des Saatguts oder die Behandlung des angekeimten Sämlings sind naturgemäss die bevorzugten Methoden der Applikation, weil die Wirkstofffbehandlung vollständig auf die Zielkultur gerichtet ist. Man verwendet in der Regel 4,0 g bis 0,001 g Aktivsubstanz pro 1 kg Saatgut, wobei man je nach Methodik, die auch den Zusatz anderer Wirkstoffe oder Mikronährstoffe ermöglicht, von den angegebenen Grenzkonzentrationen nach oben oder unten abweichen kann (Wiederholungsbeize).

ii) <u>Kontrollierte Wirkstoffabgabe</u>

Der Wirkstoff wird in Lösung auf mineralische Granulatträger oder polymerisierte Granulate (Harnstoff/Formaldehyd) aufgezogen und trocknen gelassen. Gegebenenfalls kann ein Ueberzug aufgebracht werden (Umhüllungsgranulate), der es erlaubt, den Wirkstoff über einen bestimmten Zeitraum dosiert abzugeben.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen, in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermählen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether, Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäure-methyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfate oder -sulfonate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykolethergruppen und 10 bis 100 Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyethylenglykolether, Polypropylen-polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben:

"Mc Cutcheon's Detergents and Emulsifiers Annual" MC Publishing Corp., Ridgewood, New Jersey, 1981;
H. Stache, "Tensid-Taschenbuch", 2. Aufl., C. Hanser Verlag, München, Wien, 1981;
M. and J. Ash. "Encyclopedia of Surfactants", Vol. I-III, Chemical Publishing Co., New York, 1980-1981.

Die agrochemischen Zubereitungen enthalten in der Regel 0,1 bis 95 %, insbesondere 0,1 bis 80 % Wirkstoff der Formel I, 1 bis 99,9 % eines festen oder flüssigen Zusatzstoffes und 0 bis 25 %, insbesondere 0,1 bis 25 % eines Tensides.

Insbesondere setzen sich bevorzugte Formulierungen folgendermassen zusammen: (% = Gewichtsprozent).

| Emulgierbare Konzentrate: | |
|---|---|
| Aktiver Wirkstoff: | 1 bis 20%, bevorzugt 5 bis 10 % |
| oberflächenaktive Mittel: | 5 bis 30%, vorzugsweise 10 bis 20 % |
| flüssiges Trägermittel: | 50 bis 94 %, vorzugsweise 70 bis 85 % |

| Stäubemittel: | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

| Suspensions-Konzentrate: | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

| Benetzbares Pulver: | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 99 %, vorzugsweise 15 bis 90 % |

| Granulate: | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 %. |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,001 bis 5 kg AS/ha, vorzugsweise 0,005 bis 3kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Formulierungsbeispiele:

Beispiel F 1: Formulierungsbeispiele für Wirkstoffe der Formel I (% = Gewichtsprozent)

a)

| Spritzpulver | a) | b) | c) |
|---|---|---|---|
| Wirkstoff Nr. 1.001 | 20 % | 50 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyethylenglykoläther (7-8 Mol AeO) | - | 2 % | 2 % |
| Hochdisperse Kieselsäure | 5 % | 27 % | 27 % |
| Kaolin | 67 % | 10 % | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b)

| Emulsions-Konzentrat | a) | b) |
|---|---|---|
| Wirkstoff Nr. 1.001 | 10 % | 1 % |
| Octylphenolpolyethylenglykolether (4-5 Mol AeO) | 3 % | 3 % |
| Ca-Dodecylbenzolsulfonat | 3 % | 3 % |
| Ricinusölpolyglykolether (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c)

| Stäubemittel | a) | b) |
|---|---|---|
| Wirkstoff Nr. 1.002 | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird

d)

| Extruder Granulat | a) | b) |
|---|---|---|
| Wirkstoff Nr. 1.001 | 10 % | 1% |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e)

| Umhüllungs-Granulat | |
|---|---|
| Wirkstoff Nr. 1.001 | 3 % |
| Polyethylenglykol (MG 200) | 3 % |
| Kaolin | 94 % |

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

f)

| Suspensions-Konzentrat | a) | b) |
|---|---|---|
| Wirkstoff Nr. 1.002 | 40 % | 5 % |
| Ethylenglykol | 10 % | 10 % |
| Nonylphenolpolyethylenglykolether (15 Mol AeO) | 6 % | 1 % |
| Na-Ligninsulfonat | 10 % | 5 % |
| Carboxymethylcellulose | 1 % | 1 % |
| 37 %ige wässrige Formaldehyde-Lösung | 0,2 % | 0,2 % |
| Silikonöl in Form einer 75 %igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g)

| Salzlösung | |
|---|---|
| Wirkstoff Nr. 1.002 | 5 % |
| Isopropylamin | 1 % |
| Octylphenolpolyethylenglykolether (78 Mol AeO) | 3 % |
| Wasser | 91 % |

Herstellungsbeispiele:

Beispiel H1: Herstellung von 2-Isopropylthio-3-fluorpyridin (Zwischenprodukt):

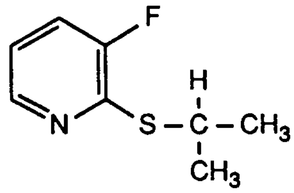

Zu einer Suspension von 324 g wasserfreiem Kaliumcarbonat und 335,6 g 2,3-Difluorpyridin in 4500 ml Dimethylformamid gibt man bei einer Temperatur von 0 bis + 2°C innerhalb von 25 Minuten 181,2 g Isopropylmercaptan tropfenweise hinzu. Nach 18-stündigem Rühren bei Raumtemperatur giesst man das Reaktiongemisch in eine Mischung aus 8000 ml Eiswasser und 4000 ml Essigsäureethylester. Anschliessend wird die organische Phase abgetrennt und jeweils mit Eiswasser und Essigsäureethylester gewaschen.

Die vereinigten organischen Phasen werden mit Natriumsulfat getrocknet und anschliessend eingedampft. Nach säulenchromatographischer Reinigung über Kieselgel-Essigsäureethylester/n-Hexan (1:9) erhält man 362,9 g 2-Isopropylthio-3-fluor-pyridin in Form eines Oeles.

Beispiel H2: Herstellung von 3-Fluor-pyridin-2-yl-sulfonamid (Verbindung Nr. 3.001):

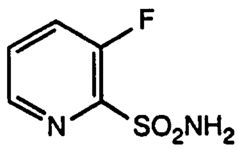

Variante a):

In eine Mischung aus 21,4 g 2-Isopropylthio-3-fluorpyridin, 340 ml Dichlormethan und 500 ml 1 N Salzsäure leitet man bei einer Temperatur von -5 °C innerhalb von 36 Minuten 36,9 g gasförmiges Chlor ein. Anschliessend wird die Mischung 30 Minuten lang bei einer Temperatur von 0 °C gerührt, wobei während der letzten 10 Minuten intensiv Stickstoff durch die Lösung geleitet wird. Die organische Phase wird abgetrennt, jeweils mit Eiswasser und Dichlormethan gewaschen und über Natriumsulfat getrocknet.

Anschliessend gibt man die vereinigten organischen Phasen bei einer Temperatur von -75 °C bis -50 °C innerhalb von 25 Minuten zu einer Mischung aus 31,8 g Ammoniak gelöst in 63,6 ml Dichlormethan tropfenweise hinzu. Nach Entfernen der Kühlung wird die Reaktionsmischung 150 Minuten lang gerührt und anschliessend filtriert. Nach Eindampfen und chromatographischer Reinigung über Kieselgel-Essigsäureethylester/n-Hexan (1:1) erhält man 11,1 g 3-Fluor-pyridin-2-yl-sulfonamid (Verbindung Nr. 3.001) mit einem Schmelzpunkt von + 124 bis + 125 °C.

Variante b):

5,3 g (0,030 Mol) 3-Amino-pyridin-2-yl-sulfonamid werden in 12 ml Dimethylsulfoxid gelöst und bei 0 °C zu 14,3 g (0,72 mol) Fluorwasserstoff getropft (exotherme Reaktion). Dann werden bei 0 °C 2,4g (0,035 Mol) Natriumnitrit portionenweise eingetragen. Nach einer Stunde wird auf 70 °C erwärmt ($N_2$-Entwicklung).
Nach dem Abkühlen wird das Reaktionsgemisch auf eine Eis/Wasser-Mischung gegeben und nach Zugabe von Natriumbikarbonat und Natriumchlorid mit Tetrahydrofuran und Essigsäureethylester extrahiert. Die vereinigten Extrakte werden mit gesättigter Natriumchloridlösung gewaschen, mit Magnesiumsulfat getrocknet, filtriert und evaporiert. Der Rückstand wird in Diethylether/Petrolether verrührt. Nach der Filtration erhält man 3,7 g 3-Fluorpyridin-2-yl-sulfonamid (Verbindung Nr. 3.001) mit einem Schmelzpunkt von +124 bis +125 °C.

Beispiel H3: Herstellung von 3-Mercapto-pyridin-2-yl-sulfonamid (Verbindung Nr. 3.008):

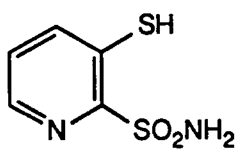

In eine Mischung aus 6,88 g Kalium-tert.-butylat und 5 g 3-Fluorpyridin-2-yl-sulfonamid in 90 ml Dimethylformamid leitet man unter Stickstoffatmosphäre bei einer Temperatur von +16 bis +20 °C 1,06 g Schwefelwasserstoff ein. Nach 4½-stündigem Rühren bei einer Temperatur von +50 °C gibt man 0,32 g Kalium-tert.-butylat hinzu und leitet 0,1 g Schwefelwasserstoff ein. Nach 2-stündigem Rühren bei einer Temperatur von +50 °C wird die Reaktionsmischung im Vakuum bei einer Temperatur von +75 °C eingeengt. Der so erhaltene ölige Rückstand wird mit 135 ml kalter, 0,75 N Schwefelsäure verrieben. Nach Filtration und Waschen des Rückstandes mit wenig Wasser wird das erhaltene Produkt im Vakuum über

Phosphorpentoxid getrocknet. Man erhält 3,58 g 3-Mercapto-pyridin-2-yl-sulfonamid mit einem Schmelzpunkt von +102 bis +104 °C.

Beispiel H4: Herstellung von 3-(3-Fluorpropylthio)-pyridin-2-yl-sulfonamid (Zwischen-produkt der Formel XX)

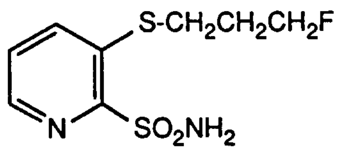

(XX)

Eine Mischung bestehend aus 0,285 g 3-Mercaptopyridin-2-yl-sulfonamid, 0,455 g Kaliumcarbonat, 5 ml trockenem Dimethylformamid und 0,23 g 3-Fluor-1-Brompropanwird bei einer Temperatur von +25 °C 90 Minuten lang in einer Stickstoffatmosphäre gerührt. Anschliessend wird die Reaktionsmischung in eine Eiswassermischung gegeben. Nach Einstellen des pH-Wertes auf pH2 mit 2,8 ml 2 N HCl wird die Reaktionsmischung zweimal mit Essigsäureethylester extrahiert. Die vereinigten organischen Phasen werden dreimal mit Wasser gewaschen und anschliessend über Natriumsulfat getrocknet. Nach Eindampfen und anschliessender chromatographischer Reinigung des Rückstandes über Kieselgel-Essigsäureethylester/n-Hexan (1:1) erhält man 0,12 g 3-(3-Fluorpropylthio)-pyridin-2-yl-sulfonamid mit einem Schmelzpunkt von +85 bis +86 °C.

Beispiel H5: Herstellung von 3-Allylthio-pyridin-2-yl-sulfonamid:

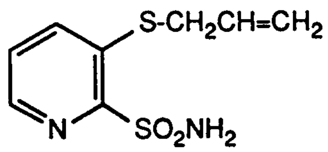

Zu einer auf + 5 °C gekühlten Lösung aus 17,62 g (0,1 Mol) 3-Fluor-pyridin-2-yl-sulfonamid in 400 ml Acetonitril gibt man 33,2 g (0,24 Mol) Kaliumcarbonat, sowie 1,32 g (0,005 Mol) 18-Crown-6 und 15,8 g (0,17 Mol) 80%iges Allylmercaptan in 50 ml Acetonitril hinzu.

Nach 46-stündigem Rühren der Reaktionsmischung bei Raumtemperatur wird der entstandene Niederschlag abfiltriert. Das erhaltene Filtrat wird eingeengt und über einer Kieselgelsäule mit Essigsäureethylester/Cyclohexan (2:1) chromatographisch gereinigt. Man erhält 10g (50 % d.Th.) 3-Allylthio-pyridin-2-yl-sulfonamid.

Beispiel H6: Herstellung von 3-Allylsulfonyl-pyridin-2-yl-sulfonamid (Verbindung Nr. 2.001):

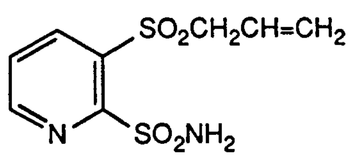

Zu einer auf + 8 °C gekühlten Lösung aus 10 g (0,050 Mol) eines gemäss Beispiel H5 erhaltenen 3-Allylthio-pyridin-2-yl-sulfonamids in 130 ml Essigsäure lässt man 62,5 g 40 %ige Peressigsäure zutropfen. Anschliessend wird das Reaktionsgemisch 19 Stunden lang bei einer Temperatur von + 8 bis + 30 °C gerührt. Anschliessend wird das Reaktionsgemisch auf Eiswasser gegossen und viermal mit Essigsäureethylester extrahiert. Die vereinigten organischen Phasen werden mit Natriumhydrogencarbonatlösung gewaschen, mit Kaliumsulfit behandelt und anschliessend mit Magnesiumsulfat getrocknet. Nach Abfiltrieren und Eindampfen des Filtrats wird der erhaltene Rückstand in Essigsäureethylester/n-Hexan umkristallisiert und anschliessend filtriert.

Nach dem Trocknen des Filterrückstandes im Vakuum bei Raumtemperatur erhält man 8,1 g (62 % d.Th.) 3-Allylsulfonyl-pyridin-2-yl-sulfonamid (Verbindung Nr. 2.001) mit einem Schmelzpunkt von + 178 °C.

Beispiel H7: Herstellung von N-(3-Allylsulfonyl-pyridin-2-yl-sulfonyl)-N'-(4,6-dimethoxypyrimidin-2-yl)-harnstoff (Verbindung Nr. 1.001):

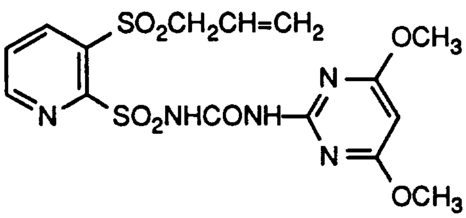

Zu einer auf + 5 °C gekühlten Lösung aus 7,1 g (0,027 Mol) eines gemäss Beispiel H6 erhaltenen 3-Allylsulfonyl-pyridin-2-yl-sulfonamides in 90 ml Acetonitril gibt man innerhalb von 10 Minuten tropfenweise eine Lösung aus 4,66 g (0,029 Mol) 1,5-Diazabicyclo [5.4.0] undec-5-en in 15 ml Acetonitril hinzu. Nach Zugabe von 7,8 g N-(4,6-Dimethoxy-pyrimidin-2-yl)-phenylcarbamat wird das Reaktionsgemisch 2 1/2 Stunden gerührt. Nach Abdestillieren des Acetonitrils unter vermindertem Druck bei einer Temperatur von + 45 °C wird der erhaltene Rückstand mit einer kleinen Menge Eis versetzt und anschliessend 15 ml 2 N Salzsäure zugegeben.

Nach intensivem Rühren unter Zugabe von einer kleinen Menge Diethylether setzt die Kristallisation des Produktes ein. Die Kristalle werden abfiltriert, mit Wasser und Diethylether gewaschen und nochmals mit Diethylether verrührt.

Nach Abfiltrieren und Trocknen des Rückstandes erhält man schliesslich nach Umkristallisation aus Essigsäureethylester/Acetonitril 8,1 g (68 % d.Th.) N-(3-Allylsulfonyl-pyridin-2-yl-sulfonyl)-N'-(4,6-dimethoxypyrimidin-2-yl)harnstoff (Verbindung Nr. 1.001) mit einem Schmelzpunkt von + 185 °C.

In analoger Weise werden die in den angeschlossenen Tabellen aufgelisteten Verbindungen der Formel I sowie die Zwischenprodukte der Formeln II und III hergestellt.

Tabelle 1

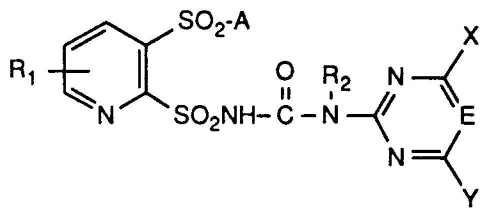

| Nr. | A | $R_1$ | $R_2$ | E | X | Y | Schmelzpunkt [°C] |
|---|---|---|---|---|---|---|---|
| 1.001 | $-CH_2CH=CH_2$ | H | H | CH | $OCH_3$ | $OCH_3$ | +185 (Zers.) |
| 1.002 | $-CH_2CH_2OCH_3$ | H | H | CH | $OCH_3$ | $OCH_3$ | +158 bis +159 |
| 1.003 | $-CH_2CH=CH_2$ | H | H | CH | $OCH_3$ | $CH_3$ | +172 (Zers.) |
| 1.004 | $-CH_2CH=CH_2$ | H | H | CH | $OCH_3$ | Cl | |
| 1.005 | $-CH_2CH=CH_2$ | H | H | CH | $OCHF_2$ | $OCHF_2$ | |
| 1.006 | $-CH_2CH=CH_2$ | H | H | CH | $OCHF_2$ | $OCH_3$ | |
| 1.007 | $-CH_2CH=CH_2$ | H | H | CH | $OCHF_2$ | $CH_3$ | |
| 1.008 | $-CH_2CH=CH_2$ | H | H | N | $OCH_3$ | $OCH_3$ | +181 (Zers.) |
| 1.009 | $-CH_2CH=CH_2$ | H | H | N | $OCH_3$ | $CH_3$ | +170 (Zers.) |
| 1.010 | $-CH_2CH=CH_2$ | H | H | N | $OC_2H_5$ | $CH_3$ | |
| 1.011 | $-CH_2CH=CH_2$ | H | H | N | | $NHCH_3$ | |
| 1.012 | $-CH_2CH=CH_2$ | H | H | N | $OCH_3$ | $N(CH_3)_2$ | |
| 1.013 | $-CH_2CH=CH_2$ | H | H | N | $OCH_3$ | $C_3H_5$-cycl. | |
| 1.014 | $-CH_2CH=CH_2$ | H | H | N | $OC_2H_5$ | $C_3H_5$-cycl. | |
| 1.015 | $-CH_2CH=CH_2$ | 6-$CH_3$ | H | CH | $OCH_3$ | $OCH_3$ | |
| 1.016 | $-CH_2CH=CH_2$ | 6-$CH_3$ | H | N | $OCH_3$ | $OCH_3$ | |
| 1.017 | $-CH_2CH=CH_2$ | 6-$CH_3$ | H | N | $OCH_3$ | $CH_3$ | |
| 1.018 | $-CH_2CH=CH_2$ | 5-$CH_3$ | H | CH | $OCH_3$ | $OCH_3$ | |
| 1.019 | $-CH_2CH=CH_2$ | 5-$CH_3$ | H | N | $OCH_3$ | $OCH_3$ | |
| 1.020 | $-CH_2CH=CH_2$ | 5-$CH_3$ | H | N | $OCH_3$ | $CH_3$ | |
| 1.021 | $-CH_2CH=CH_2$ | 4-$CH_3$ | H | CH | $OCH_3$ | $OCH_3$ | |
| 1.022 | $-CH_2CH=CH_2$ | 4-$CH_3$ | H | N | $OCH_3$ | $OCH_3$ | |
| 1.023 | $-CH_2CH=CH_2$ | 4-$CH_3$ | H | N | $OCH_3$ | $CH_3$ | |
| 1.024 | $CH_3$<br>$-CH_2-C=CH_2$ | H | H | CH | $OCH_3$ | $OCH_3$ | +166 (Zers.) |
| 1.025 | $CH_3$<br>$-CH_2-C=CH_2$ | H | H | CH | $OCH_3$ | $CH_3$ | |
| 1.026 | $CH_3$<br>$-CH_2-C=CH_2$ | H | H | CH | $OCH_3$ | Cl | |
| 1.027 | $CH_3$<br>$-CH_2-C=CH_2$ | H | H | CH | $OCH_3$ | $OCHF_2$ | |
| 1.028 | $CH_3$<br>$-CH_2-C=CH_2$ | H | H | CH | $OCHF_2$ | $OCHF_2$ | |
| 1.029 | $CH_3$<br>$-CH_2-C=CH_2$ | H | H | N | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.030 | $-CH_2-\overset{CH_3}{\overset{\vert}{C}}=CH_2$ | H | H | N | $OCH_3$ | $CH_3$ | |
| 1.031 | $-CH_2-\overset{CH_3}{\overset{\vert}{C}}=CH_2$ | H | H | N | $OC_2H_5$ | $CH_3$ | |
| 1.032 | $-CH_2-\overset{CH_3}{\overset{\vert}{C}}=CH_2$ | H | H | N | $OC_2H_5$ | $NHCH_3$ | |
| 1.033 | $-CH_2-\overset{CH_3}{\overset{\vert}{C}}=CH_2$ | H | H | N | $OCH_3$ | $OCH_3$ | |
| 1.034 | $-CH_2-\overset{CH_3}{\overset{\vert}{C}}=CH_2$ | H | H | N | $OC_2H_5$ | $C_3H_5$-cycl. | |
| 1.035 | $-CH_2-\overset{CH_3}{\overset{\vert}{C}}=CH_2$ | H | H | CH | $OCHF_2$ | $CH_3$ | |
| 1.036 | $-CH_2-\overset{CH_3}{\overset{\vert}{C}}=CH_2$ | 6-$CH_3$ | H | CH | $OCH_3$ | $OCH_3$ | |
| 1.037 | $-CH_2-\overset{CH_3}{\overset{\vert}{C}}=CH_2$ | 5-Cl | H | CH | $OCH_3$ | $OCH_3$ | |
| 1.038 | $-CH_2-CH=CHCl$ | H | H | CH | $OCH_3$ | $OCH_3$ | |
| 1.039 | $-CH_2-CH=CHCl$ | H | H | CH | $OCH_3$ | Cl | |
| 1.040 | $-CH_2-CH=CHCl$ | H | H | CH | $OCH_3$ | $CH_3$ | |
| 1.041 | $-CH_2-CH=CHCl$ | H | H | N | $OCH_3$ | $CH_3$ | |
| 1.042 | $-CH_2-\overset{Cl}{\overset{\vert}{C}}=CH_2$ | H | H | CH | $OCH_3$ | $OCH_3$ | |
| 1.043 | $-CH_2-\overset{Cl}{\overset{\vert}{C}}=CH_2$ | H | H | CH | $OCH_3$ | $OCH_3$ | |
| 1.044 | $-CH_2-\overset{Cl}{\overset{\vert}{C}}=CH_2$ | H | H | CH | $OCH_3$ | Cl | |
| 1.045 | $-CH_2-\overset{Cl}{\overset{\vert}{C}}=CH_2$ | H | H | N | $OCH_3$ | $CH_3$ | |
| 1.046 | $-CH_2CH_2CH_2F$ | H | H | CH | $OCH_3$ | $OCH_3$ | +156 bis +157 |
| 1.047 | $-CH_2CH_2CH_2F$ | H | H | CH | $OCH_3$ | Cl | |
| 1.048 | $-CH_2CH_2CH_2F$ | H | H | CH | $OCH_3$ | $CH_3$ | |
| 1.049 | $-CH_2CH_2CH_2F$ | H | H | CH | $OCH_3$ | $OCHF_2$ | |
| 1.050 | $-CH_2CH_2CH_2F$ | H | H | CH | $OCH_3$ | $OCHF_2$ | |
| 1.051 | $-CH_2CH_2CH_2F$ | H | H | N | $OCH_3$ | $CH_3$ | |
| 1.052 | $-CH_2CH_2CH_2F$ | H | H | N | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.053 | $-CH_2CH_2CH_2F$ | H | H | N | $OC_2H_5$ | $C_3H_5$-cycl. | |
| 1.054 | $-CH_2CH_2CH_2F$ | H | H | N | $OC_2H_5$ | $CH_3$ | |
| 1.055 | $-CH_2CH_2CH_2F$ | H | H | N | $OC_2H_5$ | $NHCH_3$ | |
| 1.056 | $-CH_2CH_2CH_2F$ | H | H | N | $OCH_3$ | $N(CH_3)_2$ | |
| 1.057 | $-CH_2CH_2CH_2F$ | 6-$CH_3$ | H | CH | $OCH_3$ | $OCH_3$ | |
| 1.058 | $-CH_2CH_2CH_2F$ | 6-$CH_3$ | H | CH | $OCH_3$ | Cl | |
| 1.059 | $-CH_2CH_2CH_2F$ | 6-$CH_3$ | H | N | $OCH_3$ | $OCH_3$ | |
| 1.060 | $-CH_2CH_2CH_2F$ | O-$OCH_3$ | H | CH | $OCH_3$ | $OCH_3$ | |
| 1.061 | $-CH_2CH_2CH_2F$ | 6-F | H | CH | $OCH_3$ | $OCH_3$ | |
| 1.062 | $-CH_2CH_2OCH_3$ | H | H | CH | $OCH_3$ | $CH_3$ | |
| 1.063 | $-CH_2CH_2OCH_3$ | H | H | N | $OCH_3$ | $CH_3$ | |
| 1.064 | $-CH_2CH=CH_2$ | 5-Cl | H | CH | $OCH_3$ | $OCH_3$ | +153 bis +156 (Zers.) |
| 1.065 | $-CH_2CH=CH_2$ | 5-Cl | H | CH | $OCH_3$ | $CH_3$ | |
| 1.066 | $-CH_2CH=CH_2$ | 5-Cl | H | N | $OCH_3$ | $CH_3$ | |
| 1.067 | $-CH_2CH=CH_2$ | 5-Cl | H | CH | $OCH_3$ | Cl | |
| 1.068 | $-CH_2CH=CH_2$ | 5-Cl | H | CH | $CH_3$ | $CH_3$ | |
| 1.069 | $-CH(CH_3)CH_2F$ | H | H | CH | $OCH_3$ | $OCH_3$ | |
| 1.070 | $-CH(CH_3)CH_2F$ | H | H | CH | Cl | $OCH_3$ | |
| 1.071 | $-CH(CH_3)CH_2F$ | H | H | CH | $OCH_3$ | $CH_3$ | |
| 1.072 | $-CH(CH_3)CH_2F$ | H | H | N | $OCH_3$ | $CH_3$ | |
| 1.073 | $-CH(CH_3)CH_2F$ | H | H | N | $OCH_3$ | $OCH_3$ | |
| 1.074 | $-CH(CH_3)CH_2F$ | H | H | N | $OC_2H_5$ | $C_3H_5$-cycl. | |
| 1.075 | $-CH(CH_3)CH_2F$ | H | H | N | $OC_2H_5$ | $CH_3$ | |
| 1.076 | $-CH(CH_3)CH_2F$ | 6-$CH_3$ | H | CH | $OCH_3$ | $OCH_3$ | |
| 1.077 | $-CH(CH_3)CH_2F$ | 6-$CH_3$ | H | N | $OCH_3$ | $CH_3$ | |
| 1.078 | $-CH(CH_2F)C_2H_5$ | H | H | CH | $OCH_3$ | $OCH_3$ | |
| 1.079 | $-CH(CH_2F)C_2H_5$ | H | H | CH | Cl | $OCH_3$ | |
| 1.080 | $-CH(CH_2F)C_2H_5$ | H | H | N | $OCH_3$ | $CH_3$ | |
| 1.081 | $-CH(CH_2F)CH_2F$ | H | H | N | $OCH_3$ | $CH_3$ | |
| 1.082 | $-CH(CH_2F)CH_2F$ | H | H | CH | $OCH_3$ | $OCH_3$ | |
| 1.083 | $-CH(CH_2F)CH_2F$ | H | H | CH | Cl | $OCH_3$ | |
| 1.084 | $-CH(CH_2F)CH_2F$ | 6-$CH_3$ | H | CH | $OCH_3$ | $OCH_3$ | |
| 1.085 | $-CH(CH_2F)CH_2F$ | 6-$CH_3$ | H | N | $OCH_3$ | $CH_3$ | |
| 1.086 | $-CH(CH_2F)CH_2F$ | 6-$CH_3$ | H | N | $OCH_3$ | $CH_3$ | |
| 1.087 | $-CH(CH_2F)C_2H_5$ | 6-$CH_3$ | H | CH | $OCH_3$ | $OCH_3$ | |
| 1.088 | 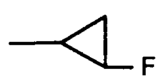 | H | H | CH | $OCH_3$ | $OCH_3$ | |
| 1.089 | 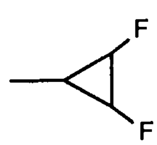 | H | H | CH | $OCH_3$ | $OCH_3$ | |
| 1.090 | 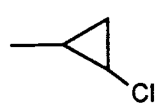 | H | H | CH | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.091 | 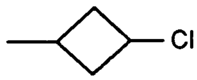 | H | H | N | $OCH_3$ | $CH_3$ | |
| 1.092 | 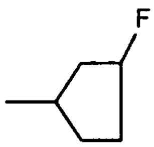 | H | H | CH | $OCH_3$ | $CH_3$ | |
| 1.093 | 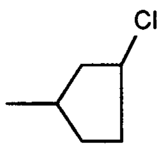 | H | H | CH | $OCH_3$ | $CH_3$ | |
| 1.094 | 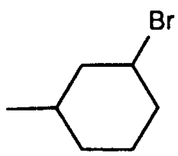 | H | H | CH | $OCH_3$ | $OCH_3$ | |
| 1.095 | 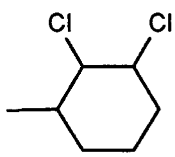 | H | H | CH | $OCH_3$ | $OCH_3$ | |
| 1.096 | 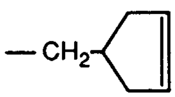 | H | H | CH | $OCH_3$ | $OCH_3$ | |
| 1.097 | 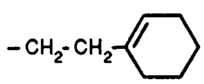 | H | H | CH | $OCH_3$ | $OCH_3$ | |
| 1.098 | 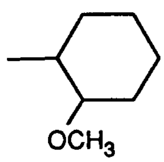 | H | H | CH | $OCH_3$ | $OCH_3$ | |
| 1.099 | 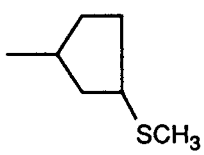 | H | H | CH | $OCH_3$ | $OCH_3$ | |
| 1.100 | 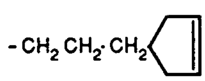 | H | H | CH | $OCH_3$ | $OCH_3$ | |
| 1.101 | 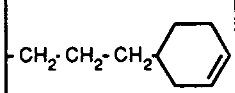 | H | H | CH | $CH_3$ | $CH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.102 | $-CH_2CH=CH_2$ | $6-OCH_3$ | H | CH | $OCH_3$ | $OCH_3$ | |
| 1.103 | $-CH_2CH=CH_2$ | H | $CH_3$ | CH | $OCH_3$ | $OCH_3$ | |
| 1.104 | $-CH_2CH=CH_2$ | $5-OCH_3$ | H | CH | $OCH_3$ | $OCH_3$ | |
| 1.105 | $-CH_2CH=CH_2$ | $6-OCH_3$ | H | CH | $CH_3$ | $OCH_3$ | |
| 1.106 | $-CH_2CH=CH_2$ | $6-OCH_3$ | H | CH | $OCH_3$ | $OCH_3$ | |
| 1.107 | $-CH_2C{\equiv}CH$ | H | H | CH | $OCH_3$ | $OCH_3$ | |
| 1.108 | $-CH_2C{\equiv}CH$ | H | H | N | $OCH_3$ | $OCH_3$ | |
| 1.109 | $-CH_2C{\equiv}CH$ | H | H | CH | $OCH_3$ | $CH_3$ | |
| 1.110 | $-CH_2C{\equiv}CH$ | $6-CH_3$ | H | CH | $OCH_3$ | $OCH_3$ | |
| 1.111 | $-CH_2C{\equiv}CH$ | $6-OCH_3$ | H | CH | $OCH_3$ | $OCH_3$ | |
| 1.112 | $-CH_2C{\equiv}CH$ | H | H | CH | $OCH_3$ | $OCHF_2$ | |
| 1.113 | $-CH_2C{\equiv}CH$ | H | H | CH | $OCHF_2$ | $OCHF_2$ | |
| 1.114 | $-CH_2CH_2CH=CH_2$ | H | H | CH | $OCH_3$ | $OCH_3$ | +168 (Zers.) |
| 1.115 | $-CH_2CH_2CH=CH_2$ | H | H | CH | $CH_3$ | $OCH_3$ | |
| 1.116 | $-CH_2CH_2CH=CH_2$ | H | H | N | $OCH_3$ | $OCH_3$ | |
| 1.117 | $-CH_2CH_2CH=CH_2$ | H | H | CH | $OCH_3$ | $OCHF_2$ | |
| 1.118 | $-CH_2CH_2CH=CH_2$ | $6-CH_3$ | H | CH | $OCH_3$ | $OCH_3$ | |
| 1.119 | $-CH_2CH_2CH=CH_2$ | $6-CH_3$ | H | N | $OCH_3$ | $OCH_3$ | |
| 1.120 | $-CH_2CH_2CH=CH_2$ | $5-CH_3$ | H | CH | $OCH_3$ | $OCH_3$ | |
| 1.121 | $-CH_2CH_2CH=CH_2$ | $5-CH_3$ | H | CH | $OCH_3$ | $OCHF_2$ | |
| 1.122 | $-CH_2CH_2CH=CH_2$ | $4-CH_3$ | H | CH | $OCH_3$ | $OCH_3$ | |
| 1.123 | $-CH_2CH_2CH=CH_2$ | $4-CH_3$ | H | N | $OCH_3$ | $OCH_3$ | |
| 1.124 | $-CH_2CH_2CH=CH_2$ | $4-CH_3$ | H | CH | $OCH_3$ | $N(CH_3)_2$ | |
| 1.125 | $-CH_2CH_2CH=CH_2$ | $6-OCH_3$ | H | CH | $OCH_3$ | $OCH_3$ | |
| 1.126 | $-CH_2CH_2CH=CH_2$ | $6-OCH_3$ | H | N | $OCH_3$ | $OCH_3$ | |
| 1.127 | $-CH(CH_3)CH=CH_2$ | H | H | CH | $OCH_3$ | $OCH_3$ | +113 (Zers.) |
| 1.128 | $-CH(CH_3)CH=CH_2$ | H | H | N | $OCH_3$ | $OCH_3$ | |
| 1.129 | $-CH(CH_3)CH=CH_2$ | H | H | CH | $OCH_3$ | $CH_3$ | |
| 1.130 | $-CH(CH_3)CH=CH_2$ | H | H | CH | $OCH_3$ | $OCHF_2$ | |
| 1.131 | $-CH(CH_3)CH=CH_2$ | $6-CH_3$ | H | CH | $OCH_3$ | $OCH_3$ | |
| 1.132 | $-CH(CH_3)CH=CH_2$ | $6-CH_3$ | H | N | $OCH_3$ | $OCH_3$ | |
| 1.133 | $-CH(CH_3)CH=CH_2$ | $6-OCH_3$ | H | CH | $OCH_3$ | $OCH_3$ | |
| 1.134 | $-CH(CH_3)CH=CH_2$ | $6-OCH_3$ | H | N | $OCH_3$ | $OCH_3$ | |
| 1.135 | $-CH(CH_3)CH=CH_2$ | $5-CH_3$ | N | CH | $OCH_3$ | $OCH_3$ | |
| 1.136 | $-CH(CH_3)CH=CH_2$ | 5-Cl | H | CH | $OCH_3$ | $OCH_3$ | |
| 1.137 | $-CH(CH_3)CH=CH_2$ | $4-CH_3$ | H | CH | $OCH_3$ | $OCH_3$ | |
| 1.138 | $-CH(CH_3)CH=CH_2$ | $4-CH_3$ | H | CH | $OCH_3$ | $N(CH_3)_2$ | |
| 1.139 | $-CH(CH_3)CH=CH_2$ | $4-CH_3$ | H | N | $OCH_3$ | $OCH_3$ | |
| 1.140 | $-CH_2CH=CHCH_3$ | H | H | CH | $OCH_3$ | $OCH_3$ | |
| 1.141 | $-CH_2CH=CHCH_3$ | H | H | N | $OCH_3$ | $OCH_3$ | |
| 1.142 | $-CH_2CH=CHCH_3$ | H | H | CH | $OCH_3$ | $CH_3$ | |
| 1.143 | $-CH_2CH=CHCH_3$ | H | H | CH | $OCH_3$ | $OCHF_2$ | |
| 1.144 | $-CH_2CH=CHCH_3$ | $6-CH_3$ | H | CH | $OCH_3$ | $OCH_3$ | |
| 1.145 | $-CH_2CH=CHCH_3$ | $6-CH_3$ | H | N | $OCH_3$ | $OCH_3$ | |
| 1.146 | $-CH_2CH=CHCH_3$ | $6-OCH_3$ | H | CH | $OCH_3$ | $OCH_3$ | |
| 1.147 | $-CH_2CH=CHCH_3$ | $6-OCH_3$ | H | N | $OCH_3$ | $OCH_3$ | |
| 1.148 | $-CH_2CH=CHCH_3$ | $5-CH_3$ | H | CH | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.149 | $-CH_2CH=CHCH_3$ | $4-CH_3$ | H | CH | $OCH_3$ | $OCH_3$ | |
| 1.150 | $-CH_2CH=CHCH_3$ | $4-CH_3$ | H | CH | $OCH_3$ | $N(CH_3)_2$ | |
| 1.151 | $-CH_2-C\equiv C-CH_3$ | H | H | CH | $OCH_3$ | $OCH_3$ | |
| 1.152 | $-CH_2-C\equiv C-CH_3$ | H | H | N | $OCH_3$ | $OCH_3$ | |
| 1.153 | $-CH_2-C\equiv C-CH_3$ | H | H | CH | $CH_3$ | $CH_3$ | |
| 1.154 | $-CH_2-C\equiv C-CH_3$ | H | H | CH | $OCH_3$ | $OCHF_2$ | |
| 1.155 | $-CH_2-C\equiv C-CH_3$ | $6-CH_3$ | H | CH | $OCH_3$ | $OCH_3$ | |
| 1.156 | $-CH_2-C\equiv C-CH_3$ | $6-OCH_3$ | H | CH | $OCH_3$ | $OCH_3$ | |
| 1.157 | $-CH_2-C\equiv C-CH_3$ | $6-OCHF_2$ | H | CH | $OCH_3$ | $OCH_3$ | |
| 1.158 | $-CH_2-C\equiv C-CH_3$ | $5-CH_3$ | H | CH | $OCH_3$ | $OCH_3$ | |
| 1.159 | $-CH_2-C\equiv C-CH_3$ | $4-CH_3$ | H | CH | $OCH_3$ | $OCH_3$ | |
| 1.160 | $-CH_2-C\equiv C-CH_3$ | $4-CH_3$ | H | CH | $OCH_3$ | $N(CH_3)_2$ | |
| 1.161 | $-CH_2CH_2C\equiv CH$ | H | H | CH | $OCH_3$ | $OCH_3$ | |
| 1.162 | $-CH_2CH_2C\equiv CH$ | H | H | N | $OCH_3$ | $OCH_3$ | |
| 1.163 | $-CH_2CH_2C\equiv CH$ | H | H | CH | $OCH_3$ | $CH_3$ | |
| 1.164 | $-CH_2CH_2C\equiv CH$ | H | H | CH | $OCH_3$ | $OCHF_2$ | |
| 1.165 | $-CH_2CH_2C\equiv CH$ | $6-CH_3$ | H | CH | $OCH_3$ | $OCH_3$ | |
| 1.166 | $-CH_2CH_2C\equiv CH$ | $6-OCH_3$ | H | CH | $OCH_3$ | $OCH_3$ | |
| 1.167 | $-CH_2CH_2C\equiv CH$ | $6-OCHF_2$ | H | CH | $OCH_3$ | $OCH_3$ | |
| 1.168 | $-CH_2CH_2C\equiv CH$ | $5-CH_3$ | H | CH | $OCH_3$ | $OCH_3$ | |
| 1.169 | $-CH_2CH_2C\equiv CH$ | $4-CH_3$ | H | CH | $OCH_3$ | $OCH_3$ | |
| 1.170 | $-CH_2CH_2C\equiv CH$ | $6-OCHF_2$ | H | CH | $OCH_3$ | $OCH_3$ | |
| 1.171 | $-CH_2CH=CHCl$ (E) | H | H | CH | $OCH_3$ | $OCH_3$ | +184 (Zers.) |
| 1.172 | $-CH_2CH=CHCl$ (E) | H | H | N | $OCH_3$ | $OCH_3$ | |
| 1.173 | $-CH_2CH=CHCl$ (E) | H | H | CH | $OCH_3$ | $CH_3$ | |
| 1.174 | $-CH_2CH=CHCl$ (E) | H | H | CH | $OCH_3$ | $OCHF_2$ | |
| 1.175 | $-CH_2CH=CHCl$ (E) | $6-CH_3$ | H | CH | $OCH_3$ | $OCH_3$ | |
| 1.176 | $-CH_2CH=CHCl$ (E) | $6-OCH_3$ | H | CH | $OCH_3$ | $OCH_3$ | |
| 1.177 | $-CH_2CH=CHCl$ (E) | $5-CH_3$ | H | CH | $OCH_3$ | $OCH_3$ | |
| 1.178 | $-CH_2CH=CHCl$ (E) | $4-CH_3$ | H | CH | $OCH_3$ | $OCH_3$ | |
| 1.179 | $-CH_2CH=CHCl$ (E) | $6-OCH_3$ | H | N | $OCH_3$ | $OCH_3$ | |
| 1.180 | $-CH_2CH=CHCl$ (Z) | H | H | CH | $OCH_3$ | $OCH_3$ | +151 bis +152 (Zers.) |
| 1.181 | $-CH_2CH=CHCl$ (Z) | H | H | N | $OCH_3$ | $OCH_3$ | |
| 1.182 | $-CH_2CH=CHCl$ (Z) | H | H | CH | $OCH_3$ | $CH_3$ | |
| 1.183 | $-CH_2CH=CHCl$ (Z) | H | H | CH | $OCH_3$ | $OCHF_2$ | |
| 1.184 | $-CH_2CH=CHCl$ (Z) | $6-CH_3$ | H | CH | $OCH_3$ | $OCH_3$ | |
| 1.185 | $-CH_2CH=CHCl$ (Z) | $6-OCH_3$ | H | CH | $OCH_3$ | $OCH_3$ | |
| 1.186 | $-CH_2CH=CHCl$ (Z) | $6-OCH_3$ | H | N | $OCH_3$ | $OCH_3$ | |
| 1.187 | $-CH_2CH=CHCl$ (Z) | $5-CH_3$ | H | CH | $OCH_3$ | $OCH_3$ | |
| 1.188 | $-CH_2CH=CHCl$ (Z) | $4-CH_3$ | H | CH | $OCH_3$ | $OCH_3$ | |
| 1.189 | $-CH_2CH=CHCl$ (Z) | $4-CH_3$ | H | N | $OCH_3$ | $OCH_3$ | |
| 1.190 | $-CH_2CH=CHCl$ (Z) | $4-CH_3$ | H | CH | $OCH_3$ | $N(CH_3)_2$ | |
| 1.191 | $-CH_2CH=CHCl$ (Z) | $6-OCHF_2$ | H | CH | $OCH_3$ | $OCH_3$ | |
| 1.192 | $-CH_2CH=CHCF_3$ | H | H | CH | $OCH_3$ | $OCH_3$ | |
| 1.193 | $-CH_2CH=CHCF_3$ | H | H | N | $OCH_3$ | $OCH_3$ | |
| 1.194 | $-CH_2CH=CHCF_3$ | H | H | CH | $OCH_3$ | $CH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.195 | $-CH_2CH=CHBr$ (Z/E) | H | H | CH | $OCH_3$ | $OCH_3$ | +157 bis 158 (Zers.) |
| 1.196 | $-CH_2CH=CHBr$ (Z/E) | H | H | N | $OCH_3$ | $OCH_3$ | |
| 1.197 | $-CH_2CH=CHBr$ (Z/E) | H | H | CH | $OCH_3$ | $CH_3$ | |
| 1.198 | $-CH_2CH=CHBr$ (Z/E) | H | H | CH | $OCH_3$ | $OCHF_2$ | |
| 1.199 | $-CH_2CH=CHBr$ (Z/E) | 6-$CH_3$ | H | CH | $OCH_3$ | $OCH_3$ | |
| 1.200 | $-CH_2CH=CHBr$ (Z/E) | 6-$CH_3$ | H | N | $OCH_3$ | $OCH_3$ | |
| 1.201 | $-CH_2CH=CHBr$ (Z/E) | 6-$OCH_3$ | H | CH | $OCH_3$ | $OCH_3$ | |
| 1.202 | $-CH_2CH=CHBr$ (Z/E) | 6-$OCH_3$ | H | N | $OCH_3$ | $OCH_3$ | |
| 1.203 | $-CH_2CH=CHBr$ (Z/E) | 5-$CH_3$ | H | CH | $OCH_3$ | $OCH_3$ | |
| 1.204 | $-CH_2CH=CHBr$ (Z/E) | 4-$CH_3$ | H | CH | $OCH_3$ | $OCH_3$ | |
| 1.205 | $-CH_2CH=CHBr$ (Z/E) | 4-$CH_3$ | H | N | $OCH_3$ | $OCH_3$ | |
| 1.206 | $-CH_2CH=CHBr$ (Z/E) | 4-$CH_3$ | H | CH | $OCH_3$ | $N(CH_3)_2$ | |
| 1.207 | 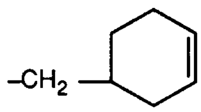 | H | H | CH | $OCH_3$ | $OCH_3$ | |
| 1.208 | 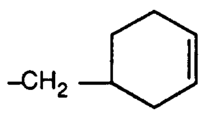 | H | H | N | $OCH_3$ | $OCH_3$ | |
| 1.209 | 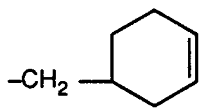 | 6-$CH_3$ | H | CH | $OCH_3$ | $OCH_3$ | |
| 1.210 | 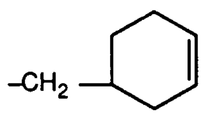 | 6-$CH_3$ | H | N | $OCH_3$ | $OCH_3$ | |
| 1.211 | 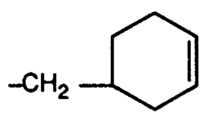 | 6-$OCH_3$ | H | CH | $OCH_3$ | $OCH_3$ | |
| 1.212 | 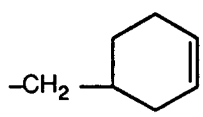 | 6-$OCH_3$ | N | N | $OCH_3$ | $OCH_3$ | |
| 1.213 | 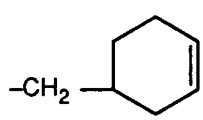 | 5-$CH_3$ | H | CH | $OCH_3$ | $OCH_3$ | |
| 1.214 | 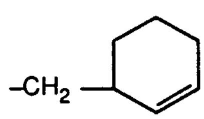 | H | H | CH | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.215 | 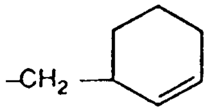 | H | H | N | $OCH_3$ | $OCH_3$ | |
| 1.216 | 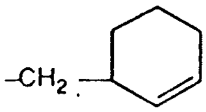 | H | H | CH | $OCH_3$ | $OCH_3$ | |
| 1.217 | 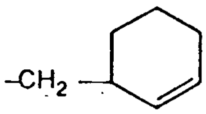 | H | H | CH | $OCH_3$ | $OCHF_2$ | |
| 1.218 | 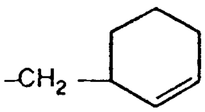 | 6-$CH_3$ | H | CH | $OCH_3$ | $OCH_3$ | |
| 1.219 | 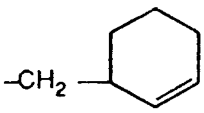 | 6-$CH_3$ | H | CH | $OCH_3$ | $OCH_3$ | |
| 1.220 | 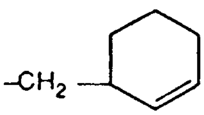 | 6-$OCHF_2$ | H | CH | $OCH_3$ | $OCH_3$ | |
| 1.221 | 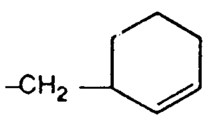 | 5-$CH_3$ | H | CH | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.222 | -$CH_2$ 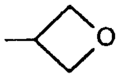 | H | H | CH | $OCH_3$ | $OCH_3$ | |
| 1.223 | -$CH_2$ 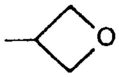 | H | H | N | $OCH_3$ | $OCH_3$ | |
| 1.224 | -$CH_2$ 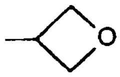 | H | H | CH | $OCH_3$ | $CH_3$ | |
| 1.225 | -$CH_2$ 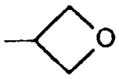 | 6-$CH_3$ | H | CH | $OCH_3$ | $OCH_3$ | |
| 1.226 | -$CH_2$ 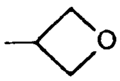 | 6-$OCH_3$ | H | CH | $OCH_3$ | $OCH_3$ | |
| 1.227 | -$CH_2$ 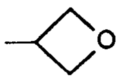 | H | H | CH | $OCH_3$ | $OCHF_2$ | |
| 1.228 | -$CH_2$ 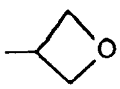 | 6-$OCHF_2$ | H | CH | $OCH_3$ | $OCH_3$ | |
| 1.229 | -$CH_2$ 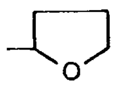 | H | H | CH | $OCH_3$ | $OCH_3$ | +138 bis +139 |
| 1.230 | -$CH_2$ 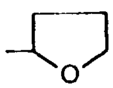 | H | H | N | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.231 | 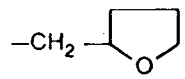 | H | H | CH | $OCH_3$ | $CH_3$ | |
| 1.232 | 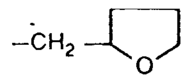 | H | H | CH | $OCH_3$ | $OCHF_2$ | |
| 1.233 | 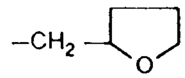 | 6-$CH_3$ | H | CH | $OCH_3$ | $OCH_3$ | |
| 1.234 | 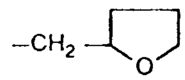 | 6-$OCH_3$ | H | CH | $OCH_3$ | $OCH_3$ | |
| 1.235 | 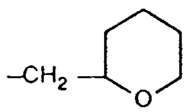 | H | H | CH | $OCH_3$ | $OCH_3$ | |
| 1.236 | 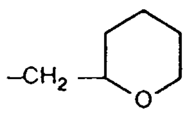 | H | H | N | $OCH_3$ | $OCH_3$ | |
| 1.237 | 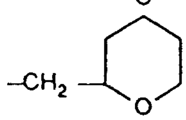 | H | H | CH | $OCH_3$ | $CH_3$ | |
| 1.238 | 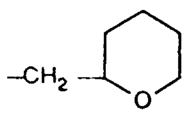 | H | H | CH | $OCH_3$ | $OCHF_2$ | |
| 1.239 | 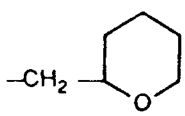 | 6-$CH_3$ | H | CH | $OCH_3$ | $OCH_3$ | |
| 1.240 | 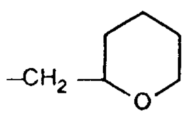 | 6-$OCH_3$ | H | CH | $OCH_3$ | $OCH_3$ | |
| 1.241 | 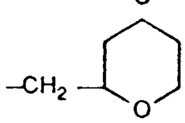 | 6-$OCHF_2$ | H | CH | $OCH_3$ | $OCH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.242 | $-CH_2-$ 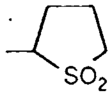 | H | H | CH | $OCH_3$ | $OCH_3$ | |
| 1.243 | $-CH_2-$ 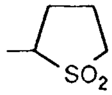 | H | H | N | $OCH_3$ | $OCH_3$ | |
| 1.244 | $-CH_2-$ 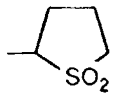 | H | H | CH | $OCH_3$ | $CH_3$ | |
| 1.245 | $-CH_2-$ 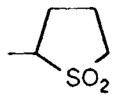 | 6-$CH_3$ | H | CH | $OCH_3$ | $OCH_3$ | |
| 1.246 | $-CH_2-$ 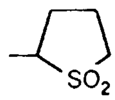 | 6-$OCH_3$ | H | CH | $OCH_3$ | $OCH_3$ | |
| 1.247 | $-CHF_2$ | H | H | CH | $OCH_3$ | $OCH_3$ | |
| 1.248 | $-CHF_2$ | H | H | CH | $OCH_3$ | $CH_3$ | |
| 1.249 | $-CHF_2$ | H | H | N | $OCH_3$ | $CH_3$ | |
| 1.250 | $-CHF_2$ | H | H | N | $OCH_3$ | $OCH_3$ | |
| 1.251 | $-CF_2CHF_2$ | H | H | N | $OCH_3$ | $OCH_3$ | |
| 1.252 | $-CF_2CHF_2$ | H | H | N | $OCH_3$ | $CH_3$ | |
| 1.253 | $-CF_2CHF_2$ | H | H | CH | $OCH_3$ | $CH_3$ | |
| 1.254 | $-CF_2CHF_2$ | H | H | CH | $OCH_3$ | $OCH_3$ | |
| 1.255 | $-CF_2CHF_2$ | H | H | CH | $CH_3$ | $CH_3$ | |
| 1.256 | $-CH_2CH_2N(CH_3)_2$ | H | H | CH | $CH_3$ | $CH_3$ | |
| 1.257 | $-CH_2CH_2N(CH_3)_2$ | H | H | CH | $OCH_3$ | $OCH_3$ | |
| 1.258 | $-CH_2CH_2N(CH_3)_2$ | H | H | N | $OCH_3$ | $OCH_3$ | |
| 1.259 | $-CH_2CH_2N(CH_3)_2$ | H | H | N | $OCH_3$ | $CH_3$ | |

Tabelle 1 (Fortsetzung)

| Nr. | A | $R_1$ | $R_2$ | E | X | Y | Schmelzpunkt [°C] |
|---|---|---|---|---|---|---|---|
| 1.260 | $-CH_2CH_2N(C_2H_5)_2$ | H | H | CH | $OCH_3$ | $OCH_3$ | |
| 1.261 | $-CH_2CH_2N(C_2H_5)_2$ | H | H | CH | $OCH_3$ | $CH_3$ | |
| 1.262 | $-CH_2CH_2N(C_2H_5)_2$ | H | H | CH | $OCH_3$ | $OCH_3$ | |
| 1.263 | $-CH_2CH_2NHCH_3$ | H | H | CH | $OCH_3$ | $OCH_3$ | |
| 1.264 | $-CH_2CH_2NHCH_3$ | H | H | CH | $OCH_3$ | $CH_3$ | |
| 1.265 | $-CH_2CH_2NHCH_3$ | H | H | N | $OCH_3$ | $CH_3$ | |
| 1.266 | $-CH(CH_3)CH_2OCH_3$ | H | H | N | $CH_3$ | $OCH_3$ | |
| 1.267 | $-CH(CH_3)CH_2OCH_3$ | H | H | CH | $OCH_3$ | $OCH_3$ | +136 bis +138 |
| 1.268 | $-CH(CH_3)CH_2OCH_3$ | 6-$CH_3$ | H | CH | $OCH_3$ | $OCH_3$ | |
| 1.269 | $-CH(CH_3)CH_2OCH_3$ | 6-$CH_3$ | H | N | $OCH_3$ | $CH_3$ | |
| 1.270 | $-CH(CH_3)CH_2OCH_3$ | H | H | CH | $CH_3$ | $CH_3$ | |
| 1.271 | $-CH(CH_3)CH_2OCH_3$ | H | H | CH | $OCH_3$ | $CH_3$ | |
| 1.272 | $-CH_2CH_2SO_2CH_3$ | H | H | CH | $OCH_3$ | $OCH_3$ | +133 bis +137 |
| 1.273 | $-CH_2CH_2SO_2CH_3$ | H | H | CH | $OCH_3$ | $CH_3$ | |
| 1.274 | $-CH_2CH_2SO_2CH_3$ | H | H | N | $OCH_3$ | $CH_3$ | |
| 1.275 | $-CH_2CH_2SO_2CH_3$ | H | H | N | $OCH_3$ | $OCH_3$ | |
| 1.276 | $-CH_2CH_2CH_2Cl$ | H | H | N | $OCH_3$ | $OCH_3$ | +166 bis +168 |
| 1.277 | $-CH_2CH_2CH_2Cl$ | H | H | N | $OCH_3$ | $OCH_3$ | |
| 1.278 | $-CH_2CH_2CH_2Cl$ | H | H | N | $OCH_3$ | $CH_3$ | |
| 1.279 | $-CH_2CH_2CH_2Cl$ | 6-$CH_3$ | H | N | $OCH_3$ | $CH_3$ | |
| 1.300 | $-CH_2CH_2CH_2Cl$ | 6-$CH_3$ | H | CH | $OCH_3$ | $CH_3$ | |
| 1.301 | $-CH_2CH_2CH_2Cl$ | 6-$CH_3$ | H | CH | $OCH_3$ | $OCH_3$ | |
| 1.302 | $-CH(CH_3)CH_2Cl$ | H | H | CH | $OCH_3$ | $OCH_3$ | +145 bis +148 |
| 1.303 | $-CH(CH_3)CH_2Cl$ | H | H | N | $OCH_3$ | $OCH_3$ | |
| 1.304 | $-CH(CH_3)CH_2Cl$ | H | H | N | $OCH_3$ | $CH_3$ | |
| 1.305 | $-CH(CH_3)CH_2OCH_3$ | H | H | CH | $OCH_3$ | Cl | |
| 1.306 | $-CH_2CH_2CH_2Cl$ | H | H | CH | $OCH_3$ | Cl | |

Tabelle 2

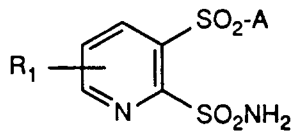

(II)

| Nr. | A | $R_1$ | Schmelzpunkt [°C] |
|---|---|---|---|
| 2.001 | $-CH_2CH=CH_2$ | H | +178 |
| 2.002 | $-CH_2CH_2OCH_3$ | H | |
| 2.003 | $-CH_2CH=CH_2$ | $6-CH_3$ | |
| 2.004 | $-CH_2CH=CH_2$ | $5-CH_3$ | |
| 2.005 | $-CH_2CH=CH_2$ | $4-CH_3$ | |
| 2.006 | $CH_3$<br>$-CH_2-C=CH_2$ | H | +180 |
| 2.007 | $CH_3$<br>$-CH_2-C=CH_2$ | $6-CH_3$ | |
| 2.008 | $CH_3$<br>$-CH_2-C=CH_2$ | 5-Cl | |
| 2.009 | $-CH_2CH=CH_2$ | 5-Cl | +193 bis +195 (Zers.) |
| 2.010 | $-CH_2CH=CHCl$ | H | |
| 2.011 | Cl<br>$-CH_2-C=CH_2$ | H | |
| 2.012 | $-CH_2CH_2CH_2F$ | H | |
| 2.013 | $-CH_2CH_2CH_2F$ | $6-CH_3$ | |
| 2.014 | $-CH_2CH_2CH_2F$ | $6-OCH_3$ | |
| 2.015 | $-CH_2CH_2CH_2F$ | 6-F | |
| 2.016 | $-CH(CH_3)CH_2F$ | 6-F | |
| 2.017 | $-CH(CH_3)CH_2F$ | $6-CH_3$ | |
| 2.018 | $-CH(CH_2F)C_2H_5$ | H | |
| 2.019 | $-CH(CH_2F)C_2H_5$ | $6-CH_3$ | |
| 2.020 | $-CH(CH_2F)CH_2F$ | H | |
| 2.021 | $-CH(CH_2F)CH_2F$ | $6-CH_3$ | |
| 2.022 | $-CH_2CH=CHCl$ (E) | $6-OCHF_2$ | |
| 2.023 | $-CH_2CH=CHCl$ (E) | $5-CH_3$ | |
| 2.024 | $-CH_2CH=CHCl$ (E) | $4-CH_3$ | |
| 2.025 | $-CH_2CH=CH-CF_3$ | H | |
| 2.026 | $-CH_2CH=CHBr$ | H | +188 bis +190 |
| 2.027 | $-CH_2CH=CHBr$ | $6-CH_3$ | |
| 2.028 | $-CH_2CH=CHBr$ | $6-OCH_3$ | |
| 2.029 | $-CH_2CH=CHBr$ | $5-CH_3$ | |
| 2.030 | $-CH_2CH=CHBr$ | $4-CH_3$ | |
| 2.031 | 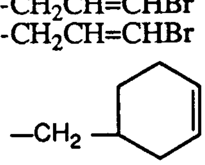 | H | |

Tabelle 2 (Fortsetzung)

| Nr. | A | $R_1$ | Schmelzpunkt [°C] |
|---|---|---|---|
| 2.032 | 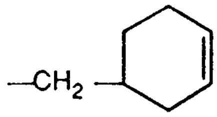 | 6-$CH_3$ | |
| 2.033 | 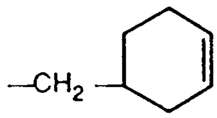 | 6-$OCH_3$ | |
| 2.034 | 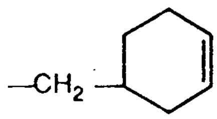 | 5-$CH_3$ | |
| 2.035 | 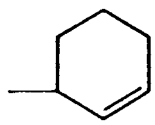 | H | |
| 2.036 | 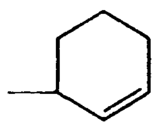 | 6-$CH_3$ | |
| 2.037 | 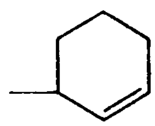 | 6-$OCH_3$ | |
| 2.038 | 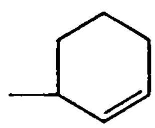 | 6-$OCHF_2$ | |
| 2.039 | 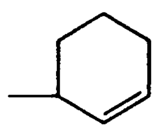 | 5-$CH_3$ | |
| 2.040 | 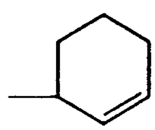 | 4-$CH_3$ | |

Tabelle 2 (Fortsetzung)

| Nr. | A | $R_1$ | Schmelzpunkt [°C] |
|---|---|---|---|
| 2.041 | 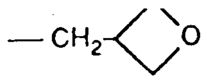 | H | |
| 2.042 | 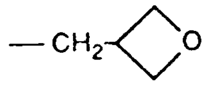 | 6-$CH_3$ | |
| 2.043 | 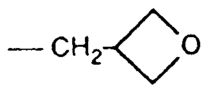 | 6-$OCH_3$ | |
| 2.044 | 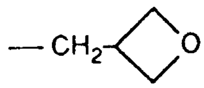 | 6-$OCHF_2$ | |
| 2.045 | 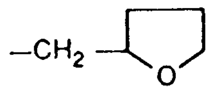 | H | |
| 2.046 | 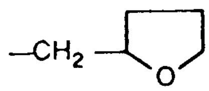 | 6-$CH_3$ | |
| 2.047 | 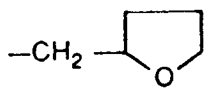 | 6-$OCH_3$ | |
| 2.048 | 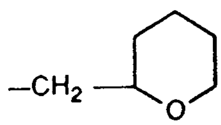 | H | |
| 2.049 | 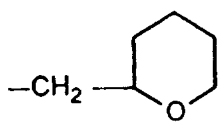 | 6-$CH_3$ | |

Tabelle 2 (Fortsetzung)

| Nr. | A | $R_1$ | Schmelzpunkt [°C] |
|---|---|---|---|
| 2.050 | 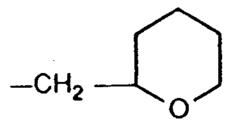 | 6-$OCH_3$ | |
| 2.051 | 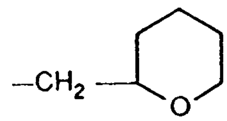 | 6-$OCHF_2$ | |
| 2.052 | 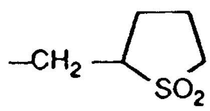 | H | |
| 2.053 | 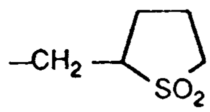 | 6-$CH_3$ | |
| 2.054 | 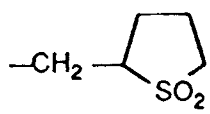 | 6-$OCH_3$ | |

Tabelle 2 (Fortsetzung)

| Nr. | A | $R_1$ | Schmelzpunkt [°C] |
|---|---|---|---|
| 2.055 | $-CH_2-CBr=CH_2$ | H | +158 bis +159 |
| 2.056 | $-CH_2-CCl=CH_2$ | H | +172 bis +173 |
| 2.057 | $-CH_2CH=CHCl$ (Z) | H | +196 bis +197 |
| 2.058 | $-CH_2CH=CHCl$ (Z) | $6-CH_3$ | |
| 2.059 | $-CH_2CH=CHCl$ (Z) | $6-OCH_3$ | |
| 2.060 | $CH_3$<br>\|<br>$-CHCH_2OCH_3$ | H | +232 bis +234 |
| 2.061 | $CH_3$<br>\|<br>$-CHCH_2OCH_3$ | $6-CH_3$ | |
| 2.062 | $-CH_2CH_2SO_2CH_3$ | H | +105 bis +110 |
| 2.063 | $-CHCH_2Cl$<br>\|<br>$CH_3$ | H | +185 bis +188 |
| 2.064 | $-CH_2CH_2CH_2Cl$ | H | +194 bis +195 |
| 2.065 | $-CH_2CH_2CH_2Cl$ | $6-CH_3$ | |
| 2.066 | $-CH_2CH_2CH_2Cl$ | $6-OCH_3$ | |
| 2.067 | $-CHF_2$ | H | |
| 2.068 | $-CF_2CHF_2$ | H | |
| 2.069 | $CH_3$<br>/<br>$-CH_2CH_2N$<br>\\<br>$CH_3$ | H | |
| 2.070 | $-CH_2CH_2N(C_2H_5)_2$ | H | |
| 2.071 | $-CH_2CH_2NHCH_3$ | H | |
| 2.072 | $-CH_2CH_2NHCH_3$ | $6-CH_3$ | |
| 2.073 | $-CH_2CH_2N(CH_3)_2$ | $6-CH_3$ | |
| 2.074 | $-CH_2CH=CH_2$ | $6-OCH_3$ | |
| 2.075 | $-CH_2CH=CH_2$ | $6-OCHF_2$ | |
| 2.076 | $-CH_2C\equiv CH$ | H | +164 bis +165 |
| 2.077 | $-CH_2C\equiv CH$ | $6-CH_3$ | |
| 2.078 | $-CH_2C\equiv CH$ | $6-OCH_3$ | |
| 2.079 | $-CH_2CF=CHCH_3$ | H | +116 bis +118 (Zers.) |
| 2.080 | $-CH_2CH_2CH=CH_2$ | H | |
| 2.081 | $-CH_2CH_2CH=CH_2$ | $6-CH_3$ | |
| 2.082 | $-CH_2CH_2CH=CH_2$ | $6-OCH_3$ | |
| 2.083 | $-CH_2CH_2CH=CH_2$ | $5-CH_3$ | |

Tabelle 2 (Fortsetzung)

| Nr. | A | $R_1$ | Schmelzpunkt [°C] |
|---|---|---|---|
| 2.103 | $-CH_2CH_2CH=CH_2$ | $4-CH_3$ | |
| 2.104 | $-CH(CH_3)CH=CH_2$ | H | +176 (Zers.) |
| 2.105 | $-CH(CH_3)CH=CH_2$ | $6-CH_3$ | |
| 2.106 | $-CH(CH_3)CH=CH_2$ | $6-OCH_3$ | |
| 2.107 | $-CH(CH_3)CH=CH_2$ | $5-CH_3$ | |
| 2.108 | $-CH(CH_3)CH=CH_2$ | 5-Cl | |
| 2.109 | $-CH(CH_3)CH=CH_2$ | $4-CH_3$ | |
| 2.110 | $-CH_2CH=CHCH_3$ | H | |
| 2.111 | $-CH_2CH=CHCH_3$ | $6-CH_3$ | |
| 2.112 | $-CH_2CH=CHCH_3$ | $6-OCH_3$ | |
| 2.113 | $-CH_2CH=CHCH_3$ | $5-CH_3$ | |
| 2.114 | $-CH_2CH=CHCH_3$ | $4-CH_3$ | |
| 2.115 | $-CH_2C\equiv C-CH_3$ | H | |
| 2.116 | $-CH_2C\equiv C-CH_3$ | $6-CH_3$ | |
| 2.117 | $-CH_2C\equiv C-CH_3$ | $6-OCH_3$ | |
| 2.118 | $-CH_2C\equiv C-CH_3$ | $6-OCHF_2$ | |
| 2.119 | $-CH_2C\equiv C-CH_3$ | $5-CH_3$ | |
| 2.120 | $-CH_2C\equiv C-CH_3$ | 5-Cl | |
| 2.121 | $-CH_2C\equiv C-CH_3$ | $4-CH_3$ | |
| 2.122 | $-CH_2CH_2C\equiv CH$ | H | |
| 2.123 | $-CH_2CH_2C\equiv CH$ | $6-CH_3$ | |
| 2.124 | $-CH_2CH_2C\equiv CH$ | $6-OCH_3$ | |
| 2.125 | $-CH_2CH_2C\equiv CH$ | $6-OCHF_2$ | |
| 2.126 | $-CH_2CH_2C\equiv CH$ | $5-CH_3$ | |
| 2.127 | $-CH_2CH_2C\equiv CH$ | $4-CH_3$ | |
| 2.128 | $-CH_2CH=CHCl$ (E) | H | +230 (Zers.) |
| 2.129 | $-CH_2CH=CHCl$ (E) | $6-CH_3$ | |
| 2.130 | $-CH_2CH=CHCl$ (E) | $6-OCH_3$ | |
| 2.131 | $-CH_2CH=CHCl$ (Z) | $6-OCHF_2$ | |
| 2.132 | $-CH_2CH=CHCl$ (Z) | $5-CH_3$ | |
| 2.133 | $-CH_2CH=CHCl$ (Z) | 5-Cl | |
| 2.134 | $-CH_2CH=CHCl$ (Z) | $4-CH_3$ | |

Tabelle 3

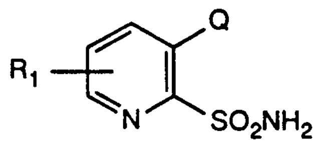

| Nr. | $R_1$ | Q | Schmelzpunkt [°C] |
|---|---|---|---|
| 3.001 | H | F | +121 bis +123 |
| 3.002 | 5-Cl | F | +119 bis +122 |
| 3.003 | 5-$CH_3$ | F | |
| 3.004 | 6-F | F | |
| 3.005 | 6-$CH_3$ | F | |
| 3.006 | 6-$OCH_3$ | F | |
| 3.007 | 4-$CH_3$ | F | |
| 3.008 | H | SH | +102 bis +104 |
| 3.009 | 6-$CH_3$ | SH | |
| 3.010 | 6-$OCHF_2$ | F | |
| 3.011 | 6-$OCHF_2$ | SH | |

Biologische Beispiele

Beispiel B1: Pre-emergente Herbizid-Wirkung
Im Gewächshaus wird unmittelbar nach der Einsaat der Versuchspflanzen in Saatschalen die Erdoberfläche mit einer wässrigen Spritzbrühe entsprechend einer Aufwandmenge von 4 kg Wirksubstanz/Hektar behandelt. Die Saatschalen werden im Gewächshaus bei 22 - 25 °C und 50 - 70 % relativer Luftfeuchtigkeit gehalten.

Nach 3 Wochen wird die Herbizidwirkung mit einem neunstufigen (1 = vollständige Schädigung, 9 = keine Wirkung) Boniturschema im Vergleich in einer unbehandelten Kontrollgruppe bewertet.

Boniturnoten von 1 bis 4 (insbesondere 1 bis 3) weisen auf eine gute bis sehr gute Herbizidwirkung hin. Boniturnoten von 6 bis 9 (insbesondere von 7 bis 9) weisen auf eine gute Toleranz (insbesondere bei Kulturpflanzen) hin.

In diesem Versuch zeigen die Verbindungen der Tabelle 1 starke Herbizidwirkung.

Beispiel B2: Post-emergente Herbizid-Wirkung (Kontaktherbizid)
Eine Anzahl Unkräuter, sowohl monokotyle wie dikoryle, wurden nach dem Auflaufen (im 4- bis 6-Blattstadium) mit einer wässrigen Wirkstoffdispersion in einer Dosierung von 4 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch ausgewertet. Auch in diesem Versuch zeigen die Verbindungen der Tabelle 1 gute Herbizidwirkung.

Beispiel B3: Herbizidwirkung für Wasserreis (paddy) Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat durch eine Spritzung der Prüfsubstanzen auf die Gefässe. Die verwendete Dosis entspricht einer Wirkstoffmenge von 4 kg AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit.

Die Auswertung der Versuche findet 3 Wochen nach Applikation statt. Die Verbindungen der Tabelle 1 schädigen dabei die Unkräuter, nicht aber den Reis.

Beispiel B4: Wuchshemmung bei tropischen Bodenbedecker-Leguminosen (cover crops) Die Versuchspflanzen Centrosema pubescens und Psophocarpus palustris werden in 4cm - Torftöpfen mit Landerde (45 %), Torf (45 %) und Zonolit (10 %) durch Stecklinge vermehrt. Die Anzucht erfolgt im Gewächshaus bei einer Tagestemperatur von 27 °C und einer Nachttemperatur von 23 °C. Die Beleuchtungsdauer ist mindestens 14 Stunden/Tag bei einer Intensität von mindestens 7000 Lux.

Ca. 50 Tage nach Ansatz der Stecklinge erfolgt das Vertopfen in 13 cm-Töpfe, 4 - 5 Pflanzen/Topf. Nach weiteren 60 Tagen werden die Pflanzen auf ca 15 cm Höhe zurückgeschnitten und appliziert. Dabei werden sie mit 0,1 bis 300 g Wirkstoff/ha (in der Regel 25 %ig formuliert) in wässriger Spritzbrühe besprüht. Die Wasseraufwandmenge beträgt ca. 200 l/ha.

4 Wochen nach der Applikation wird der Neuzuwachs im Gewicht ermittelt und in % zum Durchschnitt der unbehandelten Kontrollen dargestellt. Die nekrotischen Schäden sind in % zur gesamten Blattfläche aufgeführt.

Der Neuzuwachs der behandelten Pflanzen erweist sich als deutlich geringer als der der unbehandelten Kontrollen.

Beispiel B5: Wuchsregulierung an Sojabohnen Versuchspflanzen der Sorte Williams werden in 11 cm - Tontöpfen mit Landerde (45 %), Torf (45 %) und Zonolit (10 %) angesät und in der Klimakammer bei einer Tagestemperatur von 24 °C und einer Nachttemperatur von 19 °C angezogen. Die Beleuchtungsdauer ist 16 Stunden pro Tage bei einer Intensität von ca. 350 Mikro-Einstein.

Ca. 24 Tage nach der Saat erfolgt das Umtopfen in 18 cm - Töpfe, 2 Pflanzen/Topf. Nach weiteren 12 Tagen und im Stadium von 5 - 6 Trifolia-Blätter findet die Applikation mit 0,1 bis 300 g Wirkstoff/ha statt, in der Regel 25 %ig formuliert und in wässriger Spritzbrühe. Die Wasseraufwandmenge beträgt ca. 200 l/ha.

Ca. 4 Wochen nach der Applikation findet die Auswertung statt. Hierbei wird die Höhe des Neuzuwachses gemessen und in % zum Durchschnitt der unbehandelten Kontrollen dargestellt Die nekrotischen Schäden sind in % zur gesamten Blattfläche aufgeführt.

Die behandelten Pflanzen zeigen einen deutlich geringeren Neuzuwachs als die unbehandelten Kontrollen.

Beispiel B6: Wuchshemmung bei Getreide

Versuchspflanzen (Sommergerste der Sorte Iban) werden in 15 cm-Kunststofftöpfen mit steriler Landerde angesät und in der Klimakammer bei einer Tagestemperatur von 10 - 15 °C und einer Nachttemperatur von 5 - 10 °C angezogen. Die Beleuchtungsdauer ist 13.5 Stunden pro Tag bei einer Intensität von ca. 25000 Lux.

Ca. 34 Tage nach der Saat und dem Ausdünnen auf 4 Pflanzen/Topf erfolgt die Applikation mit 0,1 bis 300g Wirkstoff/ha, in der Regel 25 %ig formuliert und in wässriger Spritzbrühe. Die Wasseraufwandmenge ist ca. 500 l/ha. Nach der Applikation werden die Pflanzen im Gewächshaus bei einer Tagestemperatur von mindestens 10 °C aufgestellt. Die Beleuchtungsdauer ist mind. 13.5 Stunden/Tag.

Ca. 28 Tage nach der Behandlung findet die Auswertung statt. Hierbei wird die Höhe des Neuzuwachses in % zum Durchschnitt der unbehandelten Kontrollen dargestellt. Die nekrotischen Schäden sind in % zur gesamten Blattfläche aufgeführt.

Die behandelten Pflanzen weisen im Vergleich zu unbehandelten Kontrollen eine Verringerung des Neuzuwachsese auf.

Beispiel B7: Wuchshemmung bei Gräsern

Eine Mischung von Gräsern (z.B. Poa, Festuca, Lolium, Bromus, Cynosurus) und Klee (Trifolium pratense/repens) wird in 15 cm-Kunststofftöpfen mit steriler Landerde angesät und im Gewächshaus bei einer Tagestemperatur von 21 °C und einer Nachttemperatur von 17 °C angezogen. Die Beleuchtungsdauer ist 13.5 Stunden/Tag bei einer Lichtintensität von mind. 7000 Lux. Nach dem Auflauf werden die Pflanzen wöchentlich auf ca. 6cm Höhe zurückgeschnitten. Ca. 42 Tage nach der Saat und 1 Tag nach dem letzten Schnitt erfolgt die Applikation mit 0,1 bis 300g Wirkstoff/ha, in der Regel 25 %ig formuliert und in wässriger Spritzbrühe. Die Wasseraufwandmenge ist ca. 500 l/ha.

Ca. 3 Wochen nach der Behandlung findet die Auswertung statt. Hierbei wird die Höhe des Neuzuwachses gemessen und in % zum Durchschnitt der unbehandelten Kontrollen dargestellt. Die nekrotischen Schäden sind in % zur gesamten Blattfläche aufgeführt.

Die geprüften Verbindungen der Tabelle 1 bewirken eine Reduzierung des Neuzuwachses im Vergleich zur unbehandelten Kontrolle.

**Patentansprüche**
**Patentansprüche für folgende Vertragsstaaten : AT, BE, CH, DE, DK, FR, GB, GR, IT, LI, LU, NL, SE**

1. N-Pyridinsulfonyl-N'-pyrimidinyl- und -triazinylharnstoffe der Formel I

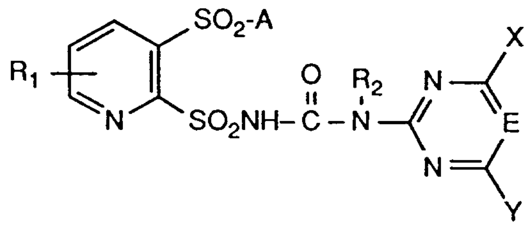

(I)

worin $R_1$ Wasserstoff, Halogen, $C_1$-$C_4$-Alkyl, $C_1$-$C_4$-Alkoxy, $C_1$-$C_4$-Alkylthio, $C_1$-$C_4$-Halogenalkoxy oder $CF_3$;
$R_2$ Wasserstoff oder $C_1$-$C_4$-Alkyl;
A durch Halogen ein- bis dreifach substituiertes $C_1$-$C_6$-Alkyl oder $C_3$-$C_6$-Cycloalkyl oder durch $C_1$-$C_4$-Alkoxy, $C_1$-$C_4$-Alkylthio, $C_1$-$C_4$-Alkylsulfonyl, $C_1$-$C_4$-Alkylsulfinyl, $C_2$-$C_4$-Alkenyl, $C_5$-$C_6$-Cycloalkenyl, Amino, $C_1$-$C_4$-Alkylamino, $C_1$-$C_4$-Dialkylamino oder $C_2$-$C_4$-Alkinyl substituiertes $C_1$-$C_4$-Alkyl oder $C_3$-$C_6$-Cycloalkyl, wobei der $C_2$-$C_4$-Alkenylrest sowie der $C_5$-$C_6$-Cycloalkenylrest noch ein- bis dreifach durch Halogen substituiert sein kann; oder $C_1$-$C_4$-Alkyl welches durch einen 4- bis 6-gliedrigen gesättigten Heterocyclus, der ein Heteroatom ausgewählt aus der Gruppe O, N und $SO_2$ enthält, substituiert ist;
X $C_1$-$C_3$-Alkyl, durch Halogen ein- bis dreifach substituiertes $C_1$-$C_3$-Alkyl, $C_1$-$C_3$-Alkoxy oder durch Halogen ein- bis dreifach substituiertes $C_1$-$C_3$-Alkoxy;
Y Halogen, $C_1$-$C_3$-Alkyl, durch Halogen ein- bis dreifach substituiertes $C_1$-$C_3$-Alkyl, $C_1$-$C_3$-Alkoxy, durch Halogen ein- bis dreifach substituiertes $C_1$-$C_3$-Alkoxy, Cyclopropyl, Methylamino oder Dimethylamino; und
E Stickstoff oder die Methingruppe bedeuten,
sowie die Salze dieser Verbindungen.

2. Verbindungen gemäss Anspruch 1, dadurch gekennzeichnet, dass
$R_1$ Wasserstoff, Halogen, $C_1$-$C_4$-Alkyl, $C_1$-$C_4$-Alkoxy, $C_1$-$C_4$-Alkylthio oder $CF_3$;
$R_2$ Wasserstoff oder $C_1$-$C_4$-Alkyl;
A durch Halogen ein- bis dreifach substituiertes $C_2$-$C_6$-Alkyl; oder durch $C_1$-$C_4$-Alkoxy, $C_1$-$C_4$-Alkylthio, $C_1$-$C_4$-Alkylsulfonyl, $C_1$-$C_4$-Alkylsulfinyl, $C_2$-$C_4$-Alkenyl oder $C_2$-$C_4$-Alkinyl substituiertes $C_1$-$C_4$-Alkyl, wobei der $C_2$-$C_4$-Alkenylrest noch ein-bis dreifach durch Halogen substituiert sein kann;
X $C_1$-$C_3$-Alkyl, durch Halogen ein- bis dreifach substituiertes $C_1$-$C_3$-Alkyl, $C_1$-$C_3$-Alkoxy oder durch Halogen ein- bis dreifach substituiertes $C_1$-$C_3$-Alkoxy;
Y Halogen, $C_1$-$C_3$-Alkyl, durch Halogen ein- bis dreifach substituiertes $C_1$-$C_3$-Alkyl, $C_1$-$C_3$-Alkoxy, durch Halogen ein- bis dreifach substituiertes $C_1$-$C_3$-Alkoxy, Cyclopropyl, Methylamino oder Dimethylamino; und
E Stickstoff oder die Methingruppe bedeuten,
sowie die Salze dieser Verbindungen.

3. Verbindungen gemäss Anspruch 1, dadurch gekennzeichnet, dass
$R_2$ Wasserstoff bedeutet.

4. Verbindungen gemäss Anspruch 1, dadurch gekennzeichnet, dass
X für $C_1$-$C_3$-Alkyl, $C_1$-$C_3$-Alkoxy oder durch Halogen ein- bis dreifach substituiertes $C_1$-$C_3$-Alkoxy; und
Y für Chlor, $C_1$-$C_3$-Alkyl, $C_1$-$C_3$-Alkoxy oder durch Halogen ein- bis dreifach substituiertes $C_1$-$C_3$-Alkoxy steht.

5. Verbindungen der Formel I gemäss Anspruch 1, dadurch gekennzeichnet, dass
X für Methyl, Methoxy, Ethoxy oder Difluormethoxy; und
Y für Methyl, Methoxy, Ethoxy, Difluormethoxy oder Chlor steht.

**6.** Verbindungen der Formel I gemäss Anspruch 1, dadurch gekennzeichnet, dass X für Methoxy oder Ethoxy; und Y für Methyl oder Methoxy steht.

**7.** Verbindungen der Formel I gemäss Anspruch 1, dadurch gekennzeichnet, dass E die Methinbrücke bedeutet.

**8.** Verbindungen der Formel I gemäss Anspruch 1, dadurch gekennzeichnet, dass $R_1$ für Wasserstoff, Methyl, Methoxy, Fluor oder Chlor steht.

**9.** Verbindungen der Formel I gemäss Anspruch 1, dadurch gekennzeichnet, dass
A $C_1$-$C_6$-Alkyl oder $C_3$-$C_6$-Cycloalkyl, welches ein- bis dreifach durch Halogen substituiert ist; oder $C_1$-$C_4$-Alkyl oder $C_3$-$C_6$-Cycloalkyl, welches durch $C_1$-$C_4$-Alkoxy, $C_5$-$C_6$-Cycloalkyl oder $C_2$-$C_4$-Alkenyl substituiert ist, bedeutet; wobei der $C_2$-$C_4$-Alkenylrest sowie der $C_5$-$C_6$-Cycloalkenylrest noch ein- bis dreifach durch Halogen substituiert sein kann.

**10.** Verbindungen der Formel I gemäss Anspruch 1, dadurch gekennzeichnet, dass
A durch Halogen ein- bis dreifach substituiertes $C_2$-$C_6$-Alkyl; oder durch $C_1$-$C_4$-Alkoxy oder $C_2$-$C_4$-Alkenyl substituiertes $C_1$-$C_4$-Alkyl bedeutet, wobei der $C_2$-$C_4$-Alkenylrest durch ein bis drei Halogenatome substituiert sein kann.

**11.** Verbindungen der Formel I gemäss Anspruch 10, dadurch gekennzeichnet, dass
$R_1$ und $R_2$ für Wasserstoff stehen.

**12.** Verbindungen der Formel I gemäss Anspruch 10, dadurch gekennzeichnet, dass
X für $C_1$-$C_3$-Alkyl, $C_1$-$C_3$-Alkoxy, oder durch Halogen ein- bis dreifach substituiertes $C_1$-$C_3$-Alkoxy und Y für $C_1$-$C_3$-Alkyl, $C_1$-$C_3$-Alkoxy, durch Halogen ein- bis dreifach substituiertes $C_1$-$C_3$-Alkoxy oder Chlor steht.

**13.** Verbindungen der Formel I gemäss Anspruch 10, dadurch gekennzeichnet, dass
X für Methyl, Methoxy, Ethoxy oder Difluormethoxy und Y für Methyl, Methoxy, Ethoxy, Difluormethoxy oder Chlor steht.

**14.** Verbindungen der Formel I gemäss Anspruch 10, dadurch gekennzeichnet, dass
X für Methoxy oder Ethoxy und Y für Methyl oder Methoxy steht.

**15.** Verbindungen der Formel I gemäss Anspruch 1, dadurch gekennzeichnet, dass
A Allyl, Methallyl, 2-Methylpropenyl, 3-Chlorallyl, Methoxyethyl, durch Fluor oder Chlor substituiertes $C_3$-$C_6$-Cycloalkyl oder 3-Fluorpropyl bedeutet.

**16.** Verbindungen der Formel I gemäss Anspruch 15, dadurch gekennzeichnet, dass
$R_1$ und $R_2$ für Wasserstoff stehen.

**17.** Verbindungen der Formel I gemäss Anspruch 1, dadurch gekennzeichnet, dass
$R_1$ Wasserstoff, Halogen, $C_1$-$C_2$-Alkyl, $C_1$-$C_2$-Alkoxy, $C_1$-$C_2$-Alkylthio, $C_1$-$C_2$-Halogenalkoxy oder $CF_3$;
$R_2$ Wasserstoff oder $C_1$-$C_2$-Alkyl;
A ein- bis dreifach durch Halogen substituiertes $C_1$-$C_4$-Alkyl oder $C_3$-$C_6$-Cycloalkyl, oder durch $C_1$-$C_2$-Alkoxy, $C_1$-$C_2$-Alkylthio, $C_1$-$C_2$-Alkylsulfonyl, $C_1$-$C_2$-Alkylsulfinyl, $C_2$-$C_3$-Alkenyl, $C_5$-$C_6$-Cycloalkenyl, Amino, $C_1$-$C_2$-Alkylamino, $C_1$-$C_4$-Dialkylamino oder $C_2$-$C_3$-Alkinyl substituiertes $C_1$-$C_2$-Alkyl oder $C_3$-$C_6$-Cycloalkyl, wobei der $C_2$-$C_3$-Alkenylrest sowie der $C_5$-$C_6$-Cycloalkenylrest noch ein- bis dreifach durch Halogen substituiert sein kann; oder $C_1$-$C_2$-Alkyl welches durch einen 4- bis 6-gliedrigen gesättigten Heterocyclus, der ein Heteroatom ausgewählt aus der Gruppe O, N und $SO_2$ enthält, substituiert ist;
X $C_1$-$C_2$-Alkyl, durch Halogen ein- bis dreifach substituiertes $C_1$-$C_2$-Alkyl, $C_1$-$C_2$-Alkoxy, oder durch Halogen ein- bis dreifach substituiertes $C_1$-$C_2$-Alkoxy;
Y Halogen, $C_1$-$C_2$-Alkyl, durch Halogen ein- bis dreifach substituiertes $C_1$-$C_2$-Alkyl, $C_1$-$C_2$-Alkoxy, durch Halogen ein- bis dreifach substituiertes $C_1$-$C_2$-Alkoxy, Cyclopropyl, Methylamino oder Dimethylamino bedeuten.

**18.** Verbindungen der Formel I gemäss Anspruch 1, dadurch gekennzeichnet, dass
$R_1$ Wasserstoff, Halogen, $C_1$-$C_2$-Alkyl, $C_1$-$C_2$-Alkylthio oder $CF_3$;
$R_2$ Wasserstoff oder $C_1$-$C_2$-Alkyl;
A ein- bis dreifach durch Halogen substituiertes $C_2$-$C_4$-Alkyl oder durch $C_1$-$C_2$-Alkoxy, $C_1$-$C_2$-Alkylthio, $C_1$-$C_2$-Alkylsulfonyl, $C_1$-$C_2$-Alkylsulfinyl, $C_2$-$C_3$-Alkenyl oder $C_2$-$C_3$-Alkinyl substituiertes $C_1$-$C_2$-Alkyl, wobei der $C_2$-$C_3$-Alkenylrest noch ein- bis dreifach durch Halogen substituiert sein kann;
X $C_1$-$C_2$-Alkyl, durch Halogen ein- bis dreifach substituiertes $C_1$-$C_2$-Alkyl, $C_1$-$C_2$-Alkoxy, oder durch Halogen ein- bis dreifach substituiertes $C_1$-$C_2$-Alkoxy;
Y Halogen, $C_1$-$C_2$-Alkyl, durch Halogen ein- bis dreifach substituiertes $C_1$-$C_2$-Alkyl, $C_1$-$C_2$-Alkoxy, durch Halogen ein- bis dreifach substituiertes $C_1$-$C_2$-Alkoxy, Cyclopropyl, Methylamino oder Dimethylamino bedeuten.

**19.** Verbindungen der Formel I gemäss Anspruch 1, dadurch gekennzeichnet, dass
$R_1$ und $R_2$ Wasserstoff;
A durch Halogen ein- bis dreifach substituiertes $C_1$-$C_4$-Alkyl oder durch $C_1$-$C_4$-Alkoxy, $C_5$-$C_6$-Cycloalkyl oder $C_2$-$C_4$-Alkenyl substituiertes $C_1$-$C_2$-Alkyl oder $C_3$-$C_6$-Cycloalkyl, wobei der $C_2$-$C_4$-Alkenylrest sowie der $C_5$-$C_6$-Cycloalkenylrest noch ein- bis dreifach durch Fluor oder Chlor substituiert sein kann, bedeutet.

**20.** Verbindungen gemäss Anspruch 16, dadurch gekennzeichnet, dass
A für Allyl, Methallyl, 3-Chlorallyl, Methoxyethyl, 2-Fluorisopropyl oder 3-Fluorpropyl;
X für Methyl, Methoxy, Ethoxy oder Difluormethoxy und
Y für Methyl, Methoxy, Ethoxy, Difluormethoxy oder Chlor steht.

**21.** Verbindungen der Formel I gemäss Anspruch 1, dadurch gekennzeichnet, dass
$R_1$ und $R_2$ Wasserstoff;
A durch Halogen ein- bis dreifach substituiertes $C_2$-$C_4$-Alkyl oder durch $C_1$-$C_4$-Alkoxy oder $C_2$-$C_4$-Alkenyl substituiertes $C_1$-$C_2$-Alkyl, wobei der $C_2$-$C_4$-Alkenylrest noch ein- bis dreifach durch Fluor oder Chlor substituiert sein kann, bedeutet.

**22.** Verbindungen gemäss Anspruch 16, dadurch gekennzeichnet, dass
A für Allyl, Methallyl, 3-Chlorallyl, Methoxyethyl, oder 3-Fluorpropyl;
X für Methyl, Methoxy, Ethoxy oder Difluormethoxy und
Y für Methyl, Methoxy, Ethoxy, Difluormethoxy oder Chlor steht.

**23.** Eine Verbindung gemäss Anspruch 1, das N-(3-Fluorpropylsulfonyl-pyridin-2-yl-sulfonyl)-N'-(4,6-dimethoxypyrimidin-2-yl)-harnstoff.

**24.** Eine Verbindung gemäss Anspruch 1, das N-(3-Allylsulfonyl-pyridin-2yl-sulfonyl)-N'-(4,6-dimethoxypyrimidin-2-yl)-harnstoff.

**25.** Eine Verbindung gemäss Anspruch 1, das N-(3-Methoxyethylsulfonyl-pyridin-2-yl-sulfonyl)-N'-(4,6-dimethoxypyrimidin-2-yl)-harnstoff.

**26.** Verfahren zur Herstellung der Verbindungen der Formel I gemäss einem der Ansprüche 1 bis 25, dadurch gekennzeichnet, dass man ein Pyridylsulfonamid der Formel II

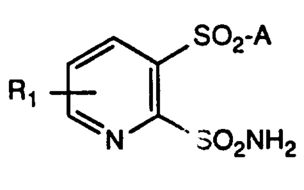

(II)

worin $R_1$ und A die unter Formel I im Anspruch 1 angegebene Bedeutung haben, mit einem N-Pyrimidinyl- oder N-Triazinylcarbamat der Formel IV

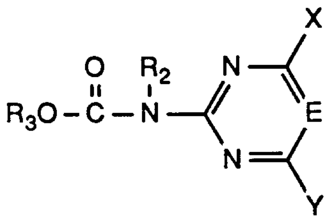

(IV)

worin X, Y, $R_2$ und E die unter Formel I in Anspruch 1 angegebene Bedeutung haben und $R_3$ $C_1$-$C_4$-Alkyl oder Phenyl, welches durch $C_1$-$C_4$-Alkyl oder Halogen substituiert sein kann, bedeutet; in Gegenwart einer Base umsetzt.

**27.** Verfahren zur Herstellung der Verbindungen der Formel I gemäss einem der Ansprüche 1 bis 25, dadurch gekennzeichnet, dass man eine Verbindung der Formel XVI

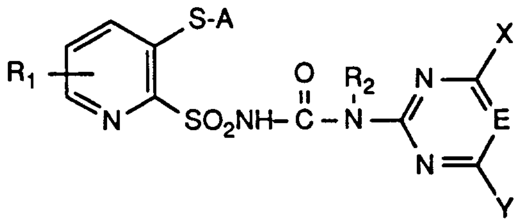

(XVI)

worin $R_1$, $R_2$, A, E, X und Y die unter Formel I in Anspruch 1 angegebene Bedeutung haben, oxidiert.

**28.** Verfahren zur Herstellung der Verbindungen der Formel I gemäss einem der Ansprüche 1 bis 25, dadurch gekennzeichnet, dass man ein Pyridylsulfonamid der Formel II

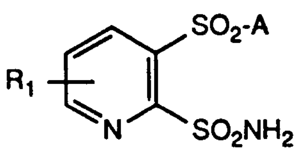

(II)

worin $R_1$ und A die unter Formel I im Anspruch 1 angegebene Bedeutung haben, in Gegenwart einer Base mit einem Pyrimidinyl- oder Triaziflylisocyanat der Formel VIII

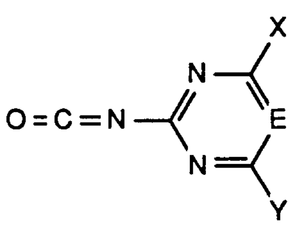

(VIII)

worin E, X und Y die unter Formel I angegebene Bedeutung haben, umsetzt.

**29.** Verfahren zur Herstellung der Pyridylsulfonamide der Formel II

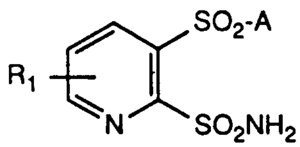

(II)

worin $R_1$ und A die unter Formel I im Anspruch 1 angegebene Bedeutung haben, dadurch gekennzeichnet, dass man ein 3-Mercaptopyridin-2-yl-sulfonamid der Formel IIIb

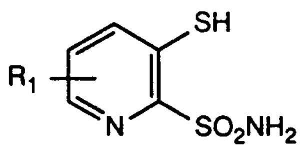

(IIIb)

worin $R_1$ die unter Formel I im Anspruch 1 angegebene Bedeutung hat, in Gegenwart einer Base mit einer Verbindung der Formel XVIII

Z-A (XVIII)

worin A die unter Formel I angegebene Bedeutung hat und Z für Chlor, Brom, Jod, $CH_3SO_2O$- oder

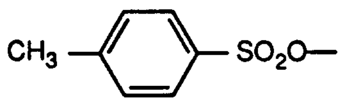

steht, zur Verbindung der Formel XX

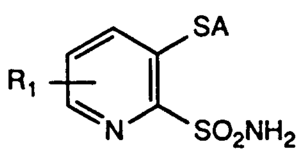

(XX)

worin A die unter Formel I angegebene Bedeutung hat, umsetzt, und diese anschliessend zur Verbindung der Formel II oxidiert.

**30.** Verbindungen der Formel II

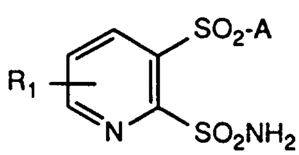

(II)

worin $R_1$ und A die unter Formel I im Anspruch 1 angegebene Bedeutung haben.

**31.** Verbindungen der Formel III

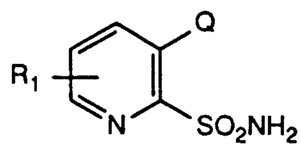

(III)

worin $R_1$ die unter Formel I angegebene Bedeutung hat und Q für Fluor oder -SH steht, mit der Maßgabe, daß Q nicht für -SH steht wenn $R_1$ Wasserstoff bedeutet.

**32.** Ein herbizides und den Pflanzenwuchs hemmendes Mittel, gekennzeichnet durch ein Gehalt an einem oder mehreren Sulfonylhamstoffen der Formel I, gemäss Anspruch 1.

**33.** Mittel gemäss Anspruch 31, dadurch gekennzeichnet, dass es zwischen 0,1 % und 95 % Wirkstoff der Formel I gemäss Anspruch 1 enthält.

**34.** Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**35.** Verfahren gemäss Anspruch 34, dadurch gekennzeichnet, dass man eine Wirkstoffmenge zwischen 0,001 und 5 kg pro Hektar appliziert.

**36.** Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**37.** Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**38.** Verfahren gemäss Anspruch 34, zur selektiven pre- oder post-emergenten Bekämpfung von Unkräutern in Nutzpflanzenkulturen.

**Patentansprüche für folgenden Vertragsstaat : ES**

**1.** Ein herbizides und den Pflanzenwuchs hemmendes Mittel, gekennzeichnet durch ein Gehalt an einem oder mehreren Sulfonylharnstoffen der Formel I

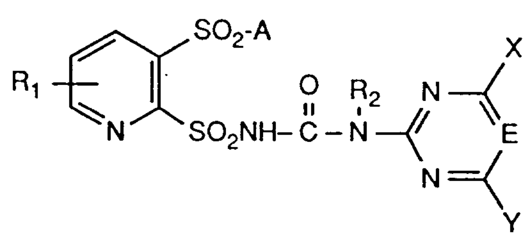

(I)

worin $R_1$ Wasserstoff, Halogen, $C_1$-$C_4$-Alkyl, $C_1$-$C_4$-Alkoxy, $C_1$-$C_4$-Alkylthio, $C_1$-$C_4$-Halogenalkoxy oder $CF_3$;
$R_2$ Wasserstoff oder $C_1$-$C_4$-Alkyl;
A durch Halogen ein- bis dreifach substituiertes $C_1$-$C_6$-Alkyl oder $C_3$-$C_6$-Cycloalkyl oder durch $C_1$-$C_4$-Alkoxy, $C_1$-$C_4$-Alkylthio, $C_1$-$C_4$-Alkylsulfonyl, $C_1$-$C_4$-Alkylsulfinyl, $C_2$-$C_4$-Alkenyl, $C_5$-$C_6$-Cycloalkenyl, Amino, $C_1$-$C_4$-Alkylamino, $C_1$-$C_4$-Dialkylamino oder $C_2$-$C_4$-Alkinyl substituiertes $C_1$-$C_4$-Alkyl oder $C_3$-$C_6$-Cycloalkyl, wobei der $C_2$-$C_4$-Alkenylrest sowie der $C_5$-$C_6$-Cycloalkenylrest noch ein- bis dreifach

durch Halogen substituiert sein kann; oder $C_1$-$C_4$-Alkyl welches durch einen 4- bis 6-gliedrigen gesättigten Heterocyclus, der ein Heteroatom ausgewählt aus der Gruppe O, N und $SO_2$ enthält, substituiert ist;
X $C_1$-$C_3$-Alkyl, durch Halogen ein- bis dreifach substituiertes $C_1$-$C_3$-Alkyl, $C_1$-$C_3$-Alkoxy oder durch Halogen ein- bis dreifach substituiertes $C_1$-$C_3$-Alkoxy;
Y Halogen, $C_1$-$C_3$-Alkyl, durch Halogen ein- bis dreifach substituiertes $C_1$-$C_3$-Alkyl, $C_1$-$C_3$-Alkoxy, durch Halogen ein- bis dreifach substituiertes $C_1$-$C_3$-Alkoxy, Cyclopropyl, Methylamino oder Dimethylamino; und
E Stickstoff oder die Methingruppe bedeuten.

**2.** Mittel gemäss Anspruch 1, dadurch gekennzeichnet, dass in der Formel I
$R_1$ Wasserstoff, Halogen, $C_1$-$C_4$-Alkyl, $C_1$-$C_4$-Alkoxy, $C_1$-$C_4$-Alkylthio oder $CF_3$;
$R_2$ Wasserstoff oder $C_1$-$C_4$-Alkyl;
A durch Halogen ein- bis dreifach substituiertes $C_2$-$C_6$-Alkyl; oder durch $C_1$-$C_4$-Alkoxy, $C_1$-$C_4$-Alkylthio, $C_1$-$C_4$-Alkylsulfonyl, $C_1$-$C_4$-Alkylsulfinyl, $C_2$-$C_4$-Alkenyl oder $C_2$-$C_4$-Alkinyl substituiertes $C_1$-$C_4$-Alkyl, wobei der $C_2$-$C_4$-Alkenylrest noch ein-bis dreifach durch Halogen substituiert sein kann;
X $C_1$-$C_3$-Alkyl, durch Halogen ein- bis dreifach substituiertes $C_1$-$C_3$-Alkyl, $C_1$-$C_3$-Alkoxy oder durch Halogen ein- bis dreifach substituiertes $C_1$-$C_3$-Alkoxy;
Y Halogen, $C_1$-$C_3$-Alkyl, durch Halogen ein- bis dreifach substituiertes $C_1$-$C_3$-Alkyl, $C_1$-$C_3$-Alkoxy, durch Halogen ein- bis dreifach substituiertes $C_1$-$C_3$-Alkoxy, Cyclopropyl, Methylamino oder Dimethylamino; und
E Stickstoff oder die Methingruppe bedeuten.

**3.** Mittel gemäss Anspruch 1, dadurch gekennzeichnet, dass in der Formel I
$R_2$ Wasserstoff bedeutet.

**4.** Mittel gemäss Anspruch 1, dadurch gekennzeichnet, dass in der Formel I
X für $C_1$-$C_3$-Alkyl, $C_1$-$C_3$-Alkoxy oder durch Halogen ein- bis dreifach substituiertes $C_1$-$C_3$-Alkoxy; und
Y für Chlor, $C_1$-$C_3$-Alkyl, $C_1$-$C_3$-Alkoxy oder durch Halogen ein- bis dreifach substituiertes $C_1$-$C_3$-Alkoxy steht.

**5.** Mittel gemäss Anspruch 1, dadurch gekennzeichnet, dass in der Formel I
X für Methyl, Methoxy, Ethoxy oder Difluormethoxy; und
Y für Methyl, Methoxy, Ethoxy, Difluormethoxy oder Chlor steht.

**6.** Mittel gemäss Anspruch 1, dadurch gekennzeichnet, dass in der Formel I X für Methoxy oder Ethoxy; und Y für Methyl oder Methoxy steht.

**7.** Mittel gemäss Anspruch 1, dadurch gekennzeichnet, dass in der Formel I E die Methinbrücke bedeutet.

**8.** Mittel gemäss Anspruch 1, dadurch gekennzeichnet, dass in der Formel I $R_1$ für Wasserstoff, Methyl, Methoxy, Fluor oder Chlor steht.

**9.** Mittel gemäss Anspruch 1, dadurch gekennzeichnet, dass in der Formel I
A $C_1$-$C_6$-Alkyl oder $C_3$-$C_6$-Cycloalkyl, welches ein- bis dreifach durch Halogen substituiert ist; oder $C_1$-$C_4$-Alkyl oder $C_3$-$C_6$-Cycloalkyl, welches durch $C_1$-$C_4$-Alkoxy, $C_5$-$C_6$-Cycloalkyl oder $C_2$-$C_4$-Alkenyl substituiert ist, bedeutet; wobei der $C_2$-$C_4$-Alkenylrest sowie der $C_5$-$C_6$-Cycloalkenylrest noch ein- bis dreifach durch Halogen substituiert sein kann.

**10.** Mittel gemäss Anspruch 1, dadurch gekennzeichnet, dass
A durch Halogen ein- bis dreifach substituiertes $C_2$-$C_6$-Alkyl; oder durch $C_1$-$C_4$-Alkoxy oder $C_2$-$C_4$-Alkenyl substituiertes $C_1$-$C_4$-Alkyl bedeutet, wobei der $C_2$-$C_4$-Alkenylrest durch ein bis drei Halogenatome substituiert sein kann.

**11.** Mittel gemäss Anspruch 10, dadurch gekennzeichnet, dass in der Formel I
$R_1$ und $R_2$ für Wasserstoff stehen.

**12.** Mittel gemäss Anspruch 10, dadurch gekennzeichnet, dass in der Formel I
X für $C_1$-$C_3$-Alkyl, $C_1$-$C_3$-Alkoxy, oder durch Halogen ein- bis dreifach substituiertes $C_1$-$C_3$-Alkoxy und Y für $C_1$-$C_3$-Alkyl, $C_1$-$C_3$-Alkoxy, durch Halogen ein- bis dreifach substituiertes $C_1$-$C_3$-Alkoxy oder Chlor steht.

**13.** Mittel gemäss Anspruch 10, dadurch gekennzeichnet, dass in der Formel I
X für Methyl, Methoxy, Ethoxy oder Difluormethoxy und Y für Methyl, Methoxy, Ethoxy, Difluormethoxy oder Chlor steht.

**14.** Mittel gemäss Anspruch 10, dadurch gekennzeichnet, dass in der Formel I
X für Methoxy oder Ethoxy und Y für Methyl oder Methoxy steht.

**15.** Mittel gemäss Anspruch 1, dadurch gekennzeichnet, dass in der Formel I
A Allyl, Methallyl, 2-Methylpropenyl, 3-Chlorallyl, Methoxyethyl, durch Fluor oder Chlor substituiertes $C_3$-$C_6$-Cycloalkyl oder 3-Fluorpropyl bedeutet.

**16.** Mittel gemäss Anspruch 15, dadurch gekennzeichnet, dass in der Formel I
$R_1$ und $R_2$ für Wasserstoff stehen.

**17.** Mittel gemäss Anspruch 1, dadurch gekennzeichnet, dass in der Formel I
$R_1$ Wasserstoff, Halogen, $C_1$-$C_2$-Alkyl, $C_1$-4-Alkoxy, $C_1$-$C_2$-Alkylthio, $C_1$-$C_2$-Halogenalkoxy oder $CF_3$;
$R_2$ Wasserstoff oder $C_1$-$C_2$-Alkyl;
A ein- bis dreifach durch Halogen substituiertes $C_1$-$C_4$-Alkyl oder $C_3$-$C_6$-Cycloalkyl, oder durch $C_1$-$C_2$-Alkoxy, $C_1$-$C_2$-Alkylthio, $C_1$-$C_2$-Alkylsulfonyl, $C_1$-$C_2$-Alkylsulfinyl, $C_2$-$C_3$-Alkenyl, $C_5$-$C_6$-Cycloalkenyl, Amino, $C_1$-$C_2$-Alkylamino, $C_1$-$C_4$-Dialkylamino oder $C_2$-$C_3$-Alkinyl substituiertes $C_1$-$C_2$-Alkyl oder $C_3$-$C_6$-Cycloalkyl, wobei der $C_2$-$C_3$-Alkenylrest sowie der $C_5$-$C_6$-Cycloalkenylrest noch ein- bis dreifach durch Halogen substituiert sein kann; oder $C_1$-$C_2$-Alkyl welches durch einen 4- bis 6-gliedrigen gesättigten Heterocyclus, der ein Heteroatom ausgewählt aus der Gruppe O, N und $SO_2$ enthält, substituiert ist;
X $C_1$-$C_2$-Alkyl, durch Halogen ein- bis dreifach substituiertes $C_1$-$C_2$-Alkyl, $C_1$-$C_2$-Alkoxy, oder durch Halogen ein- bis dreifach substituiertes $C_1$-$C_2$-Alkoxy;
Y Halogen, $C_1$-$C_2$-Alkyl, durch Halogen ein- bis dreifach substituiertes $C_1$-$C_2$-Alkyl, $C_1$-$C_2$-Alkoxy, durch Halogen ein- bis dreifach substituiertes $C_1$-$C_2$-Alkoxy, Cyclopropyl, Methylamino oder Dimethylamino bedeuten.

**18.** Mittel gemäss Anspruch 1, dadurch gekennzeichnet, dass in der Formel I
$R_1$ Wasserstoff, Halogen, $C_1$-$C_2$-Alkyl, $C_1$-$C_2$-Alkylthio oder $CF_3$;
$R_2$ Wasserstoff oder $C_1$-$C_2$-Alkyl;
A ein- bis dreifach durch Halogen substituiertes $C_2$-$C_4$-Alkyl oder durch $C_1$-$C_2$-Alkoxy, $C_1$-$C_2$-Alkylthio, $C_1$-$C_2$-Alkylsulfonyl, $C_1$-$C_2$-Alkylsulfinyl, $C_2$-$C_3$-Alkenyl oder $C_2$-$C_3$-Alkinyl substituiertes $C_1$-$C_2$-Alkyl, wobei der $C_2$-$C_3$-Alkenylrest noch ein- bis dreifach durch Halogen substituiert sein kann;
X $C_1$-$C_2$-Alkyl, durch Halogen ein- bis dreifach substituiertes $C_1$-$C_2$-Alkyl, $C_1$-$C_2$-Alkoxy, oder durch Halogen ein- bis dreifach substituiertes $C_1$-$C_2$-Alkoxy;
Y Halogen, $C_1$-$C_2$-Alkyl, durch Halogen ein- bis dreifach substituiertes $C_1$-$C_2$-Alkyl, $C_1$-$C_2$-Alkoxy, durch Halogen ein- bis dreifach substituiertes $C_1$-$C_2$-Alkoxy, Cyclopropyl, Methylamino oder Dimethylamino bedeuten.

**19.** Mittel gemäss Anspruch 1, dadurch gekennzeichnet, dass in der Formel I
$R_1$ und $R_2$ Wasserstoff;
A durch Halogen ein- bis dreifach substituiertes $C_1$-$C_4$-Alkyl oder durch $C_1$-$C_4$-Alkoxy, $C_5$-$C_6$-Cycloalkyl oder $C_2$-$C_4$-Alkenyl substituiertes $C_1$-$C_2$-Alkyl oder $C_3$-$C_6$-Cycloalkyl, wobei der $C_2$-$C_4$-Alkenylrest sowie der $C_5$-$C_6$-Cycloalkenylrest noch ein- bis dreifach durch Fluor oder Chlor substituiert sein kann, bedeutet.

**20.** Mittel gemäss Anspruch 16, dadurch gekennzeichnet, dass in der Formel I
A für Allyl, Methallyl, 3-Chlorallyl, Methoxyethyl, 2-Fluorisopropyl oder 3-Fluorpropyl;
X für Methyl, Methoxy, Ethoxy oder Difluormethoxy und
Y für Methyl, Methoxy, Ethoxy, Difluormethoxy oder Chlor steht.

**21.** Mittel gemäss Anspruch 1, dadurch gekennzeichnet, dass in der Formel I
$R_1$ und $R_2$ Wasserstoff;
A durch Halogen ein- bis dreifach substituiertes $C_2$-$C_4$-Alkyl oder durch $C_1$-$C_4$-Alkoxy oder $C_2$-$C_4$-Alkenyl substituiertes $C_1$-$C_2$-Alkyl, wobei der $C_2$-$C_4$-Alkenylrest noch ein- bis dreifach durch Fluor oder Chlor substituiert sein kann, bedeutet.

**22.** Mittel gemäss Anspruch 16, dadurch gekennzeichnet, dass in der Formel I
A für Allyl, Methallyl, 3-Chlorallyl, Methoxyethyl, oder 3-Fluorpropyl;
X für Methyl, Methoxy, Ethoxy oder Difluormethoxy und
Y für Methyl, Methoxy, Ethoxy, Difluormethoxy oder Chlor steht.

**23.** Mittel gemäss Anspruch 1, dadurch gekennzeichnet, dass es als Verbindung der Formel I N-(3-Fluorpropylsulfonyl-pyridin-2-yl-sulfonyl)-N'-(4,6 -dimethoxypyrimidin-2-yl)-harnstoff enthält.

**24.** Mittel gemäss Anspruch 1, dadurch gekennzeichnet, dass es als Verbindung der Formel N-(3-Allylsulfonyl-pyridin-2-yl-sulfonyl)-N'-(4,6-dimet hoxypyrimidin-2-yl)-harnstoff enthält.

**25.** Mittel gemäss Anspruch 1, dadurch gekennzeichnet, dass es als Verbindung der Formel N-(3-Methoxyethylsulfonyl-pyridin-2-yl-sulfonyl)-N'-(4, 6-dimethoxypyrimidin-2-yl)-harnstoff enthält.

**26.** Verfahren zur Herstellung der Verbindungen der Formel I gemäss einem der Ansprüche 1 bis 25, dadurch gekennzeichnet, dass man ein Pyridylsulfonamid der Formel II

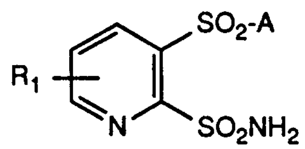

(II)

worin $R_1$ und A die unter Formel I im Anspruch 1 angegebene Bedeutung haben, mit einem N-Pyrimidinyl- oder N-Triazinylcarbamat der Formel IV

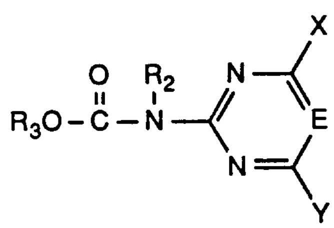

(IV)

worin X, Y, $R_2$ und E die unter Formel I in Anspruch 1 angegebene Bedeutung haben und $R_3$ $C_1$-$C_4$-Alkyl oder Phenyl, welches durch $C_1$-$C_4$-Alkyl oder Halogen substituiert sein kann, bedeutet; in Gegenwart einer Base umsetzt.

**27.** Verfahren zur Herstellung der Verbindungen der Formel I gemäss einem der Ansprüche 1 bis 25, dadurch gekennzeichnet, dass man eine Verbindung der Formel XVI

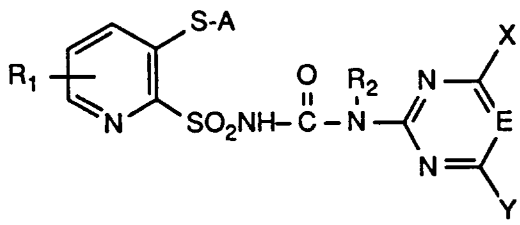

(XVI)

worin $R_1$, $R_2$, A, E, X und Y die unter Formel I in Anspruch 1 angegebene Bedeutung haben, oxidiert

**28.** Verfahren zur Herstellung der Verbindungen der Formel I gemäss einem der Ansprüche 1 bis 25, dadurch gekennzeichnet, dass man ein Pyridylsulfonamid der Formel II

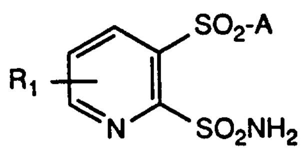

(II)

worin $R_1$ und A die unter Formel I im Anspruch 1 angegebene Bedeutung haben, in Gegenwart einer Base mit einem Pyrimidinyl- oder Triazinylisocyanat der Formel VIII

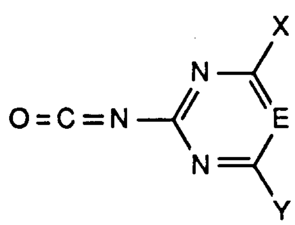

(VIII)

worin E, X und Y die unter Formel I angegebene Bedeutung haben, umsetzt.

**29.** Verfahren zur Herstellung der Pyridylsulfonamide der Formel II

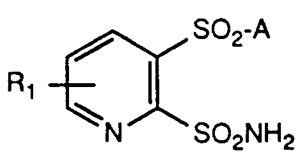

(II)

worin $R_1$ und A die unter Formel I im Anspruch 1 angegebene Bedeutung haben, dadurch gekennzeichnet, dass man ein 3-Mercaptopyridin-2-yl-sulfonamid der Formel IIIb

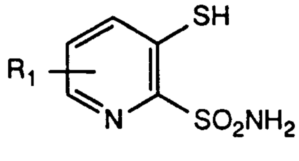

(IIIb)

worin $R_1$ die unter Formel I im Anspruch 1 angegebene Bedeutung hat, in Gegenwart einer Base mit einer Verbindung der Formel XVIII

Z-A (XVIII)

worin A die unter Formel I angegebene Bedeutung hat und Z für Chlor, Brom, Jod, $CH_3SO_2O$- oder

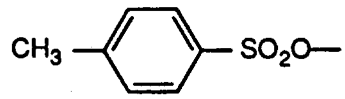

steht, zur Verbindung der Formel XX

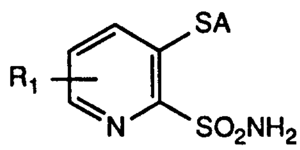

(XX)

worin A die unter Formel I angegebene Bedeutung hat, umsetzt, und diese anschliessend zur Verbindung der Formel II oxidiert.

**30.** Mittel gemäss Anspruch 1, dadurch gekennzeichnet, dass es zwischen 0,1 % und 95 % Wirkstoff der Formel I gemäss Anspruch 1 enthält.

**31.** Verfahren zur Bekämpfung unerwünschten Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**32.** Verfahren gemäss Anspruch 31, dadurch gekennzeichnet, dass man eine Wirkstoffmenge zwischen 0,001 und 5 kg pro Hektar appliziert.

**33.** Verfahren zur Hemmung des Pflanzenwachstums, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**34.** Verfahren zur Beeinflussung des Pflanzenwachstums zum Zweck der Ertragssteigerung, dadurch gekennzeichnet, dass man einen Wirkstoff der Formel I, gemäss Anspruch 1, oder ein diesen Wirkstoff enthaltendes Mittel in einer wirksamen Menge auf die Pflanzen oder deren Lebensraum appliziert.

**35.** Verfahren gemäss Anspruch 31, zur selektiven pre- oder post-emergenten Bekämpfung von Unkräutern in Nutzpflanzenkulturen.

**Claims**

**Claims for the following Contracting States : AT, BE, CH, DE, DK, FR, GB, GR, IT, LI, LU, NL, SE**

**1.** An N-pyridinesulfonyl-N'-pyrimidinyl- and -triazinylurea of the formula I

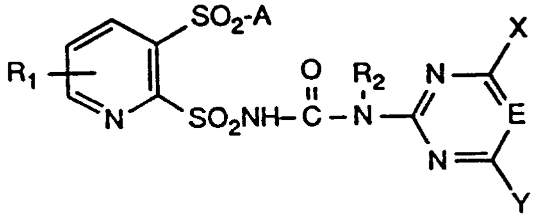

(I)

in which

$R_1$ is hydrogen, halogen, $C_1$-$C_4$alkyl, $C_1$-$C_4$alkoxy, $C_1$-$C_4$alkylthio, $C_1$-$C_4$haloalkoxy or $CF_3$; $R_2$ is hydrogen or $C_1$-$C_4$alkyl; A is $C_1$-$C_6$alkyl or $C_3$-$C_6$cycloalkyl, each of which is monosustituted to trisubstituted by halogen, or is $C_1$-$C_4$alkyl or $C_3$-$C_6$cycloalkyl, each of which is substituted by $C_1$-$C_4$alkoxy, $C_1$-$C_4$alkthio, $C_1$-$C_4$alkylsulfonyl, $C_1$-$C_4$alkylsulfinyl, $C_2$-$C_4$alkenyl, $C_5$-$C_6$cycloalkenyl, amino, $C_1$-$C_4$alkylamino, $C_1$-$C_4$dialkylamino or $C_2$-$C_4$alkynyl, it being possible for the $C_2$-$C_4$alkenyl radical and the $C_5$-$C_6$cycloalkenyl radical to be additionally monosubstituted to trisubstituted by halogen; or A is $C_1$-$C_4$alkyl which is substituted by a 4- to 6-membered saturated heterocycle which contains a hetero atom selected from the group comprising O, N and $SO_2$; X is $C_1$-$C_3$alkyl, $C_1$-$C_3$alkyl which is monosubstituted to trisubstituted by halogen, or is $C_1$-$C_3$alkoxy or $C_1$-$C_3$alkoxy which is monosubstituted to trisubstituted by halogen; Y is halogen, $C_1$-$C_3$alkyl, $C_1$-$C_3$alkyl which is monosubstituted to trisubstituted by halogen, or is $C_1$-$C_3$alkoxy, $C_1$-$C_3$alkoxy which is monosubstituted to trisubstituted by halogen, or is cyclopropyl, methylamino or dimethylamino; and E is nitrogen or the methine group, or a salt of this compound.

**2.** A compound according to claim 1, wherein $R_1$ is hydrogen, halogen, $C_1$-$C_4$alkyl, $C_1$-$C_4$alkoxy, $C_1$-$C_4$alkylthio, or $CF_3$; $R_2$ is hydrogen or $C_1$-$C_4$alkyl; A is $C_2$-$C_6$alkyl which is monosubstituted to trisubstituted by halogen; or A is $C$-$_1C_4$alkyl which is substituted by $C_1$-$C_4$alkoxy, $C_1$-$C_4$alkylthio, $C_1$-$C_4$alkylsulfonyl, $C_1$-$C_4$alkylsulfinyl, $C_2$-$C_4$alkenyl or $C_2$-$C_4$alkynyl, it being possible for the $C_2$-$C_4$alkenyl radical to be additionally monosubstituted to trisubstituted by halogen; X is $C_1$-$C_3$alkyl or $C_1$-$C_3$alkyl which is monosubstituted to trisubstituted by halogen, or is $C_1$-$C_3$alkoxy or $C_1$-$C_3$alkoxy which is monosubstituted or trisubstituted by halogen; Y is halogen, $C_1$-$C_3$alkyl, $C_1$-$C_3$alkyl which is monosubstituted to trisubstituted by halogen or is $C_1$-$C_3$alkoxy, $C_1$-$C_3$alkoxy which is monosubstituted to trisubstituted by halogen, or is cyclopropyl, methylamino or dimethylamino; and E is nitrogen or the methine group, or a salt of this compound.

**3.** A compound according to claim 1, wherein $R_2$ is hydrogen.

**4.** A compound according to claim 1, wherein X is $C_1$-$C_3$alkyl, $C_1$-$C_3$alkoxy or $C_1$-$C_3$alkoxy which is monosubstituted to trisubstituted by halogen; and Y is chlorine, $C_1$-$C_3$alkyl, $C_1$-$C_3$alkoxy or $C_1$-$C_3$alkoxy which is monosubstituted to trisubstituted by halogen.

**5.** A compound of the formula I according to claim 1, wherein X is methyl, methoxy, ethoxy or difluoromethoxy; and Y is methyl, methoxy, ethoxy, difluoromethoxy or chlorine.

**6.** A compound of the formula I according to claim 1, wherein X is methoxy or ethoxy; and Y is methyl or methoxy.

**7.** A compound of the formula I according to claim 1, wherein E is the methine bridge.

**8.** A compound of the formula I according to claim 1, wherein $R_1$ is hydrogen, methyl, methoxy, fluorine or chlorine.

**9.** A compound of the formula I according to claim 1, wherein A is $C_1$-$C_6$alkyl or $C_3$-$C_6$cycloalkyl, each of which is monosubstituted to trisubstituted by halogen; or A is $C_1$-$C_4$alkyl or $C_3$-$C_6$cycloalkyl, each of which is substituted by $C_1$-$C_4$alkoxy, $C_5$-$C_6$cycloalkyl or $C_2$-$C_4$alkenyl; it being possible for the $C_2$-$C_4$alkenyl radical and for the $C_5$-$C_6$cycloalkenyl radical to be additionally monosubstituted to trisubstituted by halogen.

**10.** A compound of the formula I according to claim 1, wherein A is $C_2$-$C_6$alkyl which is monosubstituted to trisubstituted by halogen; or A is $C_1$-$C_4$alkyl which is substituted by $C_1$-$C_4$alkoxy or $C_2$-$C_4$alkenyl, it being possible for the $C_2$-$C_4$alkenyl radical to be substituted by one to three halogen atoms.

**11.** A compound of the formula I according to claim 10, wherein $R_1$ and $R_2$ are hydrogen.

**12.** A compound of the formula I according to claim 10, wherein X is $C_1$-$C_3$alkyl, $C_1$-$C_3$alkoxy or $C_1$-$C_3$alkoxy which is monosubstituted to trisubstituted by halogen and Y is $C_1$-$C_3$alkyl, $C_1$-$C_3$alkoxy, $C_1$-$C_3$alkoxy which is monosubstituted to trisubstituted by halogen, or is chlorine.

**13.** A compound of the formula I according to claim 10, wherein X is methyl, methoxy, ethoxy or difluoromethoxy and Y is methyl, methoxy, ethoxy, difluoromethoxy or chlorine.

**14.** A compound of the formula I according to claim 10, wherein X is methoxy or ethoxy and Y is methyl or methoxy.

**15.** A compound of the formula I according to claim 1, wherein A is allyl, methallyl, 2-methylpropenyl, 3-chloroallyl, methoxyethyl, $C_3$-$C_6$cycloalkyl which is substituted by fluorine or chlorine, or is 3-fluoropropyl.

**16.** A compound of the formula I according to claim 15, wherein $R_1$ and $R_2$ are hydrogen.

**17.** A compound of the formula I according to claim 1, wherein $R_1$ is hydrogen, halogen, $C_1$-$C_2$alkyl, $C_1$-$C_2$alkoxy, $C_1$-$C_2$alkylthio, $C_1$-$C_2$haloalkoxy or $CF_3$; $R_2$ is hydrogen or $C_1$-$C_2$alkyl; A is $C_1$-$C_4$alkyl or $C_3$-$C_6$cycloalkyl, each of which is monosubstituted to trisubstituted by halogen, or is $C_1$-$C_2$alkyl or $C_3$-$C_6$cycloalkyl, each of which is substituted by $C_1$-$C_2$alkoxy, $C_1$-$C_2$alkylthio, $C_1$-$C_2$alkylsulfonyl, $C_1$-$C_2$alkylsulfinyl, $C_2$-$C_3$alkenyl, $C_5$-$C_6$cycloalkenyl, amino, $C_1$-$C_2$alkylamino, $C_1$-$C_4$dialkylamino or $C_2$-$C_3$alkynyl, it being possible for the $C_2$-$c_3$alkenyl radical and for the $C_5$-$C_6$cycloalkenyl radical to be additionally monosubstituted to trisubstituted by halogen; or A is $C_1$-$C_2$alkyl which is substituted by a 4- to 6-membered saturated heterocycle which contains a hetero atom selected from the group comprising O, N and $SO_2$; X is $C_1$-$C_2$alkyl, $C_1$-$C_2$alkyl which is monosubstituted to trisubstituted by halogen, or is $C_1$-$C_2$alkoxy or $C_1$-$C_2$alkoxy which is monosubstituted to trisubstituted by halogen; Y is halogen, $C_1$-$C_2$alkyl, $C_1$-$C_2$alkyl which is monosubstituted to trisubstituted by halogen, or is $C_1$-$C_2$alkoxy, $C_1$-$C_2$alkoxy which is monosubstituted to trisubstituted by halogen, or is cyclopropyl, methylamino or dimethylamino.

**18.** A compound of the formula I according to claim 1, wherein $R_1$ is hydrogen, halogen, $C_1$-$C_2$alkyl, $C_1$-$C_2$alkylthio or $CF_3$; $R_2$ is hydrogen or $C_1$-$C_2$alkyl; A is $C_2$-$C_4$alkyl which is monosubstituted to trisubstituted by halogen or is $C_1$-$C_2$alkyl which is substituted by $C_1$-$C_2$alkoxy, $C_1$-$C_2$alkylthio, $C_1$-$C_2$alkylsulfonyl, $C_1$-$C_2$alkylsulfinyl, $C_2$-$C_3$alkenyl or $C_2$-$C_3$alkynyl, it being possible for the $C_2$-$C_3$alkenyl radical to be additionally monosubstituted to trisubstituted by halogen; X is $C_1$-$C_2$alkyl, $C_1$-$C_2$alkyl which is monosubstituted to trisubstituted by halogen, or is $C_1$-$C_2$alkoxy or $C_1$-$C_2$alkoxy which is monosubstituted to trisubstituted by halogen; Y is halogen, $C_1$-$C_2$alkyl, $C_1$-$C_2$alkyl which is monosubstituted to trisubstituted by halogen, or is $C_1$-$C_2$alkoxy, $C_1$-$C_2$alkoxy which is monosubstituted to trisubstituted by halogen, or is cyclopropyl, methylamino or dimethylamino.

**19.** A compound of the formula I according to claim 1, wherein $R_1$ and $R_2$ are hydrogen; A is $C_1$-$C_4$cycloalkyl which is monosubstituted to trisubstituted by halogen, or is $C_1$-$C_2$alkyl or $C_3$-$C_6$cycloalkyl, each of which is substituted by $C_1$-$C_4$alkoxy, $C_5$-$C_6$cycloalkyl or $C_2$-$C_4$alkenyl, it being possible for the $C_2$-$C_4$alkenyl radical and for the $C_5$-$C_6$cycloalkenyl radical to be additionally monosubstituted to trisubstituted by fluorine or chlorine.

**20.** A compound according to claim 16, wherein A is allyl, methallyl, 3-chloroallyl, methoxyethyl, 2-fluoroisopropyl or 3-fluoropropyl; X is methyl, methoxy, ethoxy or difluoromethoxy and Y is methyl, methoxy, ethoxy difluoromethoxy or chlorine.

**21.** A compound of the formula I according to claim 1, wherein $R_1$ and $R_2$ are hydrogen; A is $C_2$-$C_4$alkyl which is monosubstituted to trisubstituted by halogen, or is $C_1$-$C_2$alkyl which is substituted by $C_1$-$C_4$alkoxy or $C_2$-$C_4$alkenyl, it being possible for the $C_2$-$C_4$alkenyl radical to be additionally monosubstituted to trisubstituted by fluorine or chlorine.

**22.** A compound according to claim 16, wherein A is allyl, methallyl, 3-chloroallyl, methoxyethyl or 3-fluoropropyl; X is methyl, methoxy, ethoxy or difluoromethoxy and Y is methyl, methoxy, ethoxy, difluoromethoxy or chlorine.

**23.** A compound according to claim 1, which is N-(3-fluoropropylsulfonylpyridin-2-ylsulfonyl)-N'-(4,6-dimethoxypyrimidin-2-yl)urea.

**24.** A compound according to claim 1, which is N-(3-allylsulfonylpyridin-2-ylsulfonyl)-N'-(4,6-dimethoxypyrimidin-2-yl)urea.

**25.** A compound according to claim 1, which is N-(3-methoxyethylsulfonylpyridin-2-ylsulfonyl)-N'-(4,6-dimethoxypyrimidin-2-yl)urea.

**26.** A process for the preparation of the compounds of the formula I according to one of claims 1 to 25, wherein a pyridylsulfonamide of the formula II

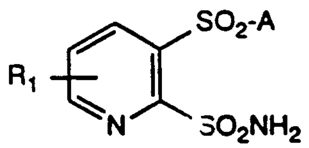

(II)

in which $R_1$ and A are as defined under formula I in claim 1 are reacted with an N-pyrimidinyl carbamate or N-triazinyl carbamate of the formula IV

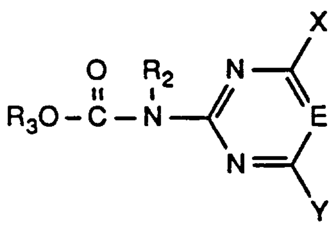

(IV)

in which X, Y, $R_2$ and E are as defined under formula I in claim 1 and $R_3$ is $C_1$-$C_4$alkyl or phenyl which can be substituted by $C_1$-$C_4$alkyl or halogen, in the presence of a base.

**27.** A process for the preparation of the compounds of the formula I according to one of claims 1 to 25, wherein a compound of the formula XVI

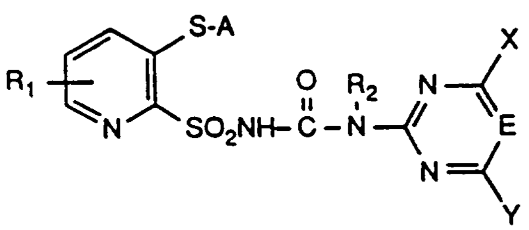

(XVI)

in which $R_1$, $R_2$, A, E, X and Y are as defined under formula I in claim 1, is oxidized.

**28.** A process for the preparation of the compounds of the formula I according to one of claims 1 to 25, wherein a pyridylsulfonamide of the formula II

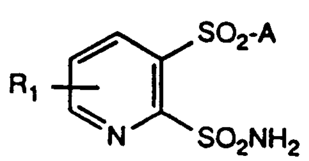

(II)

in which $R_1$ and A are as defined under formula I in claim 1, is reacted with a pyrimidinyl isocyanate or triazinyl isocyanate of the formula VIII

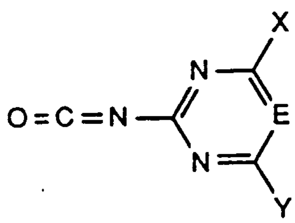

(VIII)

in which E, X, and Y are as defined under formula I, in the presence of a base.

**29.** A process for the preparation of pyridylsulfonamides of the formula II

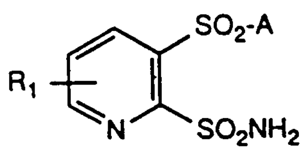

(II)

in which $R_1$ and A are as defined under formula I in claim 1, wherein a 3-mercaptopyridin-2-ylsulfonamide of the formula IIIb

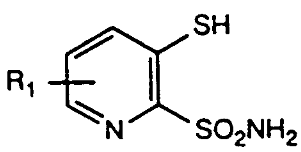

(IIIb)

in which $R_1$ is as defined under formula I in claim 1, is reacted with a compound of the formula XVIII

Z-A (XVIII)

in which A is as defined under formula I and Z is chlorine, bromine, iodine, $CH_3SO_2O$- or

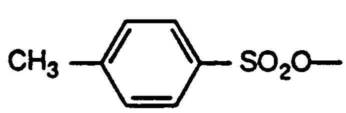

,

in the presence of a base to give the compound of the formula XX

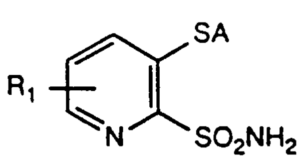

(XX)

in which A is as defined under formula I, and this compound is subsequently oxidized to give the compound of the formula II.

**30.** A compound of the formula II

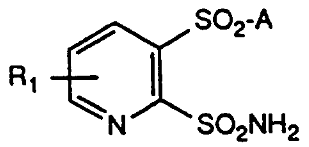

(II)

in which $R_1$ and A are as defined under formula I in claim 1.

**31.** A compound of the formula III

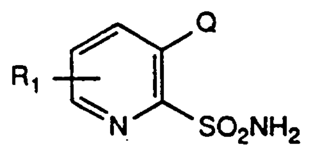

(III)

in which $R_1$ is as defined under formula I and Q is fluorine or -SH, with the proviso that Q is not -SH if $R_1$ is hydrogen.

**32.** A herbicidal and plant-growth-inhibiting composition, which contains one or more sulfonylureas of the formula I, according to claim 1.

**33.** A composition according to claim 31, which contains between 0.1 % and 95 % of active substance of the formula I according to claim 1.

**34.** A method for controlling undesired plant growth, wherein an active substance of the formula I, according to claim 1, or a composition containing this active substance, is applied in an effective amount to the plants or their environment.

**35.** A method according to claim 34, wherein an amount of active substance of between 0.001 and 5 kg per hectare is applied.

**36.** A method for inhibiting plant growth, wherein an active substance of the formula I, according to claim 1, or a composition containing this active substance, is applied in an effective amount to the plants or their environment.

**37.** A methox for influencing plant growth with the purpose of increasing the yield, wherein an active substance of the formula I, according to claim 1, or a composition containing this active substance, is applied in an effective amount to the plants or their environment.

**38.** A method according to claim 34 for selective weed control in crops, either pre- or post-emergence.

**Claims for the following Contracting State : ES**

**1.** A herbicidal and plant-growth-inhibiting composition, which contains one or more sulfonylureas of the formula I

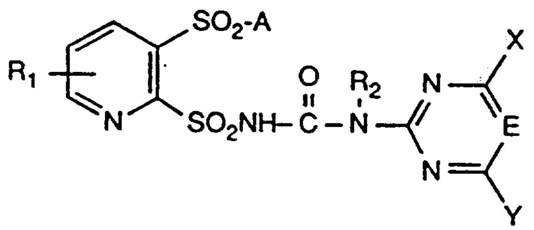

(I)

in which

$R_1$ is hydrogen, halogen, $C_1$-$C_4$alkyl, $C_1$-$C_4$alkoxy, $C_1$-$C_4$alkylthio, $C_1$-$C_4$haloalkoxy or $CF_3$; $R_2$ is hydrogen or $C_1$-$C_4$alkyl, A is $C_1$-$C_6$alkyl or $C_3$-$C_6$cycloalkyl, each of which is monosustituted to trisubstituted by halogen, or is $C_1$-$C_4$alkyl or $C_3$-$C_6$cycloalkyl, each of which is substituted by $C_1$-$C_4$alkoxy, $C_1$-$C_4$alkylthio, $C_1$-$C_4$alkylsulfonyl, $C_1$-$C_4$alkylsulfinyl, $C_2$-$C_4$alkenyl, $C_5$-$C_6$cycloalkenyl, amino, $C_1$-$C_4$alkylamino, $C_1$-$C_4$dialkylamino or $C_2$-$C_4$alkynyl, it being possible for the $C_2$-$C_4$alkenyl radical and the $C_5$-$C_6$cycloalkenyl radical to be additionally monosubstituted to trisubstituted by halogen; or A is $C_1$-$C_4$alkyl which is substituted by a 4- to 6-membered saturated heterocycle which contains a hetero atom selected from the group comprising O, N and $SO_2$; X is $C_1$-$C_3$alkyl, $C_1$-$C_3$alkyl which is monosubstituted to trisubstituted by halogen, or is $C_1$-$C_3$alkoxy or $C_1$-$C_3$alkoxy which is monosubstituted to trisubstituted by halogen; Y is halogen, $C_1$-$C_3$alkyl, $C_1$-$C_3$alkyl which is monosubstituted to trisubstituted by halogen, or is $C_1$-$C_3$alkoxy, $C_1$-$C_3$alkoxy which is monosubstituted to trisubstituted by halogen, or is cyclopropyl, methylamino or dimethylamino; and E is nitrogen or the methine group,

**2.** A composition according to claim 1, wherein, in formula I, $R_1$ is hydrogen, halogen; $C_1$-$C_4$alkyl, $C_1$-$C_4$alkoxy, $C_1$-$C_4$alkylthio or $CF_3$; $R_2$ is hydrogen or $C_1$-$C_4$alkyl; A is $C_2$-$C_6$alkyl which is monosubstituted to trisubstituted by halogen; or A is $C_1$-$C_4$alkyl which is substituted by $C_1$-$C_4$alkoxy, $C_1$-$C_4$alkylthio, $C_1$-$C_4$alkylsulfonyl, $C_1$-$C_4$alkylsulfinyl, $C_2$-$C_4$alkenyl or $C_2$-$C_4$alkynyl, it being possible for the $C_2$-$C_4$alkenyl radical to be additionally monosubstituted to trisubstituted by halogen; X is $C_1$-$C_3$alkyl or $C_1$-$C_3$alkyl which is monosubstituted to trisubstituted by halogen, or is $C_1$-$C_3$alkoxy or $C_1$-$C_3$alkoxy which is monosubstituted or trisubstituted by halogen; Y is halogen, $C_1$-$C_3$alkyl, $C_1$-$C_3$alkyl which is monosubstituted to trisubstituted by halogen, or is $C_1$-$C_3$alkoxy, $C_1$-$C_3$alkoxy which is monosubstituted to trisubstituted by halogen, or is cyclopropyl, methylamino or dimethylamino; and E is nitrogen or the methine group.

**3.** A composition according to claim 1, wherein, in formula I, $R_2$ is hydrogen.

**4.** A composition according to claim 1, wherein, in formula I, X is $C_1$-$C_3$alkyl, $C_1$-$C_3$alkoxy or $C_1$-$C_3$alkoxy which is monosubstituted to trisubstituted by halogen; and Y is chlorine, $C_1$-$C_3$alkyl, $C_1$-$C_3$alkoxy or $C_1$-$C_3$alkoxy which is monosubstituted to trisubstituted by halogen.

**5.** A composition according to claim 1, wherein, in formula I, X is methyl, methoxy, ethoxy or difluoromethoxy; and Y is methyl, methoxy, ethoxy, difluoromethoxy or chlorine.

**6.** A composition according to claim 1, wherein, in formula I, X is methoxy or ethoxy; and Y is methyl or methoxy.

**7.** A composition according to claim 1, wherein, in formula I, E is the methine bridge.

**8.** A composition according to claim 1, wherein, in formula I, $R_1$ is hydrogen, methyl, methoxy, fluorine or chlorine.

**9.** A composition according to claim 1, wherein, in formula I, A is $C_1$-$C_6$alkyl or $C_3$-$C_6$cycloalkyl, each of which is monosubstituted to trisubstituted by halogen; or A is $C_1$-$C_4$alkyl or $C_3$-$C_6$cycloalkyl, each of which is substituted by $C_1$-$C_4$alkoxy, $C_5$-$C_6$cycloalkyl or $C_2$-$C_4$alkenyl; it being possible for the $C_2$-$C_4$alkenyl radical and for the $C_5$-$C_6$cycloalkenyl radical to be additionally monosubstituted to trisubstituted by halogen.

**10.** A composition according to claim 1, wherein A is $C_2$-$C_6$alkyl which is monosubstituted to trisubstituted by halogen; or A is $C_1$-$C_4$alkyl which is substituted by $C_1$-$C_4$alkoxy or $C_2$-$C_4$alkenyl, it being possible for the $C_2$-$C_4$alkenyl radical to be substituted by one to three halogen atoms.

**11.** A composition according to claim 10, wherein, in formula I, $R_1$ and $R_2$ are hydrogen.

**12.** A composition according to claim 10, wherein, in formula I, X is $C_1$-$C_3$alkyl, $C_1$-$C_3$alkoxy or $C_1$-$C_3$alkoxy which is monosubstituted to trisubstituted by halogen and Y is $C_1$-$C_3$alkyl, $C_1$-$C_3$alkoxy, $C_1$-$C_3$alkoxy which is monosubstituted to trisubstituted by halogen, or is chlorine.

**13.** A composition according to claim 10, wherein, in formula I, X is methyl, methoxy, ethoxy or difluoromethoxy and Y is methyl, methoxy, ethoxy, difluoromethoxy or chlorine.

**14.** A composition according to claim 10, wherein, in formula I, X is methoxy or ethoxy and Y is methyl or methoxy.

**15.** A composition according to claim 1, wherein, in formula I, A is allyl, methallyl, 2-methylpropenyl, 3-chloroallyl, methoxyethyl, $C_3$-$C_6$cycloalhyl which is substituted by fluorine or chlorine, or is 3-fluoropropyl.

**16.** A composition according to claim 15, wherein, in formula I, $R_1$ and $R_2$ are hydrogen.

**17.** A composition according to claim 1, wherein, in formula I, $R_1$ is hydrogen, halogen, $C_1$-$C_2$alkyl, $C_1$-$C_2$alkoxy, $C_1$-$C_2$alkylthio, $C_1$-$C_2$haloalkoxy or $CF_3$; $R_2$ is hydrogen or $C_1$-$C_2$alkyl; A is $C_1$-$C_4$alkyl or $C_3$-$C_6$cycloalkyl, each of which is monosubstituted to trisubstituted by halogen, or is $C_1$-$C_2$alkyl or $C_3$-$C_6$cycloalkyl, each of which is substituted by $C_1$-$C_2$alkoxy, $C_1$-$C_2$alkylthio, $C_1$-$C_2$alkylsulfonyl, $C_1$-$C_2$alkylsulfinyl, $C_2$-$C_3$alkenyl, $C_5$-$C_6$cycloalkenyl, amino, $C_1$-$C_2$alkylamino, $C_1$-$C_4$dialkylamino or $C_2$-$C_3$alkynyl, it being possible for the $C_2$-$C_3$alkenyl radical and for the $C_5$-$C_6$cycloalkenyl radical to be additionally monosubstituted to trisubstituted by halogen; or A is $C_1$-$C_2$alkyl which is substituted by a 4- to 6-membered saturated heterocycle which contains a hetero atom selected from the group comprising O, N and $SO_2$; X is $C_1$-$C_2$alkyl, $C_1$-$C_2$alkyl which is monosubstituted to trisubstituted by halogen, or is $C_1$-$C_2$alkoxy or $C_1$-$C_2$alkoxy which is monosubstituted to trisubstituted by halogen; Y is halogen, $C_1$-$C_2$alkyl, $C_1$-$C_2$alkyl which is monosubstituted to trisubstituted by halogen, or is $C_1$-$C_2$alkoxy, $C_1$-$C_2$alkoxy which is monosubstituted to trisubstituted by halogen; or is cyclopropyl, methylamino or dimethylamino.

**18.** A composition according to claim 1, wherein, in formula I, $R_1$ is hydrogen, halogen, $C_1$-$C_2$alkyl, $C_1$-$C_2$alkylthio or $CF_3$; $R_2$ is hydrogen or $C_1$-$C_2$alkyl; A is $C_2$-$C_4$alkyl which is monosubstituted to trisubstituted by halogen or is $C_1$-$C_2$alkyl which is substituted by $C_1$-$C_2$alkoxy, $C_1$-$C_2$alkylthio, $C_1$-$C_2$alkylsulfonyl, $C_1$-$C_2$alkylsulfinyl, $C_2$-$C_3$alkenyl or $C_2$-$C_3$alkynyl, it being possible for the $C_2$-$C_3$alkenyl radical to be additionally monosubstituted to trisubstituted by halogen; X is $C_1$-$C_2$alkyl, $C_1$-$C_2$alkyl which is monosubstituted to trisubstituted by halogen, or is $C_1$-$C_2$alkoxy or $C_1$-$C_2$alkoxy which is monosubstituted to trisubstituted by halogen; Y is halogen, $C_1$-$C_2$alkyl, $C_1$-$C_2$alkyl which is monosubstituted to trisubstituted by halogen, or is $C_1$-$C_2$alkoxy, $C_1$-$C_2$alkoxy which is monosubstituted to trisubstituted by halogen, or is cyclopropyl, methylamino or dimethylamino.

**19.** A composition according to claim 1, wherein, in formula I, $R_1$ and $R_2$ are hydrogen; A is $C_1$-$C_4$cycloalkyl which is monosubstituted to trisubstituted by halogen, or is $C_1$-$C_2$alkyl or $C_3$-$C_6$cycloalkyl, each of which is substituted by $C_1$-$C_4$alkoxy, $C_5$-$C_6$cycloalkyl or $C_2$-$C_4$alkenyl, it being possible for the $C_2$-$C_4$alkenyl radical and for the $C_5$-$C_6$cycloalkenyl radical to be additionally monosubstituted to trisubstituted by fluorine or chlorine.

**20.** A composition according to claim 16, wherein, in formula I, A is allyl, methallyl, 3-chloroallyl, methoxyethyl, 2-fluoroisopropyl or 3-fluoropropyl; X is methyl, methoxy, ethoxy or difluoromethoxy and Y is methyl, methoxy, ethoxy difluoromethoxy or chlorine.

**21.** A composition according to claim 1, wherein, in formula I, $R_1$ and $R_2$ are hydrogen; A is $C_2$-$C_4$alkyl which is monosubstituted to trisubstituted by halogen, or is $C_1$-$C_2$alkyl which is substituted by $C_1$-

$C_4$alkoxy or $C_2$-$C_4$alkenyl, it being possible for the $C_2$-$C_4$alkenyl radical to be additionally monosubstituted to trisubstituted by fluorine or chlorine.

**22.** A composition according to claim 16, wherein, in formula I, A is allyl, methallyl, 3-chloroallyl, methoxyethyl or 3-fluoropropyl; X is methyl, methoxy, ethoxy or difluoromethoxy and Y is methyl, methoxy, ethoxy, difluoromethoxy or chlorine.

**23.** A composition according to claim 1, which comprises, as compound of the formula I, N-(3-fluoropropylsulfonylpyridin-2-ylsulfonyl)-N'-(4,6-dimethoxypyrimidin-2-yl)urea.

**24.** A composition according to claim 1, which comprises, as compound of the formula I, N-(3-allylsulfonylpyridin-2-ylsulfonyl)-N'-(4,6-dimethoxypyrimidin-2-yl)urea.

**25.** A composition according to claim 1, which comprises, as compound of the formula I, N-(3-methoxyethylsulfonyl-pyridin-2-ylsulfonyl)-N'-(4,6-dimethoxypyrimidin-2-yl)urea.

**26.** A process for the preparation of the compounds of the formula I according to one of claims 1 to 25, wherein a pyridylsulfonamide of the formula II

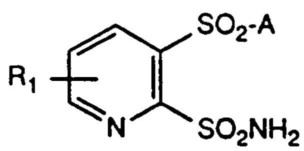

(II)

in which $R_1$ and A are as defined under formula I in claim 1 are reacted with an N-pyrimidinyl carbamate or N-triazinyl carbamate of the formula IV

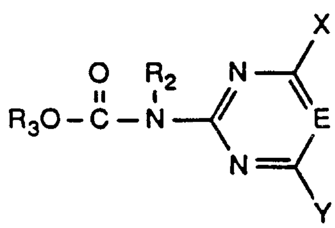

(IV)

in which X, Y, $R_2$ and E are as defined under formula I in claim 1 and $R_3$ is $C_1$-$C_4$alkyl or phenyl which can be substituted by $C_1$-$C_4$alkyl or halogen, in the presence of a base.

**27.** A process for the preparation of the compounds of the formula I according to one of claims 1 to 25, wherein a compound of the formula XVI

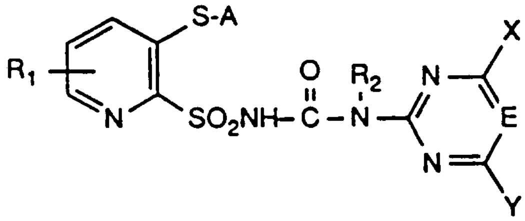

(XVI)

in which $R_1$, $R_2$, A, E, X and Y are as defined under formula I in claim 1, is oxidized.

**28.** A process for the preparation of the compounds of the formula I according to one of claims 1 to 25, wherein a pyridylsulfonamide of the formula II

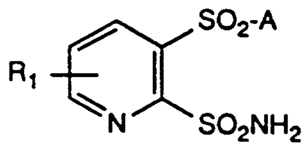

(II)

in which $R_1$ and A are as defined under formula I in claim 1, is reacted with a pyrimidinyl isocyanate or triazinyl isocyanate of the formula VIII

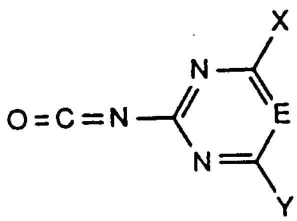

(VIII)

in which E, X, and Y are as defined under formula I, in the presence of a base.

**29.** A process for the preparation of pyridylsulfonamides of the formula II

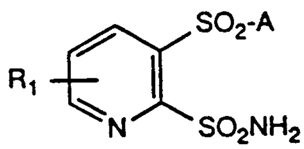

(II)

in which $R_1$ and A are as defined under formula I in claim 1, wherein a 3-mercaptopyridin-2-ylsulfonamide of the formula IIIb

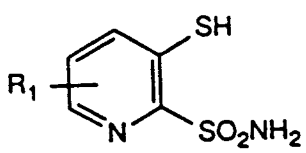

(IIIb)

in which $R_1$ is as defined under formula I in claim 1, is reacted with a compound of the formula XVIII

Z-A (XVIII)

in which A is as defined under formula I and Z is chlorine, bromine, iodine, $CH_3SO_2O$- or

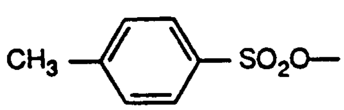

,

in the presence of a base to give the compound of the formula XX

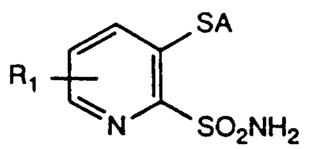

(XX)

in which A is as defined under formula I, and this compound is subsequently oxidized to give the compound of the formula II.

**30.** A composition according to claim 1, which contains between 0.1 % and 95 % of active substance of the formula I according to claim 1.

**31.** A method for controlling undesired plant growth, wherein an active substance of the formula I, according to claim 1, or a composition containing this active substance, is applied in an effective amount to the plants or their environment.

**32.** A method according to claim 31, wherein an amount of active substance of between 0.001 and 5 kg per hectare is applied.

**33.** A method for inhibiting plant growth, wherein an active substance of the formula I, according to claim 1, or a composition containing this active substance, is applied in an effective amount to the plants or their environment.

**34.** A methox for influencing plant growth with the purpose of increasing the yield, wherein an active substance of the formula I, according to claim 1, or a composition containing this active substance, is applied in an effective amount to the plants or their environment.

**35.** A method according to claim 31 for selective weed control in crops, either pre- or post-emergence.

**Revendications**
**Revendications pour les Etats contractants suivants : AT, BE, CH, DE, DK, FR, GR, IT, LI, LU, NL, SE**

**1.** N-pyridine-sulfonyl-N'-pyrimidinyl- et - triazinyl-urées de formule I

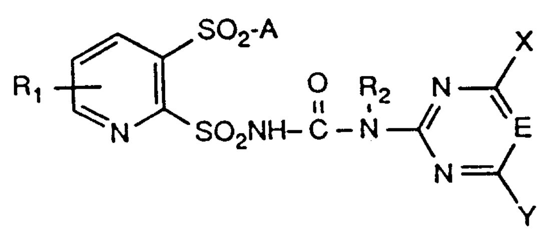

(I)

dans laquelle
$R_1$ représente l'hydrogène, un halogène, un groupe alkyle en C 1-C 4, alcoxy en C 1-C 4, alkylthio en C 1-C 4, halogénoalcoxy en C 1-C 4 ou $CF_3$;
$R_2$ représente l'hydrogène ou un groupe alkyle en C 1-C 4;
A représente un groupe alkyle en C 1-C 6 ou cycloalkyle en C 3-C 6 portant un à trois substituants halogéno, ou un groupe alkyle en C 1-C 4 ou cycloalkyle en C 3-C 6 substitué par des groupes alcoxy en C 1-C 4, alkylthio en C 1-C 4, alkylsulfinyle en C 1-C 4, alkylsulfonyle en C 1-C 4, alcényle en C 2-C 4, cycloalcényle en C 5-C 6, amino, alkylamino en C 1-C 4, di-(alkyle en C 1-C 4)-amino ou alcynyle en C 2-C 4, le groupe alcényle en C 2-C 4, de même que le groupe cycloalcényle en C 5-C 6, pouvant eux-mêmes porter un à trois substituants halogéno; ou un groupe alkyle en C 1-C 4 substitué par un hétérocycle saturé de 4 à 6 chaînons contenant un hétéroatome choisi parmi O, N et $SO_2$;
X représente un groupe alkyle en C 1-C 3, un groupe alkyle en C 1-C 3 portant un à trois substituants

halogéno, un groupe alcoxy en C 1-C 3 ou un groupe alcoxy en C 1-C 3 portant un à trois substituants halogéno;
Y représente un halogène, un groupe alkyle en C 1-C 3, un groupe alkyle en C 1-C 3 portant un à trois substituants halogéno, un groupe alcoxy en C 1-C 3, un groupe alcoxy en C 1-C 3 portant un à trois substituants halogéno, un groupe cyclopropyle, méthylamino ou diméthylamino; et
E représente l'azote ou le groupe méthine,
ainsi que les sels de ces composés.

**2.** Composés selon la revendication 1, caractérisés en ce que :
$R_1$ représente l'hydrogène, un halogène, un groupe alkyle en C 1-C 4, alcoxy en C 1-C 4, alkylthio en C 1-C 4 ou $CF_3$;
$R_2$ représente l'hydrogène ou un groupe alkyle en C 1-C 4;
A représente un groupe alkyle en C 2-C 6 portant un à trois substituants halogéno; ou un groupe alkyle en C 1-C 4 substitué par des groupes alcoxy en C 1-C 4, alkylthio en C 1-C 4, alkylsulfonyle en C 1-C 4, alkylsulfinyle en C 1-C 4, alcényle en C 2-C 4 ou alcynyle en C 2-C 4, le groupe alcényle en C 2-C 4 pouvant lui-même porter un à trois substituants halogéno;
X représente un groupe alkyle en C 1-C 3, un groupe alkyle en C 1-C 3 portant un à trois substituants halogéno, un groupe alcoxy en C 1-C 3 ou un groupe alcoxy en C 1-C 3 portant un à trois substituants halogéno;
Y représente un halogène, un groupe alkyle en C 1-C 3, un groupe alkyle en C 1-C 3 portant un à trois substituants halogéno, un groupe alcoxy en C 1-C 3, un groupe alcoxy en C 1-C 3 portant un à trois substituants halogéno, un groupe cyclopropyle, méthylamino ou diméthylamino; et
E représente l'azote ou le groupe méthine,
et les sels de ces composés.

**3.** Composés selon la revendication 1, caractérisés en ce que $R_2$ représente l'hydrogène.

**4.** Composés selon la revendication 1, caractérisés en ce que :
X représente un groupe alkyle en C 1-C 3, alcoxy en C 1-C 3 ou alcoxy en C 1-C 3 portant un à trois substituants halogéno; et
Y représente le chlore, un groupe alkyle en C 1-C 3, alcoxy en C 1-C 3 ou alcoxy en C 1-C 3 portant un à trois substituants halogéno.

**5.** Composés selon la revendication 1, caractérisés en ce que :
X représente un groupe méthyle, méthoxy, éthoxy ou difluorométhoxy; et
Y représente un groupe méthyle, méthoxy, éthoxy, difluorométhoxy ou le chlore.

**6.** Composés de formule I selon la revendication 1, caractérisés en ce que X représente un groupe méthoxy ou éthoxy et Y un groupe méthyle ou méthoxy.

**7.** Composés de formule I selon la revendication 1, caractérisés en ce que E représente le pont méthine.

**8.** Composés de formule I selon la revendication 1, caractérisés en ce que $R_1$ représente l'hydrogène, un groupe méthyle, méthoxy, le fluor ou le chlore.

**9.** Composés de formule I selon la revendication 1, caractérisés en ce que :
A représente un groupe alkyle en C 1-C 6 ou cycloalkyle en C 3-C 6 qui peut porter un à trois substituants halogéno; ou un groupe alkyle en C 1-C 4 ou cycloalkyle en C 3-C 6 substitué par des groupes alcoxy en C 1-C 4, cycloalkyle en C 5-C 6 ou alcényle en C 2-C 4; le groupe alcényle en C 2-C 4, de même que le groupe cycloalcényle en C 5-C 6, pouvant encore porter un à trois substituants halogéno.

**10.** Composés de formule I selon la revendication 1, caractérisés en ce que A représente un groupe alkyle en C 2-C 6 portant un à trois substituants halogéno; ou un groupe alkyle en C 1-C 4 substitué par un groupe alcoxy en C 1-C 4 ou alcényle en C 2-C 4, ce dernier pouvant encore porter un à trois substituants halogéno.

**11.** Composés de formule I selon la revendication 10, caractérisés en ce que $R_1$ et $R_2$ représentent l'hydrogène.

**12.** Composés de formule I selon la revendication 10, caractérisés en ce que X représente un groupe alkyle en C 1-C 3, alcoxy en C 1-C 3 ou alcoxy en C 1-C 3 portant un à trois substituants halogéno, et Y représente un groupe alkyle en C 1-C 3, alcoxy en C 1-C 3, alcoxy en C 1-C 3 portant un à trois substituants halogéno, ou le chlore.

**13.** Composés de formule I selon la revendication 10, caractérisés en ce que X représente un groupe méthyle, méthoxy, éthoxy ou difluorométhoxy et Y un groupe méthyle, méthoxy, éthoxy, difluorométhoxy ou le chlore.

**14.** Composés de formule I selon la revendication 10, caractérisés en ce que X représente un groupe méthoxy ou éthoxy et Y un groupe méthyle ou méthoxy.

**15.** Composés de formule I selon la revendication 1, caractérisés en ce que A représente un groupe allyle, méthallyle, 2-méthylpropényle, 3-chlorallyle, méthoxyéthyle, cycloalkyle en C 3-C 6 substitué par le fluor ou le chlore, ou 3-fluoropropyle.

**16.** Composés de formule I selon la revendication 15, caractérisés en ce que $R_1$ et $R_2$ représentent l'hydrogène.

**17.** Composés de formule I selon la revendication 1, caractérisés en ce que :
$R_1$ représente l'hydrogène, un halogène, un groupe alkyle en C 1-C 2, alcoxy en C 1-C 2, alkylthio en C 1-C 2, halogénoalcoxy en C 1-C 2 ou $CF_3$;
$R_2$ représente l'hydrogène ou un groupe alkyle en C 1-C 2;
A représente un groupe alkyle en C 1-C 4 ou cycloalkyle en C 3-C 6 portant un à trois substituants halogéno, ou bien un groupe alkyle en C 1-C 2 ou cycloalkyle en C 3-C 6 substitué par des groupes alcoxy en C 1-C 2, alkylthio en C 1-C 2, alkylsulfonyle en C 1-C 2, alkylsulfinyle en C 1-C 2, alcényle en C 2-C 3, cycloalcényle en C 5-C 6, amino, alkylamino en C 1-C 2, di-(alkyle en C 1-C 4)-amino ou alcynyle en C 2-C 3, le groupe alcényle en C 2-C 3, de même que le groupe cycloalcényle en C 5-C 6, pouvant porter un à trois substituants halogéno; ou un groupe alkyle en C 1-C 2 substitué par un hétérocycle saturé de 4 à 6 chaînons qui contient un hétéroatome choisi parmi O, S et $SO_2$;
X représente un groupe alkyle en C 1-C 2, alkyle en C 1-C 2 portant un à trois substituants halogéno, alcoxy en C 1-C 2 ou alcoxy en C 1-C 2 portant un à trois substituants halogéno;
Y représente un halogène, un groupe alkyle en C 1-C 2, un groupe alkyle en C 1-C 2 portant un à trois substituants halogéno, un groupe alcoxy en C 1-C 2, un groupe alcoxy en C 1-C 2 portant un à trois substituants halogéno, un groupe cyclopropyle, méthylamino ou diméthylamino.

**18.** Composés de formule I selon la revendication 1, caractérisés en ce que :
$R_1$ représente l'hydrogène, un halogène, un groupe alkyle en C 1-C 2, alkylthio en C 1-C 2 ou $CF_3$;
$R_2$ représente l'hydrogène ou un groupe alkyle en C 1-C 2; A représente un groupe alkyle en C 2-C 4 portant un à trois substituants halogéno ou un groupe alkyle en C 1-C 2 substitué par des groupes alcoxy en C 1-C 2, alkylthio en C 1-C 2, alkylsulfonyle en C 1-C 2, alkylsulfinyle en C 1-C 2, alcényle en C 2-C 3 ou alcynyle en C 2-C 3, le groupe alcényle en C 2-C 3 pouvant lui-même porter un à trois substituants halogéno;
X représente un groupe alkyle en C 1-C 2, un groupe alkyle en C 1-C 2 portant un à trois substituants halogéno, un groupe alcoxy en C 1-C 2 ou un groupe alcoxy en C 1-C 2 portant un à trois substituants halogéno;
Y représente un halogène, un groupe alkyle en C 1-C 2, un groupe alkyle en C 1-C 2 portant un à trois substituants halogéno, un groupe alcoxy en C 1-C 2, un groupe alcoxy en C 1-C 2 portant un à trois substituants halogéno, un groupe cyclopropyle, méthylamino ou diméthylamino.

**19.** Composés de formule I selon la revendication 1, caractérisés en ce que :
$R_1$ et $R_2$ représentent l'hydrogène,
A représente un groupe alkyle en C 1-C 4 portant un à trois substituants halogéno ou un groupe alkyle en C 1-C 2 ou cycloalkyle en C 3-C 6 substitué par des groupes alcoxy en C 1-C 4, cycloalcényle en C 5-C 6 ou alcényle en C 1-C 2, le groupe alcényle en C 2-C 3, de même que le groupe cycloalcényle

en C 5-C 6, pouvant porter un à trois substituants fluoro ou chloro.

**20.** Composés selon la revendication 16, caractérisés en ce que A représente un groupe allyle, méthallyle, 3-chlorallyle, méthoxyéthyle, 2-fluorisopropyle ou 3-fluoropropyle,
X représente un groupe méthyle, méthoxy, éthoxy ou difluorométhoxy, et
Y représente un groupe méthyle, méthoxy, éthoxy, difluorométhoxy ou le chlore.

**21.** Composés de formule selon la revendication 1, caractérisés en ce que $R_1$ et $R_2$ représentent l'hydrogène;
A représente un groupe alkyle en C 2-C 4 portant un à trois substituants halogéno ou un groupe alkyle en C 1-C 2 substitué par un groupe alcoxy en C 1-C 4 ou alcényle en C 2-C 4, ce dernier pouvant encore porter un à trois substituants fluoro ou chloro.

**22.** Composés selon la revendication 16, caractérisés en ce que :
A représente un groupe allyle, méthallyle, 3-chlorallyle, méthoxyéthyle ou 3-fluoropropyle,
X représente un groupe méthyle, méthoxy, éthoxy ou difluorométhoxy,
Y représente un groupe méthyle, méthoxy, éthoxy, difluorométhoxy ou le chlore.

**23.** Un composé selon la revendication 1 : la N-(3-fluoropropylsulfonyl-pyridine-2-yl-sulfonyl)-N'-(4,6-diméthoxypyrimidine-2-yl)-urée.

**24.** Un composé selon la revendication 1 : la N-(3-allylsulfonyl-pyridine-2-yl-sulfonyl)-N'-(4,6-diméthoxypyrimidine-2-yl)-urée.

**25.** Un composé selon la revendication 1 : la N-(3-méthoxyéthylsulfonyl-pyridine-2-yl-sulfonyl)-N'-(4,6-diméthoxypyrimidine-2-yl)-urée.

**26.** Procédé de préparation des composés de formule I selon une des revendications 1 à 25, caractérisé en ce que l'on fait réagir un pyridylsulfonamide de formule II

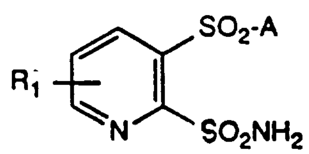

(II)

dans laquelle $R_1$ et A ont les significations indiquées en référence à la formule I dans la revendication 1, avec un N-pyrimidinyl- ou N-triazinyl-carbamate de formule IV

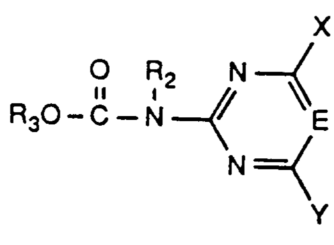

(IV)

dans laquelle X, Y, $R_2$ et E ont les significations indiquées en référence à la formule I de la revendication 1, et $R_3$ représente un groupe alkyle en C 1-C 4 ou phényle qui peut être substitué par un groupe alkyle en C 1-C 4 ou un halogène, en présence d'une base.

**27.** Procédé de préparation des composés de formule I selon une des revendications 1 à 25, caractérisé en ce que l'on oxyde un composé de formule XVI

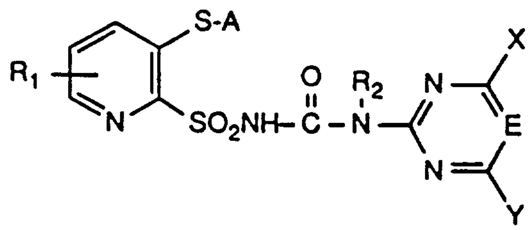

(XVI)

dans laquelle $R_1$, $R_2$, A, E, X et Y ont les significations indiquées en référence à la formule I dans la revendication 1.

**28.** Procédé de préparation des composés de formule I selon une des revendications 1 à 25, caractérisé en ce que l'on fait réagir un pyridylsulfonamide de formule II

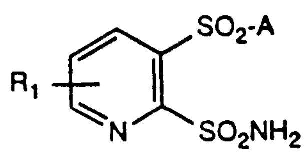

(II)

dans laquelle $R_1$ et A ont les significations indiquées en référence à la formule I dans la revendication 1, en présence d'une base, avec un pyrimidinyl- ou triazinylisocyanate de formule VIII

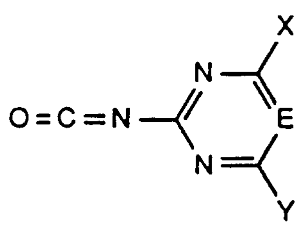

(VIII)

dans laquelle E, X et Y ont les significations indiquées en référence à la formule I.

**29.** Procédé de préparation des pyridylsulfonamides de formule II

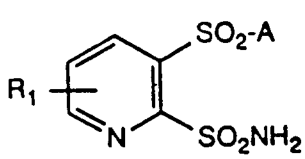

(II)

dans laquelle $R_1$ et A ont les significations indiquées en référence à la formule I dans la revendication 1, caractérisé en ce que l'on fait réagir un 3-mercaptopyridine-2-yl-sulfonamide de formule IIIb

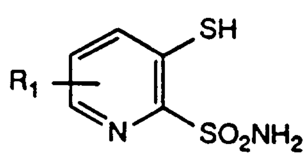

(IIIb)

dans laquelle $R_1$ a les significations indiquées en référence à la formule I dans la revendication 1, en présence d'une base, avec un composé de formule XVIII

Z-A (XVIII)

dans laquelle A a les significations indiquées en référence à la formule I et Z représente le chlore, le brome, l'iode, un groupe $CH_3SO_2O$- ou

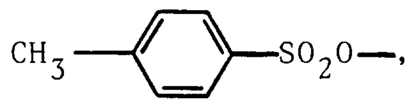

ce qui donne un composé de formule XX

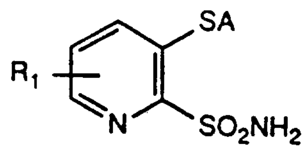

(XX)

dans laquelle A a les significations indiquées en référence à la formule I, qu'on oxyde ensuite en le composé de formule II.

**30.** Composés de formule II

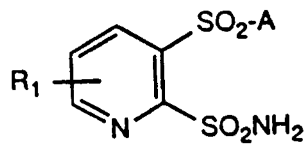

(II)

dans laquelle $R_1$ et A ont les significations indiquées en référence à la formule I dans la revendication 1.

**31.** Composés de formule III

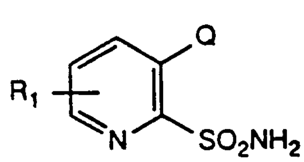

(III)

dans laquelle $R_1$ a les significations indiquées en référence à la formule I, et Q représente le fluor ou -SH, sous réserve que Q ne peut représenter -SH lorsque $R_1$ représente l'hydrogène.

**32.** Un produit herbicide et inhibiteur de la croissance des végétaux, caractérisé en ce qu'il contient une ou plusieurs sulfonylurées de formule I selon la revendication 1.

**33.** Produit selon la revendication 31, caractérisé en ce qu'il contient de 0,1% à 95% d'une substance active de formule I selon la revendication 1.

**34.** Procédé pour combattre les croissances de végétaux indésirables, caractérisé en ce que l'on applique sur les végétaux ou leur habitat une quantité efficace d'une substance active de formule I selon la revendication 1 ou d'un produit contenant une telle substance active.

**35.** Procédé selon la revendication 34, caractérisé en ce que l'on applique de 0,001 à 5 kg de substance active par ha.

**36.** Procédé pour inhiber la croissance des végétaux, caractérisé en ce que l'on applique sur les végétaux ou leur habitat une quantité efficace d'une substance active de formule I selon la revendication 1 ou d'un produit contenant une telle substance active.

**37.** Procédé pour agir sur la croissance des végétaux en vue d'une augmentation des rendements, caractérisé en ce que l'on fait agir sur les végétaux ou leur habitat une quantité efficace d'une substance active de formule I selon la revendication 1 ou d'un produit contenant une telle substance active.

**38.** Procédé selon la revendication 34, pour la lutte sélective en pré-levée ou en post-levée contre les végétaux adventices dans les cultures de végétaux utiles.

**Revendications pour l'Etat contractant suivant : ES**

**1.** Un produit herbicide et inhibiteur de la croissance des végétaux, caractérisé en ce qu'il contient une ou plusieurs sulfonylurées de formule I

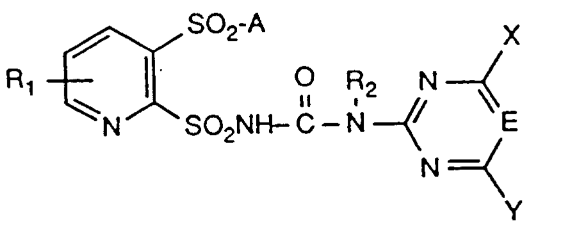

(I)

dans laquelle
$R_1$ représente l'hydrogène, un halogène, un groupe alkyle en C 1-C 4, alcoxy en C 1-C 4, alkylthio en C 1-C 4, halogénoalcoxy en C 1-C 4 ou $CF_3$;
$R_2$ représente l'hydrogène ou un groupe alkyle en C 1-C 4;
A représente un groupe alkyle en C 1-C 6 ou cycloalkyle en C 3-C 6 portant un à trois substituants halogéno, ou un groupe alkyle en C 1-C 4 ou cycloalkyle en C 3-C 6 substitué par des groupes alcoxy en C 1-C 4, alkylthio en C 1-C 4, alkylsulfinyle en C 1-C 4, alkylsulfonyle en C 1-C 4, alcényle en C 2-C 4, cycloalcényle en C 5-C 6, amino, alkylamino en C 1-C 4, di-(alkyle en C 1-C 4)-amino ou alcynyle en C 2-C 4, le groupe alcényle en C 2-C 4, de même que le groupe cycloalcényle en C 5-C 6, pouvant eux-mêmes porter un à trois substituants halogéno; ou un groupe alkyle en C 1-C 4 substitué par un hétérocycle saturé de 4 à 6 chaînons contenant un hétéroatome choisi parmi O, N et $SO_2$;
X représente un groupe alkyle en C 1-C 3, un groupe alkyle en C 1-C 3 portant un à trois substituants halogéno, un groupe alcoxy en C 1-C 3 ou un groupe alcoxy en C 1-C 3 portant un à trois substituants halogéno;
Y représente un halogène, un groupe alkyle en C 1-C 3, un groupe alkyle en C 1-C 3 portant un à trois substituants halogéno, un groupe alcoxy en C 1-C 3, un groupe alcoxy en C 1-C 3 portant un à trois substituants halogéno, un groupe cyclopropyle, méthylamino ou diméthylamino; et
E représente l'azote ou le groupe méthine.

**2.** Produit selon la revendication 1, caractérisé en ce que, dans la formule I :
$R_1$ représente l'hydrogène, un halogène, un groupe alkyle en C 1-C 4, alcoxy en C 1-C 4, alkylthio en C 1-C 4 ou $CF_3$;
$R_2$ représente l'hydrogène ou un groupe alkyle en C 1-C 4;
A représente un groupe alkyle en C 2-C 6 portant un à trois substituants halogéno; ou un groupe alkyle

en C 1-C 4 substitué par des groupes alcoxy en C 1-C 4, alkylthio en C 1-C 4, alkylsulfonyle en C 1-C 4, alkylsulfinyle en C 1-C 4, alcényle en C 2-C 4 ou alcynyle en C 2-C 4, le groupe alcényle en C 2-C 4 pouvant lui-même porter un à trois substituants halogéno;
X représente un groupe alkyle en C 1-C 3, un groupe alkyle en C 1-C 3 portant un à trois substituants halogéno, un groupe alcoxy en C 1-C 3 ou un groupe alcoxy en C 1-C 3 portant un à trois substituants halogéno;
Y représente un halogène, un groupe alkyle en C 1-C 3, un groupe alkyle en C 1-C 3 portant un à trois substituants halogéno, un groupe alcoxy en C 1-C 3, un groupe alcoxy en C 1-C 3 portant un à trois substituants halogéno, un groupe cyclopropyle, méthylamino ou diméthylamino; et
E représente l'azote ou le groupe méthine.

**3.** Produit selon la revendication 1, caractérisé en ce que, dans la formule I, $R_2$ représente l'hydrogène.

**4.** Produit selon la revendication 1, caractérisé en ce que, dans la formule I, X représente un groupe alkyle en C 1-C 3, alcoxy en C 1-C 3 ou alcoxy en C 1-C 3 portant un à trois substituants halogéno; et Y représente le chlore, un groupe alkyle en C 1-C 3, alcoxy en C 1-C 3 ou alcoxy en C 1-C 3 portant un à trois substituants halogéno.

**5.** Produit selon la revendication 1, caractérisé en ce que, dans la formule I,
X représente un groupe méthyle, méthoxy, éthoxy ou difluorométhoxy, et
Y représente un groupe méthyle, méthoxy, éthoxy, difluorométhoxy ou le chlore.

**6.** Produit selon la revendication 1, caractérisé en ce que, dans la formule I,
X représente un groupe méthoxy ou éthoxy; et Y représente un groupe méthyle ou méthoxy.

**7.** Produit selon la revendication 1, caractérisé en ce que, dans la formule I, E représente le pont méthine.

**8.** Produit selon la revendication 1, caractérisé en ce que, dans la formule I, $R_1$ représente l'hydrogène, un groupe méthyle, méthoxy, le fluor ou le chlore.

**9.** Produit selon la revendication 1, caractérisé en ce que, dans la formule I, A représente un groupe alkyle en C 1-C 6 ou cycloalkyle en C 3-C 6 qui porte un à trois substituants halogéno; ou un groupe alkyle en C 1-C 4 ou cycloalkyle en C 3-C 6 substitué par des groupes alcoxy en C 1-C 4, cycloalkyle en C 5-C 6 ou alcényle en C 2-C 4, ce dernier, de même que le groupe cycloalcényle en C 5-C 6, pouvant encore porter un à trois substituants halogéno.

**10.** Produit selon la revendication 1, caractérisé en ce que :
A représente un groupe alkyle en C 2-C 6 portant un à trois substituants halogéno; ou un groupe alkyle en C 1-C 4 substitué par un groupe alcoxy en C 1-C 4 ou alcényle en C 2-C 4, ce dernier pouvant encore porter un à trois substituants halogéno.

**11.** Produit selon la revendication 10, caractérisé en ce que, dans la formule I, $R_1$ et $R_2$ représentent l'hydrogène.

**12.** Produit selon la revendication 10, caractérisé en ce que, dans la formule I,
X représente un groupe alkyle en C 1-C 3, alcoxy en C 1-C 3 ou alcoxy en C 1-C 3 portant un à trois substituants halogéno, et
Y représente un groupe alkyle en C 1-C 3, alcoxy en C 1-C 3, alcoxy en C 1-C 3 portant un trois substituants halogéno, ou le chlore.

**13.** Produit selon la revendication 10, caractérisé en ce que, dans la formule I,
X représente un groupe méthyle, méthoxy, éthoxy ou difluorométhoxy et Y un groupe méthyle, méthoxy, éthoxy, difluorométhoxy ou le chlore.

**14.** Produit selon la revendication 10, caractérisé en ce que, dans la formule I,
X représente un groupe méthoxy ou éthoxy et Y un groupe méthyle ou méthoxy.

**15.** Produit selon la revendication 1, caractérisé en ce que, dans la formule I, A représente un groupe allyle, méthallyle, 2-méthylpropényle, 3-chlorallyle, méthoxyéthyle, cycloalkyle en C 3-C 6 substitué par le fluor ou le chlore, ou 3-fluoropropyle.

**16.** Produit selon la revendication 15, caractérisé en ce que, dans la formule I, $R_1$ et $R_2$ représentent l'hydrogène.

**17.** Produit selon la revendication 1, caractérisé en ce que, dans la formule I,
$R_1$ représente l'hydrogène, un halogène, un groupe alkyle en C 1-C 2, alcoxy en C 1-C 2, alkylthio en C 1-C 2, halogénoalcoxy en C 1-C 2 ou $CF_3$;
$R_2$ représente l'hydrogène ou un groupe alkyle en C 1-C 2; A représente un groupe alkyle en C 1-C 4 ou cycloalkyle en C 3-C 6 portant un à trois substituants halogéno, ou bien un groupe alkyle en C 1-C 2 ou cycloalkyle en C 3-C 6 substitué par des groupes alcoxy en C 1-C 2, alkylthio en C 1-C 2, alkylsulfonyle en C 1-C 2, alkylsulfinyle en C 1-C 2, alcényle en C 2-C 3, cycloalcényle en C 5-C 6, amino, alkylamino en C 1-C 2, di-(alkyle en C 1-C 4)-amino ou alcynyle en C 2-C 3, le groupe alcényle en C 2-C 3, de même que le groupe cycloalcényle en C 5-C 6, pouvant porter un à trois substituants halogéno; ou un groupe alkyle en C 1-C 2 substitué par un hétérocycle saturé de 4 à 6 chaînons qui contient un hétéroatome choisi parmi O, S et $SO_2$;
X représente un groupe alkyle en C 1-C 2, alkyle en C 1-C 2 portant un à trois substituants halogéno, alcoxy en C 1-C 2 ou alcoxy en C 1-C 2 portant un à trois substituants halogéno;
Y représente un halogène, un groupe alkyle en C 1-C 2, un groupe alkyle en C 1-C 2 portant un à trois substituants halogéno, un groupe alcoxy en C 1-C 2, un groupe alcoxy en C 1-C 2 portant un à trois substituants halogéno, un groupe cyclopropyle, méthylamino ou diméthylamino.

**18.** Produit selon la revendication 1, caractérisé en ce que, dans la formule I,
$R_1$ représente l'hydrogène, un halogène, un groupe alkyle en C 1-C 2, alkylthio en C 1-C 2 ou $CF_3$;
$R_2$ représente l'hydrogène ou un groupe alkyle en C 1-C 2;
A représente un groupe alkyle en C 2-C 4 portant un à trois substituants halogéno ou un groupe alkyle en C 1-C 2 substitué par des groupes alcoxy en C 1-C 2, alkylthio en C 1-C 2, alkylsulfonyle en C 1-C 2, alkylsulfinyle en C 1-C 2, alcényle en C 2-C 3 ou alcynyle en C 2-C 3, le groupe alcényle en C 2-C 3 pouvant lui-même porter un à trois substituants halogéno;
X représente un groupe alkyle en C 1-C 2, un groupe alkyle en C 1-C 2 portant un à trois substituants halogéno, un groupe alcoxy en C 1-C 2 ou un groupe alcoxy en C 1-C 2 portant un à trois substituants halogéno;
Y représente un halogène, un groupe alkyle en C 1-C 2, un groupe alkyle en C 1-C 2 portant un à trois substituants halogéno, un groupe alcoxy en C 1-C 2, un groupe alcoxy en C 1-C 2 portant à à trois substituants halogéno, un groupe cyclopropyle, méthylamino ou diméthylamino.

**19.** Produit selon la revendication 1, caractérisé en ce que, dans la formule I,
$R_1$ et $R_2$ représentent l'hydrogène,
A représente un groupe alkyle en C 1-C 4 portant un à trois substituants halogéno ou un groupe alkyle en C 1-C 2 ou cycloalkyle en C 3-C 6 substitué par des groupes alcoxy en C 1-C 4, cycloalcényle en C 5-C 6 ou alcényle en C 1-C 2, le groupe alcényle en C 2-C 3, de même que le groupe cycloalcényle en C 5-C 6, pouvant porter un à trois substituants fluoro ou chloro.

**20.** Produit selon la revendication 1, caractérisé en ce que, dans la formule I,
A représente un groupe allyle, méthallyle, 3-chlorallyle, méthoxyéthyle, 2-fluorisopropyle ou 3-fluoropropyle,
X représente un groupe méthyle, méthoxy, éthoxy ou difluorométhoxy, et
Y représente un groupe méthyle, méthoxy, éthoxy, difluorométhoxy ou le chlore.

**21.** Produit selon la revendication 1, caractérisé en ce que, dans la formule I,
$R_1$ et $R_2$ représentent l'hydrogène;
A représente un groupe alkyle en C 2-C 4 portant un à trois substituants halogéno ou un groupe alkyle en C 1-C 2 substitué par un groupe alcoxy en C 1-C 4 ou alcényle en C 2-C 4, ce dernier pouvant encore porter un à trois substituants fluoro ou chloro.

**22.** Produit selon la revendication 16, caractérisé en ce que, dans la formule I,
A représente un groupe allyle, méthallyle, 3-chlorallyle, méthoxyéthyle ou 3-fluoropropyle, X représente un groupe méthyle, méthoxy, éthoxy ou difluorométhoxy,
Y représente un groupe méthyle, méthoxy, éthoxy, difluorométhoxy ou le chlore.

**23.** Produit selon la revendication 1, caractérisé en ce qu'il contient en tant que composé de formule I : la N-(3-fluoropropylsulfonyl-pyridine-2-yl-sulfonyl)-N'-(4,6-diméthoxypyrimidine-2-yl)-urée.

**24.** Produit selon la revendication 1, caractérisé en ce qu'il contient en tant que composé de formule I : la N-(3-allylsulfonyl-pyridine-2-yl-sulfonyl-)N'-(4,6-diméthoxypyrimidine-2-yl)-urée.

**25.** Produit selon la revendication 1, caractérisé en ce qu'il contient en tant que composé de formule I : la N-(3-méthoxyéthylsulfonyl-pyridine-2-yl-sulfonyl)-N'-(4,6-diméthoxypyrimidine-2-yl)-urée.

**26.** Procédé de préparation des composés de formule I selon une des revendications 1 à 25, caractérisé en ce que l'on fait réagir un pyridylsulfonamide de formule II

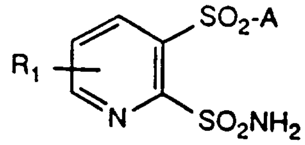

(II)

dans laquelle $R_1$ et A ont les significations indiquées en référence à la formule I dans la revendication 1, avec un N-pyrimidinyl- ou N-triazinyl-carbamate de formule IV

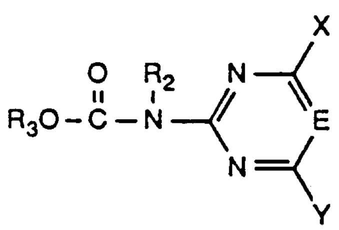

(IV)

dans laquelle X, Y, $R_2$ et E ont les significations indiquées en référence à la formule I de la revendication 1, et $R_3$ représente un groupe alkyle en C 1-C 4 ou phényle qui peut être substitué par un groupe alkyle en C 1-C 4 ou un halogène, en présence d'une base.

**27.** Procédé de préparation des composés de formule I selon une des revendications 1 à 25, caractérisé en ce que l'on oxyde un composé de formule XVI

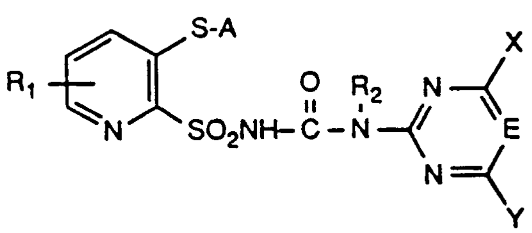

(XVI)

dans laquelle $R_1$, $R_2$, A, E, X et Y ont les significations indiquées en référence à la formule I dans la revendication 1.

**28.** Procédé de préparation des composés de formule I selon une des revendications 1 à 25, caractérisé en ce que l'on fait réagir un pyridylsulfonamide de formule II

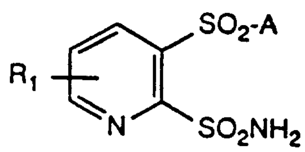

(II)

dans laquelle $R_1$ et A ont les significations indiquées en référence à la formule I dans la revendication 1, en présence d'une base, avec un pyrimidinyl- ou triazinylisocyanate de formule VIII

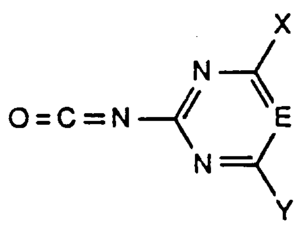

(VIII)

dans laquelle E, X et Y ont les significations indiquées en référence à la formule I.

**29.** Procédé de préparation des pyridylsulfonamides de formule II

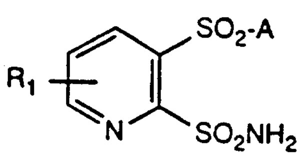

(II)

dans laquelle $R_1$ et A ont les significations indiquées en référence à la formule I dans la revendication 1, caractérisé en ce que l'on fait réagir un 3-mercaptopyridine-2-yl-sulfonamide de formule IIIb

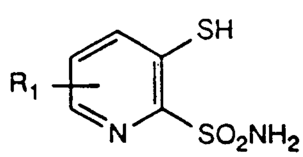

(IIIb)

dans laquelle $R_1$ a les significations indiquées en référence à la formule I dans la revendication 1, en présence d'une base, avec un composé de formule XVIII

Z-A (XVIII)

dans laquelle A a les significations indiquées en référence à la formule I et Z représente le chlore, le brome, l'iode, un groupe $CH_3SO_2O$- ou

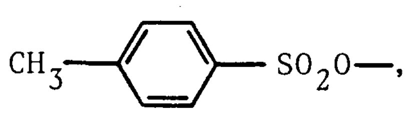

ce qui donne un composé de formule XX

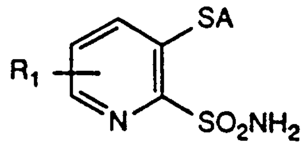

(XX)

dans laquelle A a les significations indiquées en référence à la formule I, qu'on oxyde ensuite en le composé de formule II.

**30.** Produit selon la revendication 1, caractérisé en ce qu'il contient de 0,1 à 95% d'une substance active de formule I selon la revendication 1.

**31.** Procédé pur combattre les croissances de végétaux indésirables, caractérisé en ce que l'on applique sur les végétaux ou leur habitat une quantité efficace d'une substance active de formule I selon la revendication 1 ou d'un produit contenant une telle substance active.

**32.** Procédé selon la revendication 31, caractérisé en ce que l'on applique de 0,001 à 5 kg de substance active par ha.

**33.** Procédé pour inhiber la croissance des végétaux, caractérisé en ce que l'on applique sur les végétaux ou leur habitat une quantité efficace d'une substance active de formule I selon la revendication 1 ou d'un produit contenant une telle substance active.

**34.** Procédé pour agir sur la croissance des végétaux en vue d'une augmentation des rendements, caractérisé en ce que l'on applique sur les végétaux ou leur habitat une quantité efficace d'une substance active de formule I selon la revendication 1 ou d'un produit contenant une telle substance active.

**35.** Procédé selon la revendication 31, pour la lutte sélective en pré-levée ou en post-levée contre les végétaux adventices dans les cultures de végétaux utiles.